(19) Europäisches Patentamt
European Patent Office
Office européen des brevets

(11) EP 4 501 967 A1

(12) **EUROPEAN PATENT APPLICATION**
published in accordance with Art. 153(4) EPC

(43) Date of publication:
**05.02.2025 Bulletin 2025/06**

(21) Application number: **23780835.7**

(22) Date of filing: **30.03.2023**

(51) International Patent Classification (IPC):
***C07K 16/30*** *(2006.01)* ***A61K 39/395*** *(2006.01)*
***A61K 47/68*** *(2017.01)* ***A61P 35/00*** *(2006.01)*
***C12N 15/13*** *(2006.01)*

(52) Cooperative Patent Classification (CPC):
**A61K 39/395; A61K 47/68; A61P 35/00; C07K 16/00; C07K 16/30**

(86) International application number:
**PCT/JP2023/013095**

(87) International publication number:
**WO 2023/190827 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.03.2022 JP 2022057542**

(71) Applicants:
- **The University of Tokyo**
  **Bunkyo-ku, Tokyo 113-8654 (JP)**
- **Taiho Pharmaceutical Co., Ltd.**
  **Chiyoda-ku, Tokyo 101-8444 (JP)**

(72) Inventors:
- **ISHIKAWA Shumpei**
  **Tokyo 113-8654 (JP)**
- **KATOH Hiroto**
  **Tokyo 113-8654 (JP)**
- **FURUYA Genta**
  **Tokyo 113-8654 (JP)**
- **SENGA Shogo**
  **Tsukuba-shi, Ibaraki 300-2611 (JP)**

(74) Representative: **Wächtershäuser & Hartz Patentanwaltspartnerschaft mbB**
**Weinstraße 8**
**80333 München (DE)**

Remarks:
The complete document including Reference Table(s) and the Sequence Listing(s) can be downloaded from the EPO website

(54) **PH-DEPENDENT ANTI-SULFATED GLYCOSAMINOGLYCAN ANTIBODY AND ANTIBODY-DRUG CONJUGATE**

(57) This invention provides an antibody that binds specifically to sulfated glycosaminoglycan (sGAG) in a pH-dependent manner. The antibody that binds specifically to sulfated glycosaminoglycan is at least one antibody selected from the group consisting of (1) to (3): (1) an antibody comprising a heavy chain that comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 and a light chain that comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6; (2) an antibody comprising a heavy chain that comprises CDR1 consisting of the amino acid sequence represented by SEQ ID NO: 7, CDR2 consisting of the amino acid sequence represented by SEQ ID NO: 8, and CDR3 consisting of the amino acid sequence represented by SEQ ID NO: 9 and a light chain that comprises CDR1 consisting of the amino acid sequence represented by SEQ ID NO: 10, CDR2 consisting of the amino acid sequence represented by AAS, and CDR3 consisting of the amino acid sequence represented by SEQ ID NO: 12; and (3) an antibody comprising a heavy chain that comprises CDR1 consisting of the amino acid sequence represented by SEQ ID NO: 13, CDR2 consisting of the amino acid sequence represented by SEQ ID NO: 14, and CDR3 consisting of the amino acid sequence represented by SEQ ID NO: 15 and a light chain that comprises CDR1 consisting of the amino acid sequence

EP 4 501 967 A1

represented by SEQ ID NO: 16, CDR2 consisting of the amino acid sequence represented by WAS, and CDR3 consisting of the amino acid sequence represented by SEQ ID NO: 18.

**Description**

Technical Field

The present invention relates to an antibody that binds specifically to sulfated glycosaminoglycan (hereafter abbreviated to as "sGAG") and an antibody-drug conjugate (ADC) comprising such antibody.

Background Art

Glycosaminoglycan are long-chain polysaccharides with repeating disaccharide units of an amino sugar and an uronic acid or galactose, and they are classified in accordance with, for example, constituent sugar types, degrees of sulfation, sites of sulfation, and binding patterns of constituent sugars. Representative examples of known glycosaminoglycan include heparan sulfate, heparin, chondroitin sulfate, dermatan sulfate, keratan sulfate, and hyaluronic acid, and the diversity is observed in chain lengths and degrees of sulfation. On the cell surface, glycosaminoglycan is present in the form of a proteoglycan bound to a core protein, and it plays a key role as a growth factor receptor or the like.

The constituent sugars, the degrees of sulfation, and the chain lengths of glycosaminoglycan on the cell surfaces are known to vary to a significant extent between tumor tissue and healthy tissue, and a number of reports have been made that glycosaminoglycan can be therapeutic targets against tumors (Non Patent Literature 1 to Non Patent Literature 3). A drug conjugate comprising a cytotoxic compound bound to malaria-derived VAR2CSA binding to tumor-specific sulfated glycosaminoglycan, which is rich in chondroitin sulfate, has been found to exert anti-tumor effects using *in vivo* models (Non Patent Literature 3). While efficacy of sulfated glycosaminoglycan as a therapeutic target has been indicated, a tumor-specific sugar chain structure or tumor-specific targeting has not yet been fully elucidated.

An antibody-drug conjugate (ADC) comprising a cytotoxic drug bound to an antibody binding to an antigen that can be expressed on the cell surface and internalized in the cell can deliver a drug in a target-expressing-cell-selective manner. Thus, such ADC is expected to selectively kill cancer cells (Non Patent Literature 4 and Non Patent Literature 5). Sulfated glycosaminoglycan has been indicated as a tumor-specific B cell antigen as a result of repertoire analysis and bioinformatic analysis of tumor-infiltrating B cells in diffuse-type gastric carcinoma (DGC). While efficacy of anti-sulfated glycosaminoglycan antibodies produced by tumor-infiltrating B cells or antibody-drug conjugates comprising such antibodies in the treatment of cancer has been expected, aggregation in tumors upon administration thereof or anti-tumor effects has not yet been elucidated (Patent Literature 1 and Non Patent Literature 6). In addition, sulfated glycosaminoglycan is abundant in healthy cells. From the viewpoint of safety, accordingly, development of a drug that recognizes a tumor-specific sugar chain structure or is activated in a tumor-specific manner has been awaited.

Citation List

Patent Literature

Patent Literature 1: WO 2018/235855

Non Patent Literature

Non Patent Literature 1: Int. J. Mol. Sci., 2020, 21 (17), 5983
Non Patent Literature 2: Biomolecules, 2021, 11 (3), 395
Non Patent Literature 3: Cancer Cell, 2015, 28 (4), 500-514
Non Patent Literature 4: Bioconjugate Chem., 2010, 21, 5-13
Non Patent Literature 5: Current Opinion in Chemical Biology, 2010, 14, 529-537
Non Patent Literature 6: Cell Rep., 2017, 20 (5), 1073-1087

Summary of Invention

Technical Problem

The present invention provides an antibody that binds specifically to sulfated glycosaminoglycan (sGAG) in a pH-dependent manner. The present invention also provides an antibody-drug conjugate (ADC) comprising such antibody, which is useful as an anti-tumor agent.

Solution to Problem

The present invention provides an antibody that binds to sGAG in a pH-dependent manner and an antibody-drug conjugate (ADC) comprising such antibody.

The present invention has the following features.

[1] An antibody binding specifically to sulfated glycosaminoglycan, which is at least one antibody selected from the group consisting of (1) to (3) below:

(1) an antibody comprising

a heavy chain that comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 and
a light chain that comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6;

(2) an antibody comprising

a heavy chain that comprises CDR1 consisting of the amino acid sequence represented by SEQ ID NO: 7, CDR2 consisting of the amino acid sequence represented by SEQ ID NO: 8, and CDR3 consisting of the amino acid sequence represented by SEQ ID NO: 9 and
a light chain that comprises CDR1 consisting of the amino acid sequence represented by SEQ ID NO: 10, CDR2 consisting of the amino acid sequence represented by AAS, and CDR3 consisting of the amino acid sequence represented by SEQ ID NO: 12; and

(3) an antibody comprising

a heavy chain that comprises CDR1 consisting of the amino acid sequence represented by SEQ ID NO: 13, CDR2 consisting of the amino acid sequence represented by SEQ ID NO: 14, and CDR3 consisting of the amino acid sequence represented by SEQ ID NO: 15 and
a light chain that comprises CDR1 consisting of the amino acid sequence represented by SEQ ID NO: 16, CDR2 consisting of the amino acid sequence represented by WAS, and CDR3 consisting of the amino acid sequence represented by SEQ ID NO: 18.

[2] The antibody according to [1], which is at least one antibody selected from the group consisting of (1) to (4) below:

(1) an antibody comprising

a heavy chain comprising a variable region that consists of the amino acid sequence represented by SEQ ID NO: 20 or a heavy chain comprising a variable region that has at least 90% sequence identity to the amino acid sequence represented by SEQ ID NO: 20 and has activity of binding to sulfated glycosaminoglycan and
a light chain comprising a variable region that consists of the amino acid sequence represented by SEQ ID NO: 22 or a light chain comprising a variable region that has at least 90% sequence identity to the amino acid sequence represented by SEQ ID NO: 22 and has activity of binding to sulfated glycosaminoglycan;

(2) an antibody comprising

a heavy chain comprising a variable region that consists of the amino acid sequence represented by SEQ ID NO: 24 or a heavy chain comprising a variable region that has at least 90% sequence identity to the amino acid sequence represented by SEQ ID NO: 24 and has activity of binding to sulfated glycosaminoglycan and
a light chain comprising a variable region that consists of the amino acid sequence represented by SEQ ID NO: 26 or a light chain comprising a variable region that has at least 90% sequence identity to the amino acid sequence represented by SEQ ID NO: 26 and has activity of binding to sulfated glycosaminoglycan;

(3) an antibody comprising

a heavy chain comprising a variable region that consists of the amino acid sequence represented by SEQ ID NO: 28 or a heavy chain comprising a variable region that has at least 90% sequence identity to the amino acid sequence represented by SEQ ID NO: 28 and has activity of binding to sulfated glycosaminoglycan and a light chain comprising a variable region that consists of the amino acid sequence represented by SEQ ID NO: 30 or a light chain comprising a variable region that has at least 90% sequence identity to the amino acid sequence represented by SEQ ID NO: 30 and has activity of binding to sulfated glycosaminoglycan; and

(4) an antibody comprising

a heavy chain comprising a variable region that consists of the amino acid sequence represented by SEQ ID NO: 32 or a heavy chain comprising a variable region that has at least 90% sequence identity to the amino acid sequence represented by SEQ ID NO: 32 and has activity of binding to sulfated glycosaminoglycan and a light chain comprising a variable region that consists of the amino acid sequence represented by SEQ ID NO: 34 or a light chain comprising a variable region that has at least 90% sequence identity to the amino acid sequence represented by SEQ ID NO: 34 and has activity of binding to sulfated glycosaminoglycan.

[3] The antibody according to [1] or [2], which is at least one antibody selected from the group consisting of (1) to (4) below:

(1) an antibody comprising

a heavy chain consisting of the amino acid sequence represented by SEQ ID NO: 36 or a heavy chain having at least 90% sequence identity to the amino acid sequence represented by SEQ ID NO: 36 and having activity of binding to sulfated glycosaminoglycan and
a light chain consisting of the amino acid sequence represented by SEQ ID NO: 38 or a light chain having at least 90% sequence identity to the amino acid sequence represented by SEQ ID NO: 38 and having activity of binding to sulfated glycosaminoglycan;

(2) an antibody comprising

a heavy chain consisting of the amino acid sequence represented by SEQ ID NO: 40 or a heavy chain having at least 90% sequence identity to the amino acid sequence represented by SEQ ID NO: 40 and having activity of binding to sulfated glycosaminoglycan and
a light chain consisting of the amino acid sequence represented by SEQ ID NO: 42 or a light chain having at least 90% sequence identity to the amino acid sequence represented by SEQ ID NO: 42 and having activity of binding to sulfated glycosaminoglycan;

(3) an antibody comprising

a heavy chain consisting of the amino acid sequence represented by SEQ ID NO: 44 or a heavy chain having at least 90% sequence identity to the amino acid sequence represented by SEQ ID NO: 44 and having activity of binding to sulfated glycosaminoglycan and
a light chain consisting of the amino acid sequence represented by SEQ ID NO: 46 or a light chain having at least 90% sequence identity to the amino acid sequence represented by SEQ ID NO: 46 and having activity of binding to sulfated glycosaminoglycan; and

(4) an antibody comprising

a heavy chain consisting of the amino acid sequence represented by SEQ ID NO: 48 or a heavy chain having at least 90% sequence identity to the amino acid sequence represented by SEQ ID NO: 48 and having activity of binding to sulfated glycosaminoglycan and
a light chain consisting of the amino acid sequence represented by SEQ ID NO: 50 or a light chain having at least 90% sequence identity to the amino acid sequence represented by SEQ ID NO: 50 and having activity of binding to sulfated glycosaminoglycan.

[4] The antibody according to any of [1] to [3], which is a human antibody.
[5] The antibody according to any of [1] to [4], which is an antibody of the IgG1 subclass.
[6] The antibody according to any of [1] to [5], which binds with high affinity in an acidic pH range.

[7] An antibody-drug conjugate (ADC) represented by Formula 1:

Ab-(L-D)p (1)

wherein
Ab represents an antibody binding specifically to sulfated glycosaminoglycan, which is at least one antibody selected from the group consisting of (1) to (3) below:

(1) an antibody comprising

a heavy chain that comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 and
a light chain that comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6;

(2) an antibody comprising

a heavy chain that comprises CDR1 consisting of the amino acid sequence represented by SEQ ID NO: 7, CDR2 consisting of the amino acid sequence represented by SEQ ID NO: 8, and CDR3 consisting of the amino acid sequence represented by SEQ ID NO: 9 and
a light chain that comprises CDR1 consisting of the amino acid sequence represented by SEQ ID NO: 10, CDR2 consisting of the amino acid sequence represented by AAS, and CDR3 consisting of the amino acid sequence represented by SEQ ID NO: 12; and

(3) an antibody comprising

a heavy chain that comprises CDR1 consisting of the amino acid sequence represented by SEQ ID NO: 13, CDR2 consisting of the amino acid sequence represented by SEQ ID NO: 14, and CDR3 consisting of the amino acid sequence represented by SEQ ID NO: 15 and
a light chain that comprises CDR1 consisting of the amino acid sequence represented by SEQ ID NO: 16, CDR2 consisting of the amino acid sequence represented by WAS, and CDR3 consisting of the amino acid sequence represented by SEQ ID NO: 18,

wherein

L represents 6-maleimidocaproyl-valine-citrulline-p-aminobenzyloxycarbonyl (MC-Val-Cit-PAB), 6-maleimidocaproyl-glycine-glycine-phenylalanine-glycine (MC-GGFG), N-succinimidyl 4-(N-maleimidomethyl)cyclohexane-1-carboxylate (SMCC), 4-(2-pyridyldithio)butyric acid N-hydroxysuccinimide ester (SPDB), or MC-CL2;
D represents monomethyl auristatin, maytansinoid, or a camptothecin derivative; and
p is an integer selected from 1 to 12.

[8] The antibody-drug conjugate (ADC) according to [7], wherein Ab represents at least one antibody selected from the group consisting of (1) to (4) below:

(1) an antibody comprising

a heavy chain comprising a variable region that consists of the amino acid sequence represented by SEQ ID NO: 20 or a heavy chain comprising a variable region that has at least 90% sequence identity to the amino acid sequence represented by SEQ ID NO: 20 and has activity of binding to sulfated glycosaminoglycan and
a light chain comprising a variable region that consists of the amino acid sequence represented by SEQ ID NO: 22 or a light chain comprising a variable region that has at least 90% sequence identity to the amino acid sequence represented by SEQ ID NO: 22 and has activity of binding to sulfated glycosaminoglycan;

(2) an antibody comprising

a heavy chain comprising a variable region that consists of the amino acid sequence represented by SEQ ID

NO: 24 or a heavy chain comprising a variable region that has at least 90% sequence identity to the amino acid sequence represented by SEQ ID NO: 24 and has activity of binding to sulfated glycosaminoglycan and a light chain comprising a variable region that consists of the amino acid sequence represented by SEQ ID NO: 26 or a light chain comprising a variable region that has at least 90% sequence identity to the amino acid sequence represented by SEQ ID NO: 26 and has activity of binding to sulfated glycosaminoglycan;

(3) an antibody comprising

a heavy chain comprising a variable region that consists of the amino acid sequence represented by SEQ ID NO: 28 or a heavy chain comprising a variable region that has at least 90% sequence identity to the amino acid sequence represented by SEQ ID NO: 28 and has activity of binding to sulfated glycosaminoglycan and a light chain comprising a variable region that consists of the amino acid sequence represented by SEQ ID NO: 30 or a light chain comprising a variable region that has at least 90% sequence identity to the amino acid sequence represented by SEQ ID NO: 30 and has activity of binding to sulfated glycosaminoglycan; and

(4) an antibody comprising

a heavy chain comprising a variable region that consists of the amino acid sequence represented by SEQ ID NO: 32 or a heavy chain comprising a variable region that has at least 90% sequence identity to the amino acid sequence represented by SEQ ID NO: 32 and has activity of binding to sulfated glycosaminoglycan and a light chain comprising a variable region that consists of the amino acid sequence represented by SEQ ID NO: 34 or a light chain comprising a variable region that has at least 90% sequence identity to the amino acid sequence represented by SEQ ID NO: 34 and has activity of binding to sulfated glycosaminoglycan.

[9] The antibody-drug conjugate (ADC) according to [7] or [8], wherein Ab represents at least one antibody selected from the group consisting of (1) to (4) below:

(1) an antibody comprising

a heavy chain consisting of the amino acid sequence represented by SEQ ID NO: 36 or a heavy chain having at least 95% sequence identity to the amino acid sequence represented by SEQ ID NO: 36 and having activity of binding to sulfated glycosaminoglycan and
a light chain consisting of the amino acid sequence represented by SEQ ID NO: 38 or a light chain having at least 95% sequence identity to the amino acid sequence represented by SEQ ID NO: 38 and having activity of binding to sulfated glycosaminoglycan;

(2) an antibody comprising

a heavy chain consisting of the amino acid sequence represented by SEQ ID NO: 40 or a heavy chain having at least 95% sequence identity to the amino acid sequence represented by SEQ ID NO: 40 and having activity of binding to sulfated glycosaminoglycan and
a light chain consisting of the amino acid sequence represented by SEQ ID NO: 42 or a light chain having at least 95% sequence identity to the amino acid sequence represented by SEQ ID NO: 42 and having activity of binding to sulfated glycosaminoglycan;

(3) an antibody comprising

a heavy chain consisting of the amino acid sequence represented by SEQ ID NO: 44 or a heavy chain having at least 95% sequence identity to the amino acid sequence represented by SEQ ID NO: 44 and having activity of binding to sulfated glycosaminoglycan and
a light chain consisting of the amino acid sequence represented by SEQ ID NO: 46 or a light chain having at least 95% sequence identity to the amino acid sequence represented by SEQ ID NO: 46 and having activity of binding to sulfated glycosaminoglycan; and

(4) an antibody comprising

a heavy chain consisting of the amino acid sequence represented by SEQ ID NO: 48 or a heavy chain having at least 95% sequence identity to the amino acid sequence represented by SEQ ID NO: 48 and having activity

of binding to sulfated glycosaminoglycan and
a light chain consisting of the amino acid sequence represented by SEQ ID NO: 50 or a light chain having at least 95% sequence identity to the amino acid sequence represented by SEQ ID NO: 50 and having activity of binding to sulfated glycosaminoglycan.

[10] The antibody-drug conjugate (ADC) according to any of [7] to [9], wherein Ab represents an antibody comprising a heavy chain that comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 and a light chain that comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6.
[11] The antibody-drug conjugate (ADC) according to any of [7] to [10], wherein Ab represents an antibody comprising a heavy chain comprising a variable region that consists of the amino acid sequence represented by SEQ ID NO: 20 and a light chain comprising a variable region that consists of the amino acid sequence represented by SEQ ID NO: 22.
[12] The antibody-drug conjugate (ADC) according to any of [7] to [11], wherein Ab represents an antibody comprising a heavy chain consisting of the amino acid sequence represented by SEQ ID NO: 36 and a light chain consisting of the amino acid sequence represented by SEQ ID NO: 38.
[13] The antibody-drug conjugate (ADC) according to any of [7] to [10], wherein Ab represents an antibody comprising a heavy chain comprising a variable region that consists of the amino acid sequence represented by SEQ ID NO: 32 and a light chain comprising a variable region that consists of the amino acid sequence represented by SEQ ID NO: 34.
[14] The antibody-drug conjugate (ADC) according to any of [7] to [10], wherein Ab represents an antibody comprising a heavy chain consisting of the amino acid sequence represented by SEQ ID NO: 48 and a light chain consisting of the amino acid sequence represented by SEQ ID NO: 50.
[15] The antibody-drug conjugate (ADC) according to any of [7] to [14], wherein Ab represents the IgG1 antibody.
[16] The antibody-drug conjugate (ADC) according to any of [7] to [15], wherein Ab represents a human antibody.
[17] The antibody-drug conjugate (ADC) according to any of [7] to [16], wherein Ab represents an antibody that binds with higher affinity in an acidic pH range than in a neutral pH range.
[18] The antibody-drug conjugate (ADC) according to any of [7] to [17], wherein L represents 6-maleimidocaproyl-valine-citrulline-p-aminobenzyloxycarbonyl (MC-Val-Cit-PAB), 6-maleimidocaproyl-glycine-glycine-phenylalanine-glycine (MC-GGFG), N-succinimidyl 4-(N-maleimidomethyl)cyclohexane-1-carboxylate (SMCC), 4-(2-pyridyldithio)butyric acid N-hydroxysuccinimide ester (SPDB), or MC-CL2.
[19] The antibody-drug conjugate (ADC) according to any of [7] to [18], wherein D represents MMAE, MMAF, DM1, DM4, Dx8951, Dxd, or SN-38.
[20] The antibody-drug conjugate (ADC) according to any of [7] to [19], wherein L-D represents SMCC-DM1, SPDB-DM4, MC-Val-Cit-PAB-MMAE, MC-Val-Cit-PAB-MMAF, MC-GGFG-Dx8591, or MC-CL2-SN-38.
[21] The antibody-drug conjugate (ADC) according to any of [7] to [10] or [15] to [20], wherein

Ab represents an antibody comprising a heavy chain comprising a variable region that consists of the amino acid sequence represented by SEQ ID NO: 20 and a light chain comprising a variable region that consists of the amino acid sequence represented by SEQ ID NO: 22; and
L-D represents MC-Val-Cit-PAB-MMAE.

[22] The antibody-drug conjugate (ADC) according to any of [7] to [10] or [15] to [20], wherein

Ab represents an antibody comprising a heavy chain consisting of the amino acid sequence represented by SEQ ID NO: 36 and a light chain consisting of the amino acid sequence represented by SEQ ID NO: 38; and
L-D represents MC-Val-Cit-PAB-MMAE.

[23] The antibody-drug conjugate (ADC) according to any of [7] to [10] or [15] to [20], wherein

Ab represents an antibody comprising a heavy chain comprising a variable region that consists of the amino acid sequence represented by SEQ ID NO: 32 and a light chain comprising a variable region that consists of the amino acid sequence represented by SEQ ID NO: 34; and
L-D represents MC-Val-Cit-PAB-MMAE.

[24] The antibody-drug conjugate (ADC) according to any of [7] to [10] or [15] to [20], wherein

Ab represents an antibody comprising a heavy chain consisting of the amino acid sequence represented by SEQ

ID NO: 48 and a light chain consisting of the amino acid sequence represented by SEQ ID NO: 50; and L-D represents MC-Val-Cit-PAB-MMAE.

[25] The antibody-drug conjugate (ADC) according to any of [7] to [24], wherein p is 3 to 8.
[26] A pharmaceutical composition comprising the antibody-drug conjugate (ADC) according to any of [7] to [25] and a carrier.
[27] An antitumor agent comprising, as an active ingredient, the antibody-drug conjugate (ADC) according to any of [7] to [25].
[28] The antitumor agent according to [27], which is used for treating cancer that expresses sulfated glycosaminoglycan.
[29] The antitumor agent according to [27], which is used for treating gastric cancer, pancreatic cancer, breast cancer, large bowel cancer, or lung cancer.
[30] A method for treating tumor comprising administering the antibody-drug conjugate (ADC) according to any of [7] to [25] to a patient.
[31] The method of treatment according to [30], wherein the tumor is cancer that expresses sulfated glycosaminoglycan.
[32] The method of treatment according to [30], wherein the tumor is gastric cancer, pancreatic cancer, breast cancer, large bowel cancer, or lung cancer.
[33] The antibody-drug conjugate (ADC) according to any of [7] to [25], which is used for treating tumor.
[34] The antibody-drug conjugate (ADC) according to [33], wherein the tumor is cancer that expresses sulfated glycosaminoglycan.
[35] The antibody-drug conjugate (ADC) according to [33], wherein the tumor is gastric cancer, pancreatic cancer, breast cancer, large bowel cancer, or lung cancer.
[36] Use of the antibody-drug conjugate (ADC) according to any of [7] to [25] for producing an anti-tumor agent.
[37] The use according to [36], wherein the tumor is cancer that expresses sulfated glycosaminoglycan.
[38] The use according to [36], wherein the tumor is gastric cancer, pancreatic cancer, breast cancer, large bowel cancer, or lung cancer.

The description incorporates the contents disclosed by JP Patent Application No. 2022-057542, based on which the priority of the present application claims.

Advantageous Effects of Invention

The antibody of the present invention binds specifically to sulfated glycosaminoglycan that are present in tumor tissue in a pH-dependent manner. Such antibody is useful not only as a regent for detecting sGAG but it is also useful as an antibody component of an antibody-drug conjugate (ADC). The antibody-drug conjugate (ADC) of the present invention has little side effects on healthy tissue and exerts excellent anti-tumor effects because of the pH dependence of the antibody.

Brief Description of Drawings

[Figure 1] Figure 1 shows pH-dependent binding activity of sGAG antibodies.

[Figure 2] Figure 2 demonstrates that pH-dependent binding of sGAG antibodies depends on target antigens due to inhibition of sGAG formation.

[Figure 3] Figure 3 shows target expression in healthy cells and in cancer cells using Antibody 1.

[Figure 4-1] Figure 4-1 shows antibody-linker-payload combinations in ADCs (part 1).

[Figure 4-2] Figure 4-2 shows antibody-linker-payload combinations in ADCs (part 2).

[Figure 5] Figure 5 shows 50% effective concentrations of antibodies and ADCs on NUGC-4 cells relative to the maximal binding activity at pH 6.0 designated as 100%.

[Figure 6] Figure 6 shows stability of drug linkers of ADCs in mouse and human plasma samples.

[Figure 7] Figure 7 shows cytotoxic effects of Antibody 1 to Antibody 3 and ADC1 to ADC3 on NUGC-3.

[Figure 8] Figure 8 shows cytotoxic effects of ADC4 to ADC10 on NUGC-4 and Capan-1.

[Figure 9-1] Figure 9-1 shows anti-tumor effects of ADC6 on mouse models of subcutaneous transplantation of the human pancreatic cancer cell line BxPC-3.

[Figure 9-2] Figure 9-2 shows anti-tumor effects of ADC6 on mouse models of subcutaneous transplantation of the human scirrhous gastric cancer cell line NUGC-4.

[Figure 10] Figure 10 shows sequences of SEQ ID NOs: 1 to 4 and 6 to 10.

[Figure 11] Figure 11 shows sequences of SEQ ID NOs: 12 to 16 and 18 to 20.

[Figure 12] Figure 12 shows sequences of SEQ ID NOs: 21 to 26.

[Figure 13] Figure 13 shows sequences of SEQ ID NOs: 27 to 32.

[Figure 14] Figure 14 shows sequences of SEQ ID NOs: 33 to 36.

[Figure 15] Figure 15 shows sequences of SEQ ID NOs: 37 to 39.

[Figure 16] Figure 16 shows sequences of SEQ ID NOs: 40 to 42.

[Figure 17] Figure 17 shows sequences of SEQ ID NOs: 43 to 45.

[Figure 18] Figure 18 shows sequences of SEQ ID NOs: 46 to 48.

[Figure 19] Figure 19 shows sequences of SEQ ID NOs: 49 and 50.

Description of Embodiments

Hereafter, the present invention is described in detail.

The present invention provides an antibody against sulfated glycosaminoglycan and an antibody-drug conjugate (ADC) comprising such antibody and a drug used for treating cancer.

<Antibody>

The present invention provides an antibody that binds to sulfated glycosaminoglycan with high affinity in an acidic pH range.

The antibody of the present invention binds to sulfated glycosaminoglycan with high affinity in an acidic pH range. Thus, the antibody of the present invention is useful as a reagent for detection, diagnosis, isolation, or the like of sulfated glycosaminoglycan under acidic pH conditions. Because of the pH-dependent tumor-tissue selectivity, the antibody of the present invention is useful as an antibody component of an antibody-drug conjugate (ADC) with antitumor activity.

The term "sulfated glycosaminoglycan" used herein refers to glycosaminoglycan that has received sulfation modification in a part of constitutive disaccharide units. Types of constitutive disaccharides (e.g., hyaluronic acid, chondroitin sulfate, dermatan sulfate, keratan sulfate, heparan sulfate, or heparin), degrees of sulfation, or sites of sulfation are not particularly limited.

When an antibody "binds with high affinity in an acidic pH range" herein, an antibody binds with higher affinity in an acidic pH range than in a neutral pH range. Specifically, the affinity when the antibody of the present invention binds to sGAG in an acidic pH range (5.0 to 6.8, preferably 5.5 to 6.4, and more preferably 6.0) is 4 times or greater, preferably 8 times or greater, and more preferably 10 times or greater than the affinity in a pH (7.4) region in the blood. The affinity when the antibody of the present invention binds to sGAG can be measured as the Alexa Fluor 488 signal (mean fluorescence intensity (MFI)) in accordance with a conventional technique, such as flow cytometry as described in Example 2 below. An antibody exhibiting binding affinity varying in accordance with pH levels is referred to as a pH-dependent antibody.

The antibody of the present invention has the ability of internalization, it binds to sulfated glycosaminoglycan in tumor tissue, and it is incorporated into the tumor cell.

The antibody according to the present invention is at least one antibody selected from the group consisting of (1)

to (3) below.

(1) An antibody comprising

a heavy chain that comprises a complementarity determining region (CDR) 1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 and
a light chain that comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6.

(2) An antibody comprising

a heavy chain that comprises CDR1 consisting of the amino acid sequence represented by SEQ ID NO: 7, CDR2 consisting of the amino acid sequence represented by SEQ ID NO: 8, and CDR3 consisting of the amino acid sequence represented by SEQ ID NO: 9 and
a light chain that comprises CDR1 consisting of the amino acid sequence represented by SEQ ID NO: 10, CDR2 consisting of the amino acid sequence represented by AAS, and CDR3 consisting of the amino acid sequence represented by SEQ ID NO: 12.

(3) An antibody comprising

a heavy chain that comprises CDR1 consisting of the amino acid sequence represented by SEQ ID NO: 13, CDR2 consisting of the amino acid sequence represented by SEQ ID NO: 14, and CDR3 consisting of the amino acid sequence represented by SEQ ID NO: 15 and
a light chain that comprises CDR1 consisting of the amino acid sequence represented by SEQ ID NO: 16, CDR2 consisting of the amino acid sequence represented by WAS, and CDR3 consisting of the amino acid sequence represented by SEQ ID NO: 18.

Among them, (1) an antibody comprising

a heavy chain that comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 and
a light chain that comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 is preferable.

A preferable antibody of the present invention is at least one antibody selected from the group consisting of (1) to (4) below:

(1) an antibody comprising a heavy chain comprising a variable region that consists of the amino acid sequence represented by SEQ ID NO: 20 and a light chain comprising a variable region that consists of the amino acid sequence represented by SEQ ID NO: 22;
(2) an antibody comprising a heavy chain comprising a variable region that consists of the amino acid sequence represented by SEQ ID NO: 24 and a light chain comprising a variable region that consists of the amino acid sequence represented by SEQ ID NO: 26;
(3) an antibody comprising a heavy chain comprising a variable region that consists of the amino acid sequence represented by SEQ ID NO: 28 and a light chain comprising a variable region that consists of the amino acid sequence represented by SEQ ID NO: 30; and
(4) an antibody comprising a heavy chain comprising a variable region that consists of the amino acid sequence represented by SEQ ID NO: 32 and a light chain comprising a variable region that consists of the amino acid sequence represented by SEQ ID NO: 34.

A more preferable antibody is:

(1) an antibody comprising a heavy chain comprising a variable region that consists of the amino acid sequence represented by SEQ ID NO: 20 and a light chain comprising a variable region that consists of the amino acid sequence

represented by SEQ ID NO: 22; or

(4) an antibody comprising a heavy chain comprising a variable region that consists of the amino acid sequence represented by SEQ ID NO: 32 and a light chain comprising a variable region that consists of the amino acid sequence represented by SEQ ID NO: 34.

A particularly preferable antibody is:

(4) an antibody comprising a heavy chain comprising a variable region that consists of the amino acid sequence represented by SEQ ID NO: 32 and a light chain comprising a variable region that consists of the amino acid sequence represented by SEQ ID NO: 34.

The heavy chain variable region includes not only the heavy chain variable region that consists of the amino acid sequence represented by the sequence identification number indicated above but also a heavy chain variable region that consists of a protein consisting of an amino acid sequence derived from the amino acid sequence in a region other than the CDR in the amino acid sequence indicated above by deletion, substitution, or addition of 1 or several, such as 1 to 10, preferably 1 to 5, more preferably 1 or 2, and further preferably 1 amino acid and having activity of an antibody heavy chain variable region; i.e., activity of binding to sulfated glycosaminoglycan, is within the scope of the heavy chain variable region of the present invention. The light chain variable region includes not only the light chain variable region that consists of the amino acid sequences represented by the sequence identification number indicated above but also a light chain variable region that consists of a protein consisting of an amino acid sequence derived from the amino acid sequence in a region other than the CDR in the amino acid sequence by deletion, substitution, or addition of 1 or several, such as 1 to 10, preferably 1 to 5, more preferably 1 or 2, and further preferably 1 amino acid and having activity of an antibody light chain variable region; i.e., activity of binding to sulfated glycosaminoglycan, is within the scope of the light chain variable region of the present invention. Whether or not an antibody of interest has activity of binding to sulfated glycosaminoglycan can be determined by a method known in the art.

An example of an amino acid sequence derived from the amino acid sequence represented by the sequence identification number indicated above by deletion, substitution, or addition of 1 or several amino acids is an amino acid sequence exhibiting at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence of a region other than CDR of the amino acid sequence represented by the sequence identification number indicated above, which is calculated with the use of, for example, BLAST (Basic Local Alignment Search Tool) at the National Center for Biological Information (e.g., default; i.e., initial parameters).

A protein comprising an amino acid sequence derived from the amino acid sequence represented by the sequence identification number indicated above by deletion, substitution, or addition of 1 or several amino acids is substantially identical to the protein consisting of the amino acid sequence represented by the sequence identification number indicated above.

A preferable antibody of the present invention is at least one antibody selected from the group consisting of (1) to (4) below:

(1) an antibody comprising

a heavy chain comprising a variable region that consists of the amino acid sequence represented by SEQ ID NO: 20 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 20 and has activity of binding to sulfated glycosaminoglycan and

a light chain comprising a variable region that consists of the amino acid sequence represented by SEQ ID NO: 22 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 22;

(2) an antibody comprising

a heavy chain comprising a variable region that consists of the amino acid sequence represented by SEQ ID NO: 24 and comprises CDR1 consisting of the amino acid sequence represented by SEQ ID NO: 7, CDR2 consisting of the amino acid sequence represented by SEQ ID NO: 8, and CDR3 consisting of the amino acid sequence represented by SEQ ID NO: 9 or a heavy chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 24 and has activity of binding to sulfated glycosaminoglycan and

a light chain comprising a variable region that consists of the amino acid sequence represented by SEQ ID NO: 26 and comprises CDR1 consisting of the amino acid sequence represented by SEQ ID NO: 10, CDR2 consisting of the amino acid sequence represented by AAS, and CDR3 consisting of the amino acid sequence represented by SEQ ID NO: 12 or a light chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 26 and has activity of binding to sulfated glycosaminoglycan;

(3) an antibody comprising

a heavy chain comprising a variable region that consists of the amino acid sequence represented by SEQ ID NO: 28 and comprises CDR1 consisting of the amino acid sequence represented by SEQ ID NO: 13, CDR2 consisting of the amino acid sequence represented by SEQ ID NO: 14, and CDR3 consisting of the amino acid sequence represented by SEQ ID NO: 15 or a heavy chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 28 and has activity of binding to sulfated glycosaminoglycan and

a light chain comprising a variable region that consists of the amino acid sequence represented by SEQ ID NO: 30 and comprises CDR1 consisting of the amino acid sequence represented by SEQ ID NO: 16, CDR2 consisting of the amino acid sequence represented by WAS, and CDR3 consisting of the amino acid sequence represented by SEQ ID NO: 18 or a light chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 30 and has activity of binding to sulfated glycosaminoglycan; and

(4) an antibody comprising

a heavy chain comprising a variable region that consists of the amino acid sequence represented by SEQ ID NO: 32 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 32 and has activity of binding to sulfated glycosaminoglycan and

a light chain comprising a variable region that consists of the amino acid sequence represented by SEQ ID NO: 34 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 34 and has activity of binding to sulfated glycosaminoglycan.

Among them, a more preferable antibody is:

(1) an antibody comprising

a heavy chain comprising a variable region that consists of the amino acid sequence represented by SEQ ID NO: 20 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 20 and has activity of binding to sulfated glycosaminoglycan and
a light chain comprising a variable region that consists of the amino acid sequence represented by SEQ ID NO: 22 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 22; or

(4) an antibody comprising

a heavy chain comprising a variable region that consists of the amino acid sequence represented by SEQ ID NO: 32 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 32 and has activity of binding to sulfated glycosaminoglycan and
a light chain comprising a variable region that consists of the amino acid sequence represented by SEQ ID NO: 34 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 34 and has activity of binding to sulfated glycosaminoglycan.

A further preferable antibody is:
(4) an antibody comprising

a heavy chain comprising a variable region that consists of the amino acid sequence represented by SEQ ID NO: 32 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 32 and has activity of binding to sulfated glycosaminoglycan and
a light chain comprising a variable region that consists of the amino acid sequence represented by SEQ ID NO: 34 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 34 and has activity of binding to sulfated glycosaminoglycan.

A further preferable antibody is:
(4) an antibody comprising

a heavy chain comprising a variable region that consists of the amino acid sequence represented by SEQ ID NO: 32 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain comprising a variable region that has at least 90% sequence identity to the amino acid sequence represented by SEQ ID NO: 32 and has activity of binding to sulfated glycosaminoglycan and
a light chain comprising a variable region that consists of the amino acid sequence represented by SEQ ID NO: 34 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain comprising a variable region that has at least 90% sequence identity to the amino acid sequence represented by SEQ ID NO: 34 and has activity of binding to sulfated glycosaminoglycan.

A particularly preferable antibody is:
(4) an antibody comprising

a heavy chain comprising a variable region that consists of the amino acid sequence represented by SEQ ID NO: 32 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain comprising a variable region that has at least 95% sequence identity to the amino acid sequence represented by SEQ ID NO: 32 and has activity of binding to sulfated glycosaminoglycan and
a light chain comprising a variable region that consists of the amino acid sequence represented by SEQ ID NO: 34 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain comprising a variable region that has at least 95% sequence identity to the amino acid sequence represented by SEQ ID NO: 34 and has activity of binding to sulfated glycosaminoglycan.

A preferable antibody of the present invention is at least one antibody selected from the group consisting of (1) to (4) below:

(1) an antibody comprising a heavy chain consisting of the amino acid sequence represented by SEQ ID NO: 36 and a light chain consisting of the amino acid sequence represented by SEQ ID NO: 38 (Antibody 1);
(2) an antibody comprising a heavy chain consisting of the amino acid sequence represented by SEQ ID NO: 40 and a light chain consisting of the amino acid sequence represented by SEQ ID NO: 42 (Antibody 2);
(3) an antibody comprising a heavy chain consisting of the amino acid sequence represented by SEQ ID NO: 44 and a light chain consisting of the amino acid sequence represented by SEQ ID NO: 46 (Antibody 3); and
(4) an antibody comprising a heavy chain consisting of the amino acid sequence represented by SEQ ID NO: 48 and a light chain consisting of the amino acid sequence represented by SEQ ID NO: 50 (Antibody 4).

Among them, a more preferable antibody is:

(1) an antibody comprising a heavy chain consisting of the amino acid sequence represented by SEQ ID NO: 36 and a light chain consisting of the amino acid sequence represented by SEQ ID NO: 38 (Antibody 1); or
(4) an antibody comprising a heavy chain consisting of the amino acid sequence represented by SEQ ID NO: 48 and a light chain consisting of the amino acid sequence represented by SEQ ID NO: 50 (Antibody 4).

From the viewpoint of high production efficiency and low aggregation properties, a particularly preferable antibody is:
(4) an antibody comprising a heavy chain consisting of the amino acid sequence represented by SEQ ID NO: 48 and a light chain consisting of the amino acid sequence represented by SEQ ID NO: 50 (Antibody 4).
The heavy chain includes not only the heavy chain consisting of the amino acid sequence represented by the sequence identification number indicated above but also a heavy chain consisting of a protein consisting of an amino acid sequence derived from the amino acid sequence in a region other than the CDR in the amino acid sequence indicated above by deletion, substitution, or addition of 1 or several, such as 1 to 10, preferably 1 to 5, more preferably 1 or 2, and further preferably 1 amino acid and having activity of an antibody heavy chain; i.e., activity of binding to sulfated glycosaminoglycan, is within the scope of the heavy chain of the present invention. The light chain includes not only the light chain consisting of the amino acid sequences represented by the sequence identification number indicated above but also a light chain consisting of a protein consisting of an amino acid sequence derived from the amino acid sequence in a region other than the CDR in the amino acid sequence by deletion, substitution, or addition of 1 or several, such as 1 to 10, preferably 1 to 5, more preferably 1 or 2, and further preferably 1 amino acid and having activity of an antibody light chain;

i.e., activity of binding to sulfated glycosaminoglycan, is within the scope of the light chain of the present invention. Whether or not an antibody of interest has activity of binding to sulfated glycosaminoglycan can be determined by a method known in the art.

An example of an amino acid sequence derived from the amino acid sequence represented by the sequence identification number indicated above by deletion, substitution, or addition of 1 or several amino acids is an amino acid sequence exhibiting at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence of a region other than CDR of the amino acid sequence represented by the sequence identification number indicated above, which is calculated with the use of, for example, BLAST (Basic Local Alignment Search Tool at the National Center for Biological Information (e.g., default; i.e., initial parameters).

A preferable antibody of the present invention is at least one antibody selected from the group consisting of (1) to (4) below:

(1) an antibody comprising

a heavy chain that consists of the amino acid sequence represented by SEQ ID NO: 36 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain that has at least 85%, further preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 36 and has activity of binding to sulfated glycosaminoglycan and

a light chain that consists of the amino acid sequence represented by SEQ ID NO: 38 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain having at least 85%, further preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 38 and having activity of binding to sulfated glycosaminoglycan;

(2) an antibody comprising

a heavy chain that consists of the amino acid sequence represented by SEQ ID NO: 40 and comprises CDR1 consisting of the amino acid sequence represented by SEQ ID NO: 7, CDR2 consisting of the amino acid sequence represented by SEQ ID NO: 8, and CDR3 consisting of the amino acid sequence represented by SEQ ID NO: 9 or a heavy chain that has at least 85%, further preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 40 and has activity of binding to sulfated glycosaminoglycan and

a light chain that consists of the amino acid sequence represented by SEQ ID NO: 42 and comprises CDR1 consisting of the amino acid sequence represented by SEQ ID NO: 10, CDR2 consisting of the amino acid sequence represented by AAS, and CDR3 consisting of the amino acid sequence represented by SEQ ID NO: 12 or a light chain that has at least 85%, further preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 42 and has activity of binding to sulfated glycosaminoglycan;

(3) an antibody comprising

a heavy chain that consists of the amino acid sequence represented by SEQ ID NO: 44 and comprises CDR1 consisting of the amino acid sequence represented by SEQ ID NO: 13, CDR2 consisting of the amino acid sequence represented by SEQ ID NO: 14, and CDR3 consisting of the amino acid sequence represented by SEQ ID NO: 15 or a heavy chain that has at least 85%, further preferably at least 90%, further preferably at least 91%,

further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 44 and has activity of binding to sulfated glycosaminoglycan and
a light chain that consists of the amino acid sequence represented by SEQ ID NO: 46 and comprises CDR1 consisting of the amino acid sequence represented by SEQ ID NO: 16, CDR2 consisting of the amino acid sequence represented by WAS, and CDR3 consisting of the amino acid sequence represented by SEQ ID NO: 18 or a light chain that has at least 85%, further preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 46 and having activity of binding to sulfated glycosaminoglycan; and

(4) an antibody comprising

a heavy chain that consists of the amino acid sequence represented by SEQ ID NO: 48 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain that has at least 85%, further preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 48 and has activity of binding to sulfated glycosaminoglycan and
a light chain that consists of the amino acid sequence represented by SEQ ID NO: 50 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain that has at least 85%, further preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 50 and has activity of binding to sulfated glycosaminoglycan.

A more preferable antibody is:

(1) an antibody comprising

a heavy chain that consists of the amino acid sequence represented by SEQ ID NO: 36 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain that has at least 85%, further preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 36 and has activity of binding to sulfated glycosaminoglycan and
a light chain that consists of the amino acid sequence represented by SEQ ID NO: 38 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain that has at least 85%, further preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 38 and having activity of binding to sulfated glycosaminoglycan; or

(4) an antibody comprising

a heavy chain that consists of the amino acid sequence represented by SEQ ID NO: 48 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ

ID NO: 3 or a heavy chain that has at least 85%, further preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 48 and has activity of binding to sulfated glycosaminoglycan and
a light chain that consists of the amino acid sequence represented by SEQ ID NO: 50 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain that has at least 85%, further preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 50 and has activity of binding to sulfated glycosaminoglycan.

A further preferable antibody is:
(4) an antibody comprising

a heavy chain that consists of the amino acid sequence represented by SEQ ID NO: 48 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain that has at least 85%, further preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 48 and has activity of binding to sulfated glycosaminoglycan and
a light chain that consists of the amino acid sequence represented by SEQ ID NO: 50 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain that has at least 85%, further preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 50 and has activity of binding to sulfated glycosaminoglycan.

A further preferable antibody is:
(4) an antibody comprising

a heavy chain that consists of the amino acid sequence represented by SEQ ID NO: 48 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain that has at least 95% sequence identity to the amino acid sequence represented by SEQ ID NO: 48 and has activity of binding to sulfated glycosaminoglycan and
a light chain that consists of the amino acid sequence represented by SEQ ID NO: 50 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain that has at least 95% sequence identity to the amino acid sequence represented by SEQ ID NO: 50 and has activity of binding to sulfated glycosaminoglycan.

A particularly preferable antibody is:
(4) an antibody comprising

a heavy chain that consists of the amino acid sequence represented by SEQ ID NO: 48 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain that has at least 98% sequence identity to the amino acid sequence represented by SEQ ID NO: 48 and has activity of binding to sulfated glycosaminoglycan and
a light chain that consists of the amino acid sequence represented by SEQ ID NO: 50 and comprises CDR1 consisting of the amino acid sequence represented by SEQ ID NO: 4, CDR2 consisting of the amino acid sequence represented

by LGS, and CDR3 consisting of the amino acid sequence represented by SEQ ID NO: 6 or a light chain that has at least 98% sequence identity to the amino acid sequence represented by SEQ ID NO: 50 and has activity of binding to sulfated glycosaminoglycan.

A protein comprising an amino acid sequence derived from the amino acid sequence represented by the sequence identification number indicated above by deletion, substitution, or addition of 1 or several amino acids is substantially identical to the protein consisting of the amino acid sequence represented by the sequence identification number indicated above.

Table 1 shows the sequence information of the preferable antibodies of the present invention: "Antibody 1," "Antibody 2," "Antibody 3," and "Antibody 4."

[Table 1]

| | | CDR1 | CDR2 | CDR3 | Variable region | Full-length |
|---|---|---|---|---|---|---|
| Antibody 1 | IgH | GGFITSHH (SEQ ID NO: 1) | LHHTGTT (SEQ ID NO: 2) | VREDSSSWHPGRYIQL (SEQ ID NO: 3) | Nucleotide sequence: SEQ ID NO: 19; amino acid sequence: SEQ ID NO: 20 | Nucleotide sequence: SEQ ID NO: 35; amino acid sequence: SEQ ID NO: 36 |
| | IgK | QSLLQNNRYNR (SEQ ID NO: 4) | LGS | MHALEPPYT (SEQ ID NO: 6) | Nucleotide sequence: SEQ ID NO: 21; amino acid sequence: SEQ ID NO: 22 | Nucleotide sequence: SEQ ID NO: 37; amino acid sequence: SEQ ID NO: 38 |
| Antibody 2 | IgH | GFTFNTYT (SEQ ID NO: 7) | ISLSSNTI (SEQ ID NO: 8) | ARAESYYDSHYYYNGLDV (SEQ ID NO: 9) | Nucleotide sequence: SEQ ID NO: 23; amino acid sequence: SEQ ID NO: 24 | Nucleotide sequence: SEQ ID NO: 39; amino acid sequence: SEQ ID NO: 40 |
| | IgK | QSVGGRY (SEQ ID NO: 10) | AAS | QQYGSSPRT (SEQ ID NO: 12) | Nucleotide sequence: SEQ ID NO: 25; amino acid sequence: SEQ ID NO: 26 | Nucleotide sequence: SEQ ID NO: 41; amino acid sequence: SEQ ID NO: 42 |

(continued)

| | | CDR1 | CDR2 | CDR3 | Variable region | Full-length |
|---|---|---|---|---|---|---|
| Antibody 3 | IgH | GFTFSDYY (SEQ ID NO: 13) | ISISGTTR (SEQ ID NO: 14) | ARGPELLSQNYYYY YGMDV (SEQ ID NO: 15) | Nucleotide sequence: SEQ ID NO: 27; amino acid sequence: SEQ ID NO: 28 | Nucleotide sequence: SEQ ID NO: 43; amino acid sequence: SEQ ID NO: 44 |
| | IgK | ETLLYRSNNKN Y (SEQ ID NO: 16) | WAS | QQYYTTPRT (SEQ ID NO: 18) | Nucleotide sequence: SEQ ID NO: 29; amino acid sequence: SEQ ID NO: 30 | Nucleotide sequence: SEQ ID NO: 45; amino acid sequence: SEQ ID NO: 46 |
| Antibody 4 | IgH | GGFITSHH (SEQ ID NO: 1) | LHHTGTT (SEQ ID NO: 2) | VREDSSSWHPGRYI QL (SEQ ID NO: 3) | Nucleotide sequence: SEQ ID NO: 31; amino acid sequence: SEQ ID NO: 32 | Nucleotide sequence: SEQ ID NO: 47; amino acid sequence: SEQ ID NO: 48 |
| | IgK | QSLLQNNRYNR (SEQ ID NO: 4) | LGS | MHALEPPYT (SEQ ID NO: 6) | Nucleotide sequence: SEQ ID NO: 33; amino acid sequence: SEQ ID NO: 34 | Nucleotide sequence: SEQ ID NO: 49; amino acid sequence: SEQ ID NO: 50 |

The form of the antibody of the present invention is not particularly limited, provided that such antibody is a molecule that comprises a pair of a heavy chain and a light chain and can bind to an antigen. Examples thereof include immunoglobulin and an antigen-binding fragment or derivative of a complete antibody having the whole or some functions of the complete antibody (e.g., a Fab fragment, a $F(ab')_2$ fragment, a single-stranded antibody fragment (scFv), a variable region (Fv), or Fab' iabody, which is a monovalent fragment of an antibody variable region obtained by treating $dF(ab')_2$ under reducing conditions, and a bispecific antibody).

The origin of the antibody of the present invention is not particularly limited, and examples include humans, rats, mice, rabbits, hamsters, guinea pigs, horses, monkeys, dogs, pigs, cows, goats, and sheep. The antibody of the present invention is preferably a recombinant monoclonal antibody. When the antibody is derived from a species other than the human, the antibody can be chimerized or humanized in accordance with a well-known technique. A chimerized antibody has a human constant region, and a humanized antibody is prepared by substituting a complementary determining region (CDR) of a human antibody with CDR of an anti-sulfated glycosaminoglycan antibody derived from an animal other than a human (i.e., a CDR-transplanted antibody). In addition, a human antibody, which is an expression product of a human-derived antibody gene, is within the scope of the present invention. The antibody of the present invention is preferably a human antibody, a humanized antibody, or a chimeric antibody, with a human antibody being more preferable. When the antibody of the present invention is a human antibody, the subtype thereof is not particularly limited. The subtype is preferably IgG1, IgG2, IgG3, or IgG4, with IgG1 being more preferable. Examples of heavy chain constant regions include $C\gamma1$, $C\gamma2$, $C\gamma3$, and $C\gamma4$, and examples of light chain constant regions include $C\kappa$ and $C\lambda$.

The antibody of the present invention can be prepared by any of various known methods, and a method for preparing the same is not particularly limited. Examples of known methods include the hybridoma method and the phage display method.

The antibody of the present invention can be modified by a known recombination technique, provided that the effects of the present invention are not adversely affected. For example, at least one amino acid in an antibody constant

region can be substituted with a different residue. Such modification is provided to reduce undesirable activity, such as complement-dependent cytotoxicity.

The antibody of the present invention can be prepared as a recombinant antibody. Specifically, DNA encoding each antibody region (DNA encoding a heavy chain variable region, a light chain variable region, a heavy chain variable region, or a variable chain constant region) is inserted into an expression vector, a host cell is transformed using the expression vector, and the cell is cultured to prepare the antibody of the present invention. In such a case, DNA comprising DANs each encoding a relevant region ligated to each other may be operably linked to an element, such as a promoter, polyadenylation signal, or enhancer. DNA is operably linked, so that each element can function. DNA comprising a region inserted into a vector may comprise a signal peptide that accelerates antibody secretion from a host cell. After the antibody is produced, a signal peptide may be removed, so that a mature protein can be obtained. As an expression vector, a known plasmid, phage, or the like can be used. As a host cell, a prokaryotic cell, such as an *E. coli* or *Bacillus subtilis* cell, or an eukaryotic cell, such as a yeast or animal cell, can be used. In particular, use of an eukaryotic cell with a sugar chain binding to an antibody is preferable. Examples of preferable animal cells that can be used include Chinese hamster ovarian (CHO) cells and HEK293 cells. An expression vector may be introduced into a host cell by electroporation, DEAE-dextran transfection, calcium phosphate precipitation, or other means to transform the host cell. The prepared antibody may be purified by, for example, a known method involving the use of chromatography. A lysine residue at the carboxyl terminus of a heavy chain of an antibody prepared in a cultured mammalian cell is known to be deleted, and an antibody lacking a lysine residue at the carboxyl terminus of a heavy chain is within the scope of the present invention.

<Antibody-drug conjugate (ADC)>

The present invention provides an antibody-drug conjugate (ADC) represented by Formula 1:

Ab-(L-D)p

wherein
Ab represents an antibody binding specifically to sulfated glycosaminoglycan, which is at least one antibody selected from the group consisting of (1) to (3) below:

(1) an antibody comprising

a heavy chain that comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 and
a light chain that comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6;

(2) an antibody comprising

a heavy chain that comprises CDR1 consisting of the amino acid sequence represented by SEQ ID NO: 7, CDR2 consisting of the amino acid sequence represented by SEQ ID NO: 8, and CDR3 consisting of the amino acid sequence represented by SEQ ID NO: 9 and
a light chain that comprises CDR1 consisting of the amino acid sequence represented by SEQ ID NO: 10, CDR2 consisting of the amino acid sequence represented by AAS, and CDR3 consisting of the amino acid sequence represented by SEQ ID NO: 12; and

(3) an antibody comprising

a heavy chain that comprises CDR1 consisting of the amino acid sequence represented by SEQ ID NO: 13, CDR2 consisting of the amino acid sequence represented by SEQ ID NO: 14, and CDR3 consisting of the amino acid sequence represented by SEQ ID NO: 15 and
a light chain that comprises CDR1 consisting of the amino acid sequence represented by SEQ ID NO: 16, CDR2 consisting of the amino acid sequence represented by WAS, and CDR3 consisting of the amino acid sequence represented by SEQ ID NO: 18.

In the formula above,

L represents 6-maleimidocaproyl-valine-citrulline-p-aminobenzyloxycarbonyl (MC-Val-Cit-PAB), 6-maleimidocaproyl-glycine-glycine-phenylalanine-glycine (MC-GGFG), N-succinimidyl 4-(N-maleimidomethyl)cyclohexane-1-carboxylate (SMCC), 4-(2-pyridyldithio)butyric acid N-hydroxysuccinimide ester (SPDB), or MC-CL2;
D represents monomethyl auristatin, maytansinoid, or a camptothecin derivative; and
p is an integer selected from 1 to 12.

Ab in the ADC of the present invention is preferably the antibody described above.

L in the ADC of the present invention is a linker that connects an antibody to a drug, and it may or may not comprise an enzyme-cleaved site. Examples of linkers include 6-maleimidocaproyl-valine-citrulline-p-aminobenzyloxycarbonyl (MC-Val-Cit-PAB:MC-VC-PAB), 6-maleimidocaproyl-glycine-glycine-phenylalanine-glycine (MC-GGFG), N-succinimidyl 4-(N-maleimidomethyl)cyclohexane-1-carboxylate (SMCC), 4-(2-pyridyldithio)butyric acid N-hydroxysuccinimide ester (SPDB), and MC-CL2 (J. Med. Chem., November 13, 2008; 51 (21): 6916-6926). For example, Val-Cit (valine-citrulline) is a peptide linker comprising valine and citrulline bound to each other, and it is cleaved by a cysteine protease enzyme, such as cathepsin.

D in the ADC of the present invention is a drug, and examples thereof include anticancer agents, such as monomethyl auristatin (MMA), maytansinoid, and a camptothecin derivative. Examples of monomethyl auristatin include monomethyl auristatin E (MMAE), monomethyl auristatin D (MMAD), and monomethyl auristatin F (MMAF), with monomethyl auristatin E (MMAE) being preferable. Examples of maytansinoid include DM1 (N2'-deacetyl-N2'-(3-mercapto-1-oxopropyl)-maytansine) and DM4. Examples of camptothecin derivatives include Topotecan, Irinotecan, Dx-8951, Dx-d, and SN-38.

A preferable combination of L and D in the ADC of the present invention is SMCC-maytansinoid, SPDB-maytansinoid, MC-Val-Cit-PAB-monomethyl auristatin, MC-Val-Cit-PAB-monomethyl auristatin, an MC-GGFG-camptothecin derivative, or MC-CL2-camptothecin derivative. A more preferable combination thereof is SMCC-DM1, SPDB-DM4, MC-Val-Cit-PAB-MMAE, MC-Val-Cit-PAB-MMAF, MC-GGFG-Dx8591, or MC-CL2-SN-38. A further preferable combination thereof is SMCC-DM1, SPDB-DM4, MC-Val-Cit-PAB-MMAE, MC-Val-Cit-PAB-MMAF, or MC-GGFG-Dx8591. A furthermore preferable combination thereof is MC-Val-Cit-PAB-MMAE.

p in the ADC of the present invention is an integer selected from 1 to 12, preferably 1 to 10, more preferably 2 to 9, and particularly preferably 3 to 8.

The ADC of the present invention can be produced by a well-known method. In ADC, for example, a drug is bound to an antibody via a linker, and a linker is bound to an antibody via an amino or cysteine residue of an antibody. Specifically, such binding is performed by the Michael addition reaction between a sulfhydryl group and a maleimide group of cysteine, bond formation via a disulfide bond forming reaction, or amide bond formation by condensation with carboxylic acid to the lysine amino group. p in General formula 1 indicates the number of molecules constituting a drug that can bind to one molecule of an antibody, and the number of molecules is referred to as the drug antibody ratio (DAR).

A preferable ADC of the present invention is an ADC represented by Formula 1, wherein Ab represents an antibody that binds specifically to sulfated glycosaminoglycan and is at least one antibody selected from the group consisting of (1) to (4) below:

(1) an antibody comprising a heavy chain comprising a variable region that consists of the amino acid sequence represented by SEQ ID NO: 20 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 20 and has activity of binding to sulfated glycosaminoglycan and a light chain comprising a variable region that consists of the amino acid sequence represented by SEQ ID NO: 22 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 22;
(2) an antibody comprising a heavy chain comprising a variable region that consists of the amino acid sequence represented by SEQ ID NO: 24 and comprises CDR1 consisting of the amino acid sequence represented by SEQ ID NO: 7, CDR2 consisting of the amino acid sequence represented by SEQ ID NO: 8, and CDR3 consisting of the amino acid sequence represented by SEQ ID NO: 9 or a heavy chain comprising a variable region that has at least 85%, preferably at least 90%, further preferably at least 91%, further preferably at least 92%, further preferably at least 93%,

further preferably at least 94%, further preferably at least 95%, further preferably at least 96%, further preferably at least 97%, further preferably at least 98%, and particularly preferably at least 99% sequence identity to the amino acid sequence represented by SEQ ID NO: 24 and has activity of binding to sulfated glycosaminoglycan and a light chain comprising a variable region that consists of the amino acid sequence represented by SEQ ID NO: 26 and comprises CDR1 consisting of the amino acid sequence represented by SEQ ID NO: 10, CDR2 consisting of the amino acid sequence represented by AAS, and CDR3 consisting of the amino acid sequence represented by SEQ ID NO: 12 or a light chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 26 and has activity of binding to sulfated glycosaminoglycan;

(3) an antibody comprising a heavy chain comprising a variable region that consists of the amino acid sequence represented by SEQ ID NO: 28 and comprises CDR1 consisting of the amino acid sequence represented by SEQ ID NO: 13, CDR2 consisting of the amino acid sequence represented by SEQ ID NO: 14, and CDR3 consisting of the amino acid sequence represented by SEQ ID NO: 15 or a heavy chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 28 and has activity of binding to sulfated glycosaminoglycan and a light chain comprising a variable region that consists of the amino acid sequence represented by SEQ ID NO: 30 and comprises CDR1 consisting of the amino acid sequence represented by SEQ ID NO: 16, CDR2 consisting of the amino acid sequence represented by WAS, and CDR3 consisting of the amino acid sequence represented by SEQ ID NO: 18 or a light chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 30 and has activity of binding to sulfated glycosaminoglycan; and

(4) an antibody comprising a heavy chain comprising a variable region that consists of the amino acid sequence represented by SEQ ID NO: 32 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 32 and has activity of binding to sulfated glycosaminoglycan and a light chain comprising a variable region that consists of the amino acid sequence represented by SEQ ID NO: 34 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 34 and has activity of binding to sulfated glycosaminoglycan.

In the formula indicated above,

L represents 6-maleimidocaproyl-valine-citrulline-p-aminobenzyloxycarbonyl (MC-Val-Cit-PAB), 6-maleimidocaproyl-glycine-glycine-phenylalanine-glycine (MC-GGFG), N-succinimidyl 4-(N-maleimidomethyl)cyclohexane-1-carboxylate (SMCC), 4-(2-pyridyldithio)butyric acid N-hydroxysuccinimide ester (SPDB), or MC-CL2;
D represents monomethyl auristatin, maytansinoid, or a camptothecin derivative; and
p is an integer selected from 1 to 12.

More preferably, ADC is represented by Formula 1, wherein Ab represents an antibody that binds specifically to sulfated glycosaminoglycan and is at least one antibody selected from the group consisting of (1) to (4) below:

(1) an antibody comprising a heavy chain comprising a variable region that consists of the amino acid sequence represented by SEQ ID NO: 20 and a light chain comprising a variable region that consists of the amino acid sequence represented by SEQ ID NO: 22;

(2) an antibody comprising a heavy chain comprising a variable region that consists of the amino acid sequence represented by SEQ ID NO: 24 and a light chain comprising a variable region that consists of the amino acid sequence represented by SEQ ID NO: 26;
(3) an antibody comprising a heavy chain comprising a variable region that consists of the amino acid sequence represented by SEQ ID NO: 28 and a light chain comprising a variable region that consists of the amino acid sequence represented by SEQ ID NO: 30; and
(4) an antibody comprising a heavy chain comprising a variable region that consists of the amino acid sequence represented by SEQ ID NO: 32 and a light chain comprising a variable region that consists of the amino acid sequence represented by SEQ ID NO: 34.

In the formula indicated above,

L represents 6-maleimidocaproyl-valine-citrulline-p-aminobenzyloxycarbonyl (MC-Val-Cit-PAB), 6-maleimidocaproyl-glycine-glycine-phenylalanine-glycine (MC-GGFG), N-succinimidyl 4-(N-maleimidomethyl)cyclohexane-1-carboxylate (SMCC), 4-(2-pyridyldithio)butyric acid N-hydroxysuccinimide ester (SPDB), or MC-CL2;
D represents monomethyl auristatin, maytansinoid, or a camptothecin derivative; and
p is an integer selected from 1 to 12.

More preferably, ADC is represented by Formula 1, wherein Ab represents an antibody that binds specifically to sulfated glycosaminoglycan and is at least one antibody selected from the group consisting of (1) to (4) below:

(1) an antibody comprising a heavy chain comprising a variable region that consists of the amino acid sequence represented by SEQ ID NO: 20 and a light chain comprising a variable region that consists of the amino acid sequence represented by SEQ ID NO: 22;
(2) an antibody comprising a heavy chain comprising a variable region that consists of the amino acid sequence represented by SEQ ID NO: 24 and a light chain comprising a variable region that consists of the amino acid sequence represented by SEQ ID NO: 26;
(3) an antibody comprising a heavy chain comprising a variable region that consists of the amino acid sequence represented by SEQ ID NO: 28 and a light chain comprising a variable region that consists of the amino acid sequence represented by SEQ ID NO: 30; and
(4) an antibody comprising a heavy chain comprising a variable region that consists of the amino acid sequence represented by SEQ ID NO: 32 and a light chain comprising a variable region that consists of the amino acid sequence represented by SEQ ID NO: 34.

In the formula indicated above,

L-D represents SMCC-DM1, SPDB-DM4, MC-Val-Cit-PAB-MMAE, MC-Val-Cit-PAB-MMAF, MC-GGFG-Dx8591, or MC-CL2-SN-38; and
p is an integer selected from 1 to 12.

More preferably, ADC is represented by Formula 1, wherein Ab represents an antibody that binds specifically to sulfated glycosaminoglycan and is at least one antibody selected from the group consisting of (1) to (4) below:

(1) an antibody comprising a heavy chain comprising a variable region that consists of the amino acid sequence represented by SEQ ID NO: 20 and a light chain comprising a variable region that consists of the amino acid sequence represented by SEQ ID NO: 22;
(2) an antibody comprising a heavy chain comprising a variable region that consists of the amino acid sequence represented by SEQ ID NO: 24 and a light chain comprising a variable region that consists of the amino acid sequence represented by SEQ ID NO: 26;
(3) an antibody comprising a heavy chain comprising a variable region that consists of the amino acid sequence represented by SEQ ID NO: 28 and a light chain comprising a variable region that consists of the amino acid sequence represented by SEQ ID NO: 30; and
(4) an antibody comprising a heavy chain comprising a variable region that consists of the amino acid sequence represented by SEQ ID NO: 32 and a light chain comprising a variable region that consists of the amino acid sequence represented by SEQ ID NO: 34.

In the formula indicated above,

L-D represents SMCC-DM1, SPDB-DM4, MC-Val-Cit-PAB-MMAE, MC-Val-Cit-PAB-MMAF, or MC-GGFG-Dx8591; and
p is an integer selected from 1 to 12.

More preferably, ADC is represented by Formula 1, wherein Ab represents an antibody that binds specifically to sulfated glycosaminoglycan and is at least one antibody selected from the group consisting of (1) to (4) below:

(1) an antibody comprising a heavy chain consisting of the amino acid sequence represented by SEQ ID NO: 36 and a light chain consisting of the amino acid sequence represented by SEQ ID NO: 38 (Antibody 1);
(2) an antibody comprising a heavy chain consisting of the amino acid sequence represented by SEQ ID NO: 40 and a light chain consisting of the amino acid sequence represented by SEQ ID NO: 42 (Antibody 2);
(3) an antibody comprising a heavy chain consisting of the amino acid sequence represented by SEQ ID NO: 44 and a light chain consisting of the amino acid sequence represented by SEQ ID NO: 46 (Antibody 3); and
(4) an antibody comprising a heavy chain consisting of the amino acid sequence represented by SEQ ID NO: 48 and a light chain consisting of the amino acid sequence represented by SEQ ID NO: 50 (Antibody 4).

In the formula indicated above,

L-D represents SMCC-DM1, SPDB-DM4, MC-Val-Cit-PAB-MMAE, MC-Val-Cit-PAB-MMAF, or MC-GGFG-Dx8591; and
p is an integer selected from 1 to 12.

A preferable ADC of the present invention is represented by Formula 2 below:

[Chemical formula 1]

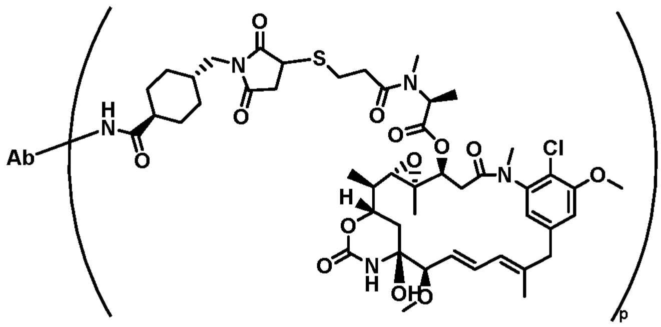

wherein Ab represents an antibody binding specifically to sulfated glycosaminoglycan, which is at least one antibody selected from the group consisting of (1) to (3) below:

(1) an antibody comprising a heavy chain that comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 and a light chain that comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6;
(2) an antibody comprising a heavy chain that comprises CDR1 consisting of the amino acid sequence represented by SEQ ID NO: 7, CDR2 consisting of the amino acid sequence represented by SEQ ID NO: 8, and CDR3 consisting of the amino acid sequence represented by SEQ ID NO: 9 and a light chain that comprises CDR1 consisting of the amino acid sequence represented by SEQ ID NO: 10, CDR2 consisting of the amino acid sequence represented by AAS, and CDR3 consisting of the amino acid sequence represented by SEQ ID NO: 12; and
(3) an antibody comprising a heavy chain that comprises CDR1 consisting of the amino acid sequence represented by SEQ ID NO: 13, CDR2 consisting of the amino acid sequence represented by SEQ ID NO: 14, and CDR3 consisting of the amino acid sequence represented by SEQ ID NO: 15 and a light chain that comprises CDR1 consisting of the amino acid sequence represented by SEQ ID NO: 16, CDR2 consisting of the amino acid sequence represented by WAS, and CDR3 consisting of the amino acid sequence represented by SEQ ID NO: 18, and
p is an integer selected from 1 to 12.

A further preferable ADC is represented by the formula indicated above, wherein Ab represents at least one antibody selected from the group consisting of (1) to (4) below:

(1) an antibody comprising a heavy chain comprising a variable region that consists of the amino acid sequence represented by SEQ ID NO: 20 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 20 and has activity of binding to sulfated glycosaminoglycan and a light chain comprising a variable region that consists of the amino acid sequence represented by SEQ ID NO: 22 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 22;
(2) an antibody comprising a heavy chain comprising a variable region that consists of the amino acid sequence represented by SEQ ID NO: 24 and comprises CDR1 consisting of the amino acid sequence represented by SEQ ID NO: 7, CDR2 consisting of the amino acid sequence represented by SEQ ID NO: 8, and CDR3 consisting of the amino acid sequence represented by SEQ ID NO: 9 or a heavy chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 24 and has activity of binding to sulfated glycosaminoglycan and a light chain comprising a variable region that consists of the amino acid sequence represented by SEQ ID NO: 26 and comprises CDR1 consisting of the amino acid sequence represented by SEQ ID NO: 10, CDR2 consisting of the amino acid sequence represented by AAS, and CDR3 consisting of the amino acid sequence represented by SEQ ID NO: 12 or a light chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 26 and has activity of binding to sulfated glycosaminoglycan;
(3) an antibody comprising a heavy chain comprising a variable region that consists of the amino acid sequence represented by SEQ ID NO: 28 and comprises CDR1 consisting of the amino acid sequence represented by SEQ ID NO: 13, CDR2 consisting of the amino acid sequence represented by SEQ ID NO: 14, and CDR3 consisting of the amino acid sequence represented by SEQ ID NO: 15 or a heavy chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 28 and has activity of binding to sulfated glycosaminoglycan and a light chain comprising a variable region that consists of the amino acid sequence represented by SEQ ID NO: 30 and comprises CDR1 consisting of the amino acid sequence represented by SEQ ID NO: 16, CDR2 consisting of the amino acid sequence represented by WAS, and CDR3 consisting of the amino acid sequence represented by SEQ ID NO: 18 or a light chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 30 and has activity of binding to sulfated glycosaminoglycan; and
(4) an antibody comprising a heavy chain comprising a variable region that consists of the amino acid sequence represented by SEQ ID NO: 32 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 32 and has activity of binding to sulfated glycosaminoglycan and a light chain

comprising a variable region that consists of the amino acid sequence represented by SEQ ID NO: 34 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 34 and has activity of binding to sulfated glycosaminoglycan; and
p is an integer selected from 1 to 12.

A further preferable ADC is represented by the formula indicated above, wherein Ab represents at least one antibody selected from the group consisting of (1) to (4) below:

(1) an antibody comprising a heavy chain comprising a variable region that consists of the amino acid sequence represented by SEQ ID NO: 20 and a light chain comprising a variable region that consists of the amino acid sequence represented by SEQ ID NO: 22;
(2) an antibody comprising a heavy chain comprising a variable region that consists of the amino acid sequence represented by SEQ ID NO: 24 and a light chain comprising a variable region that consists of the amino acid sequence represented by SEQ ID NO: 26;
(3) an antibody comprising a heavy chain comprising a variable region that consists of the amino acid sequence represented by SEQ ID NO: 28 and a light chain comprising a variable region that consists of the amino acid sequence represented by SEQ ID NO: 30; and
(4) an antibody comprising a heavy chain comprising a variable region that consists of the amino acid sequence represented by SEQ ID NO: 32 and a light chain comprising a variable region that consists of the amino acid sequence represented by SEQ ID NO: 34, and
p is an integer selected from 1 to 12.

A particularly preferable ADC is represented by the formula indicated above, wherein Ab represents at least one antibody selected from the group consisting of (1) to (4) below:

(1) an antibody comprising a heavy chain consisting of the amino acid sequence represented by SEQ ID NO: 36 and a light chain consisting of the amino acid sequence represented by SEQ ID NO: 38 (Antibody 1);
(2) an antibody comprising a heavy chain consisting of the amino acid sequence represented by SEQ ID NO: 40 and a light chain consisting of the amino acid sequence represented by SEQ ID NO: 42 (Antibody 2);
(3) an antibody comprising a heavy chain consisting of the amino acid sequence represented by SEQ ID NO: 44 and a light chain consisting of the amino acid sequence represented by SEQ ID NO: 46 (Antibody 3); and
(4) an antibody comprising a heavy chain consisting of the amino acid sequence represented by SEQ ID NO: 48 and a light chain consisting of the amino acid sequence represented by SEQ ID NO: 50 (Antibody 4), and
p is an integer selected from 1 to 12.

A preferable ADC of the present invention is represented by Formula 3 below:

[Chemical formula 2]

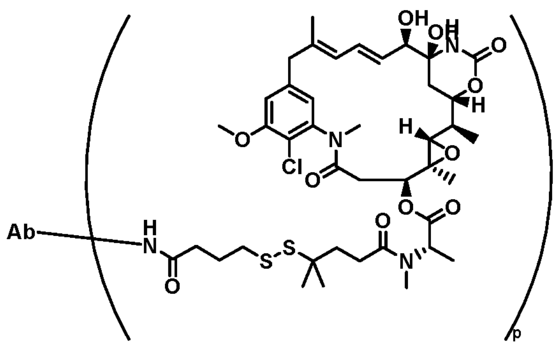

wherein Ab represents (1) an antibody comprising a heavy chain that comprises CDR1 consisting of the amino acid sequence represented by SEQ ID NO: 1, CDR2 consisting of the amino acid sequence represented by SEQ ID NO: 2, and

CDR3 consisting of the amino acid sequence represented by SEQ ID NO: 3 and a light chain that comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6, and
p is an integer selected from 1 to 12.

A further preferable ADC is represented by the formula indicated above, wherein Ab represents (4) an antibody comprising a heavy chain comprising a variable region that consists of the amino acid sequence represented by SEQ ID NO: 32 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 32 and has activity of binding to sulfated glycosaminoglycan and a light chain comprising a variable region that consists of the amino acid sequence represented by SEQ ID NO: 34 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 34 and has activity of binding to sulfated glycosaminoglycan; and
p is an integer selected from 1 to 12.

A further preferable ADC is represented by the formula indicated above, wherein Ab represents (4) an antibody comprising a heavy chain comprising a variable region that consists of the amino acid sequence represented by SEQ ID NO: 32 and a light chain comprising a variable region that consists of the amino acid sequence represented by SEQ ID NO: 34, and
p is an integer selected from 1 to 12.

A particularly preferable ADC is represented by the formula indicated above, wherein Ab represents (4) an antibody comprising a heavy chain consisting of the amino acid sequence represented by SEQ ID NO: 48 and a light chain consisting of the amino acid sequence represented by SEQ ID NO: 50 (Antibody 4), and
p is an integer selected from 1 to 12.

A preferable ADC of the present invention is represented by Formula 4 below:

[Chemical formula 3]

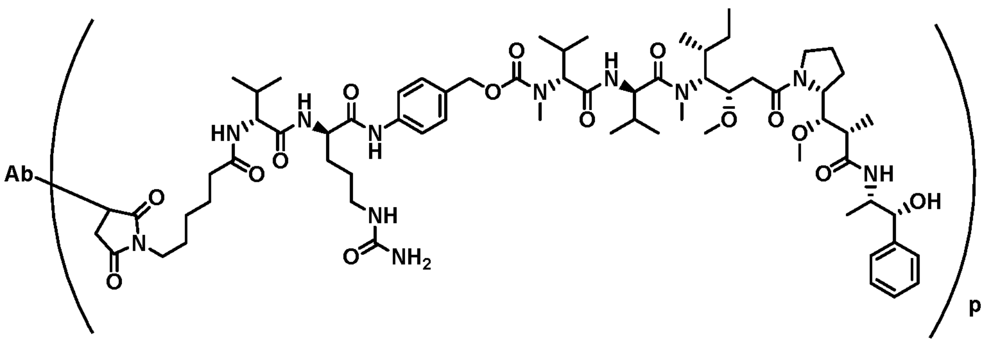

wherein Ab represents (1) an antibody comprising a heavy chain that comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 and a light chain that comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6, and
p is an integer selected from 1 to 12.

A further preferable ADC is represented by the formula indicated above, wherein Ab represents (4) an antibody comprising a heavy chain comprising a variable region that consists of the amino acid sequence represented by SEQ ID NO: 32 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain comprising a variable region that has at least 85%, preferably at least 90%, further preferably at least 91%, further preferably at least 92%, further preferably at least 93%, further preferably at least 94%,

further preferably at least 95%, further preferably at least 96%, further preferably at least 97%, further preferably at least 98%, and particularly preferably at least 99% sequence identity to the amino acid sequence represented by SEQ ID NO: 32 and has activity of binding to sulfated glycosaminoglycan and a light chain comprising a variable region that consists of the amino acid sequence represented by SEQ ID NO: 34 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 34 and has activity of binding to sulfated glycosaminoglycan; and

p is an integer selected from 1 to 12.

A further preferable ADC is represented by the formula indicated above, wherein Ab represents (4) an antibody comprising a heavy chain comprising a variable region that consists of the amino acid sequence represented by SEQ ID NO: 32 and a light chain comprising a variable region that consists of the amino acid sequence represented by SEQ ID NO: 34, and

p is an integer selected from 1 to 12.

A particularly preferable ADC is represented by the formula indicated above, wherein Ab represents (4) an antibody comprising a heavy chain consisting of the amino acid sequence represented by SEQ ID NO: 48 and a light chain consisting of the amino acid sequence represented by SEQ ID NO: 50 (Antibody 4), and

p is an integer selected from 1 to 12.

A preferable ADC of the present invention is represented by Formula 5 below:

[Chemical formula 4]

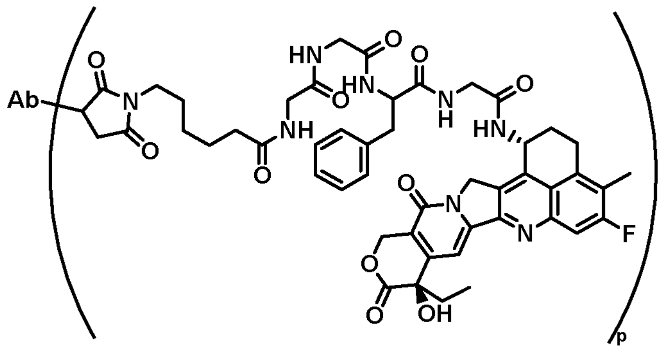

wherein Ab represents (1) an antibody comprising a heavy chain that comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 and a light chain that comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6, and

p is an integer selected from 1 to 12.

A further preferable ADC is represented by the formula indicated above, wherein Ab represents (4) an antibody comprising a heavy chain comprising a variable region that consists of the amino acid sequence represented by SEQ ID NO: 32 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 32 and has activity of binding to sulfated glycosaminoglycan and a light chain comprising a variable region that consists of the amino acid sequence represented by SEQ ID NO: 34 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 34 and has activity of binding to sulfated glycosaminoglycan; and

p is an integer selected from 1 to 12.

A further preferable ADC is represented by the formula indicated above, wherein Ab represents (4) an antibody comprising a heavy chain comprising a variable region that consists of the amino acid sequence represented by SEQ ID NO: 32 and a light chain comprising a variable region that consists of the amino acid sequence represented by SEQ ID NO: 34, and

p is an integer selected from 1 to 12.

A particularly preferable ADC is represented by the formula indicated above, wherein Ab represents (4) an antibody comprising a heavy chain consisting of the amino acid sequence represented by SEQ ID NO: 48 and a light chain consisting of the amino acid sequence represented by SEQ ID NO: 50 (Antibody 4), and

p is an integer selected from 1 to 12.

A preferable ADC of the present invention is represented by Formula 6 below:

[Chemical formula 5]

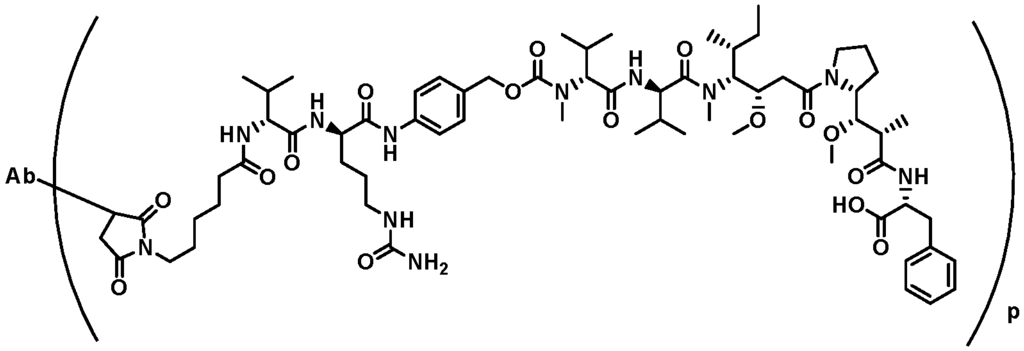

wherein Ab represents (1) an antibody comprising a heavy chain that comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 and a light chain that comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6, and

p is an integer selected from 1 to 12.

A further preferable ADC is represented by the formula indicated above, wherein Ab represents (4) an antibody comprising a heavy chain comprising a variable region that consists of the amino acid sequence represented by SEQ ID NO: 32 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 32 and has activity of binding to sulfated glycosaminoglycan and a light chain comprising a variable region that consists of the amino acid sequence represented by SEQ ID NO: 34 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 34 and has activity of binding to sulfated glycosaminoglycan; and

p is an integer selected from 1 to 12.

A further preferable ADC is represented by the formula indicated above, wherein Ab represents (4) an antibody comprising a heavy chain comprising a variable region that consists of the amino acid sequence represented by SEQ ID NO: 32 and a light chain comprising a variable region that consists of the amino acid sequence represented by SEQ ID NO: 34, and

p is an integer selected from 1 to 12.

A particularly preferable ADC is represented by the formula indicated above, wherein Ab represents (4) an antibody comprising a heavy chain consisting of the amino acid sequence represented by SEQ ID NO: 48 and a light chain consisting of the amino acid sequence represented by SEQ ID NO: 50 (Antibody 4), and

p is an integer selected from 1 to 12.

A preferable ADC of the present invention is represented by Formula 7 below:

[Chemical formula 6]

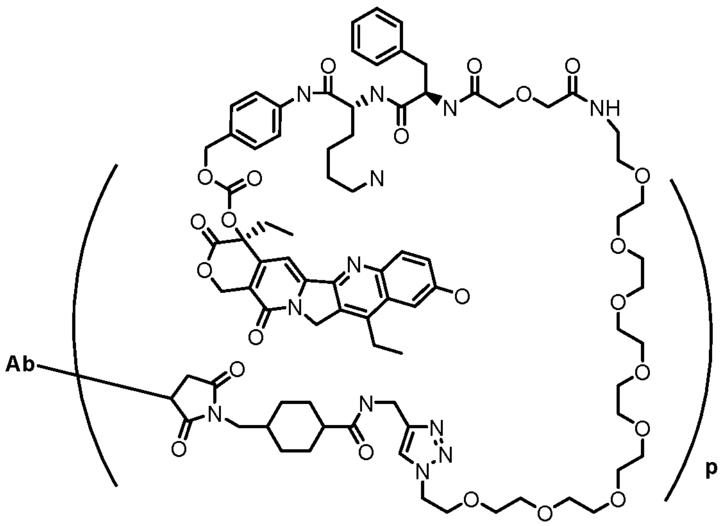

wherein Ab represents (1) an antibody comprising a heavy chain that comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 and a light chain that comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6, and
p is an integer selected from 1 to 12.

A further preferable ADC is represented by the formula indicated above, wherein Ab represents (4) an antibody comprising a heavy chain comprising a variable region that consists of the amino acid sequence represented by SEQ ID NO: 32 and comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 or a heavy chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 32 and has activity of binding to sulfated glycosaminoglycan and a light chain comprising a variable region that consists of the amino acid sequence represented by SEQ ID NO: 34 and comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6 or a light chain comprising a variable region that has at least 85%, preferably at least 90%, further preferably at least 91%, further preferably at least 92%, further preferably at least 93%, further preferably at least 94%, further preferably at least 95%, further preferably at least 96%, further preferably at least 97%, further preferably at least 98%, and particularly preferably at least 99% sequence identity to the amino acid sequence represented by SEQ ID NO: 34 and has activity of binding to sulfated glycosaminoglycan; and
p is an integer selected from 1 to 12.

A further preferable ADC is represented by the formula indicated above, wherein Ab represents (4) an antibody comprising a heavy chain comprising a variable region that consists of the amino acid sequence represented by SEQ ID NO: 32 and a light chain comprising a variable region that consists of the amino acid sequence represented by SEQ ID NO: 34, and
p is an integer selected from 1 to 12.

A particularly preferable ADC is represented by the formula indicated above, wherein Ab represents (4) an antibody comprising a heavy chain consisting of the amino acid sequence represented by SEQ ID NO: 48 and a light chain consisting of the amino acid sequence represented by SEQ ID NO: 50 (Antibody 4), and
p is an integer selected from 1 to 12.

Hereafter, a linker-drug structure and a linker structure used in the ADC of the present invention are shown. In a figure of a linker, a linker binds to an antibody on its left-hand side and it binds to a drug on its right-hand side at the sites indicated by broken lines.

ADC1 to 4 (SMCC-DM1)

[Chemical formula 7]

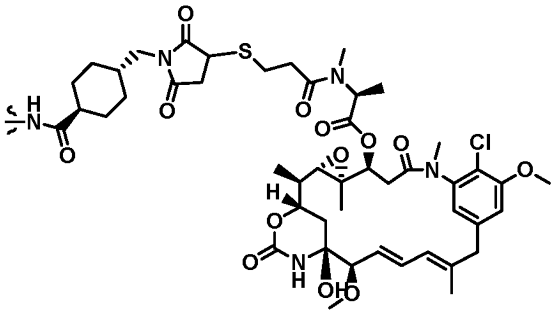

SMCC

[Chemical formula 8]

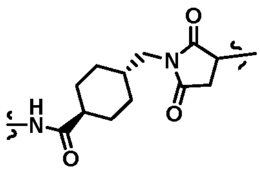

DM1

[Chemical formula 9]

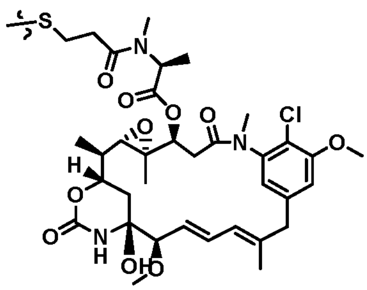

ADC5(SPDB-DM4)

[Chemical formula 10]

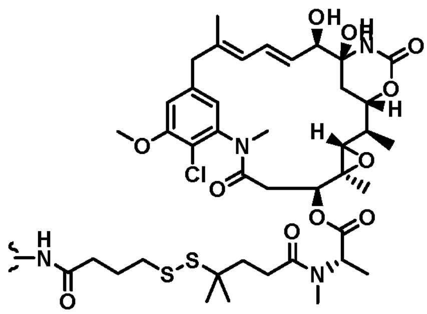

SPDB

[Chemical formula 11]

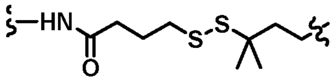

DM4

[Chemical formula 12]

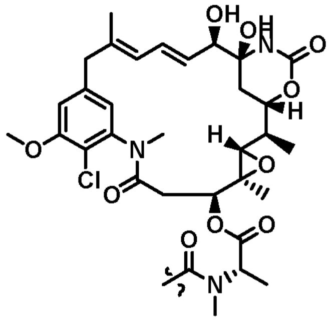

ADC6 (MC-Val-Cit-PAB-MMAE)

[Chemical formula 13]

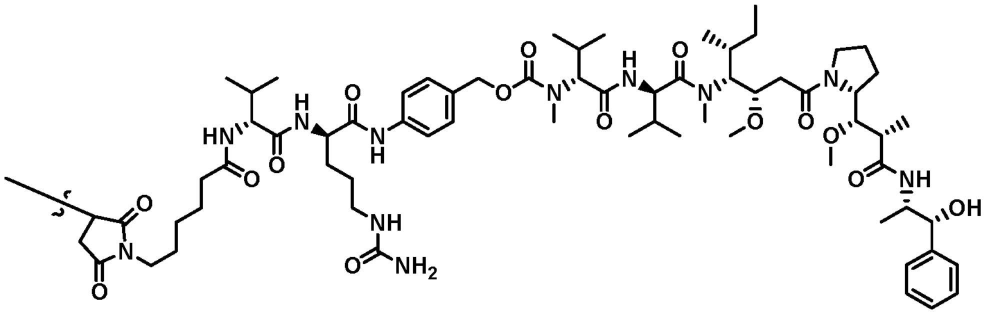

MC-Val-Cit-PAB

[Chemical formula 14]

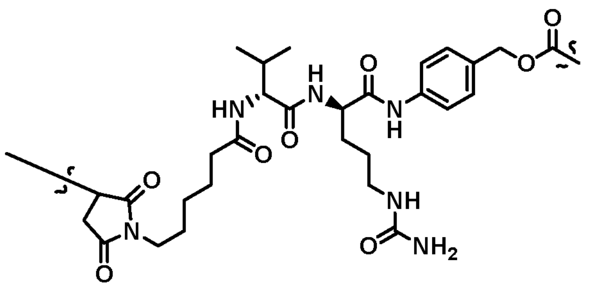

MMAE

[Chemical formula 15]

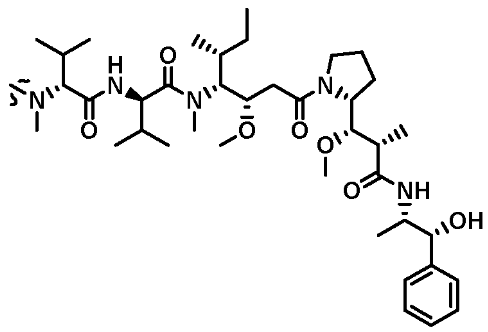

ADC7 (MC-GGFG-Dxd)

[Chemical formula 16]

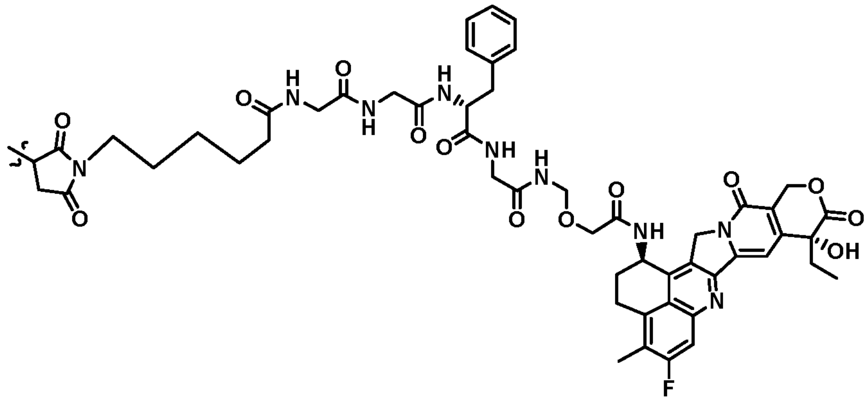

MC-GGFG

[Chemical formula 17]

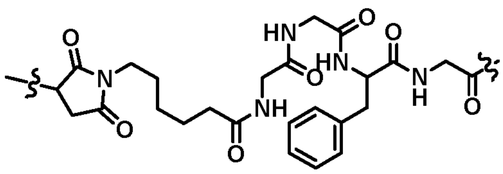

Dxd

[Chemical formula 18]

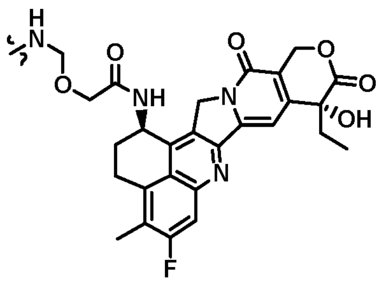

ADC8 (MC-GGFG-Dx8951)

[Chemical formula 19]

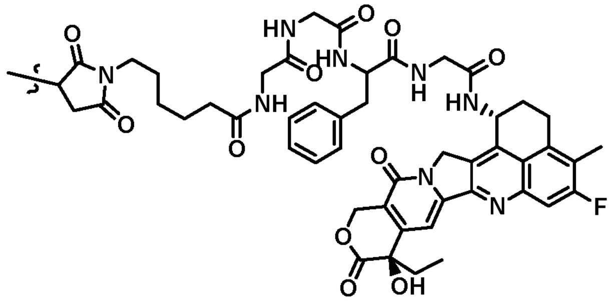

MC-GGFG

[Chemical formula 20]

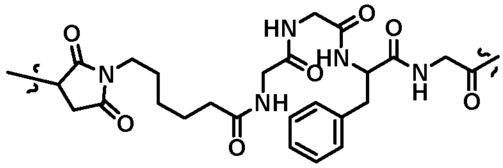

DX8951

[Chemical formula 21]

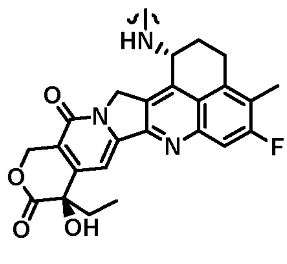

ADC9 (MC-Val-Cit-PAB-MMAF)

[Chemical formula 22]

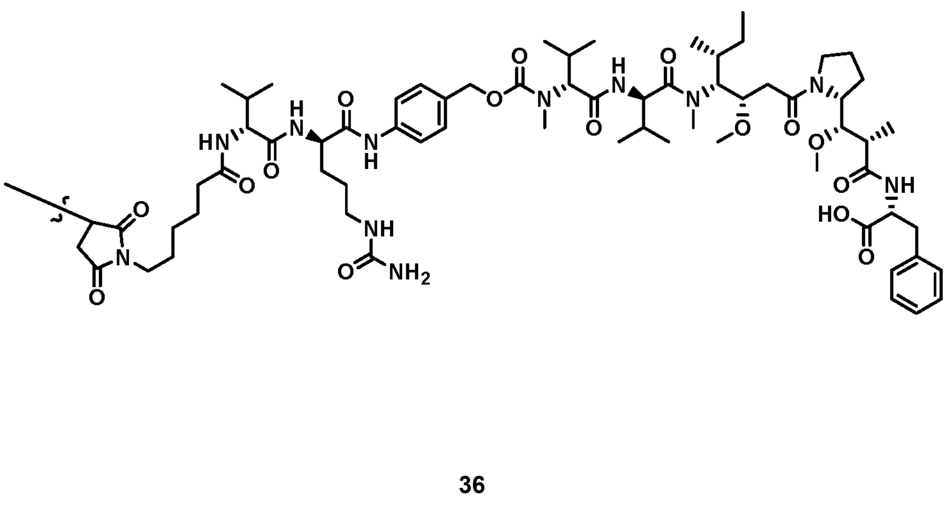

MC-Val-Cit-PAB

[Chemical formula 23]

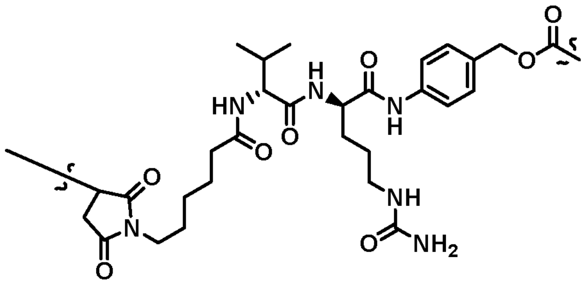

MMAF

[Chemical formula 24]

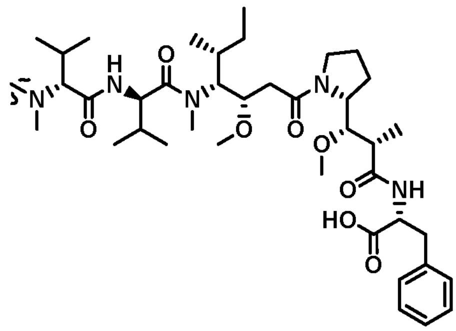

ADC10 (MC-CL2-SN-38)

[Chemical formula 25]

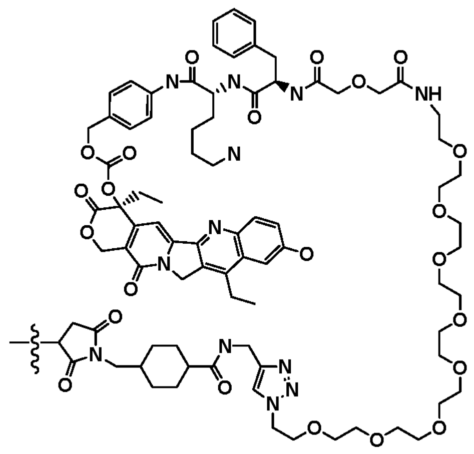

MC-CL2

[Chemical formula 26]

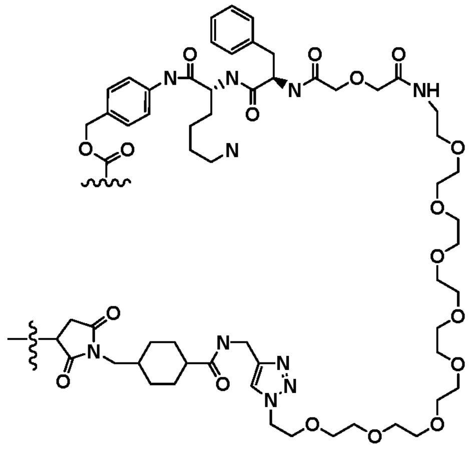

SN-38

[Chemical formula 27]

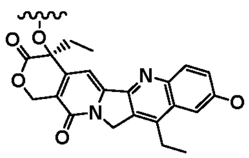

<Anti-cancer agent>

The present invention also includes an anti-cancer agent comprising, as an active ingredient, the ADC.

Types of cancers on which the antibody-drug conjugate (ADC) of the present invention exerts anti-tumor effects are not particularly limited, provided that the cancer expresses sGAG. Specific examples include gastric cancer, pancreatic cancer, large bowel cancer, hepatic cancer, lung cancer, breast cancer, skin cancer, oral cavity cancer, esophageal cancer, bile duct cancer, endometrial cancer, cervix cancer, ovarian cancer, renal cell cancer, urothelial cancer, and prostate cancer. Cancer is preferably solid cancer, and more preferably breast cancer, ovarian cancer, endometrial cancer, cervix cancer, neuroblastoma, glioma, glioblastoma, bladder cancer, melanoma, colon cancer, large bowel cancer, gastric cancer, lung cancer, pancreatic cancer, prostate cancer, hepatic cancer, nasopharyngeal cancer, renal cancer, multiple myeloma, oral squamous cell carcinoma, esophageal cancer, esophageal squamous cell carcinoma, or peripheral nerve sheath tumors (Int. J. Mol. Sci., 2020, 21 (17), 5983). Cancer is particularly preferably gastric cancer, pancreatic cancer, breast cancer, large bowel cancer, or lung cancer.

The ADC of the present invention is incorporated into the cancer cells, and it then exerts therapeutic effects.

While some sGAGs are expressed in healthy tissue, the antibody used for the ADC of the present invention is pH-dependent. Thus, such antibody does not bind to sGAG in healthy tissue at neutral pH, and it is less likely to be incorporated into healthy tissue cells. That is, it is highly unlikely that such antibody would cause side effects on healthy tissue.

The route of administration of the anti-cancer agent comprising the ADC of the present invention is not limited, and the anti-cancer agent can be administered through the oral, parenteral, or other route. Examples of parenteral administration include intravenous administration, intramuscular administration, and subcutaneous administration. Alternatively, the anti-tumor agent may be administered through the transmucosal (e.g., sublingual or buccal), percutaneous, or rectal route. The anti-cancer agent may be administered systemically or topically on the cancer tissue. Examples of dosage forms of formulations include tablets, capsules, pills, powders, granules, and injection formulations. Oral formulations, such as tablets, capsules, pills, powders, or granules, can be prepared with the use of additives, and

examples of additives include: excipients, such as glucose, sucrose, lactose, and mannitol; disintegrators, such as starch and sodium alginate; lubricants, such as magnesium stearate and talc; binders, such as polyvinyl alcohol, gelatin, and hydroxypropyl cellulose; surfactants, such as fatty acid ester; and plasticizers, such as glycerin. Injection formulations can be prepared with the use of additives, and examples of additives include: water; saccharides, such as sucrose, sorbitol, xylose, trehalose, and fructose; sugar alcohol, such as sorbitol, mannitol, and xylitol; buffers, such as phosphate buffer, citrate buffer, and glutamate buffer; and surfactants, such as fatty acid ester.

A dose of the ADC to a patient who is in need of treatment varies depending on age, gender, severity, and other conditions of the patient to which the ADC is to be administered, and the physician can determine an adequate dose. For example, the ADC may be administered at 0.05 to 10 mg/kg/body weight, and preferably 0. 1 to 2 mg/kg/body weight, per instance. Also, administration may be performed at 0.001 nM to 1000 nM, and preferably 0.001 nM to 100 nM, per instance, at, for example, the MMAE equivalent dose.

While administration may be performed once, administration may be performed 2, 3, 4, or more separate instances daily. In such a case, administration may be performed at adequate intervals for 1 day to several years.

The anti-cancer agent comprising, as an active ingredient, the ADC of the present invention can be used in combination with other anti-cancer agents or other therapeutic techniques. Examples of other therapeutic techniques include surgery, radiotherapy, and chemotherapy.

The present invention also includes a method for treating tumors comprising administering the ADC to a patient who is in need of treatment. Tumors to be treated are as described above, and methods of administration are as described above.

In addition, the present invention includes the ADC used for treating tumors. Tumors to be treated are as described above, and methods of administration are as described above.

In addition, the present invention include use of the ADC for producing anti-tumor agents. Tumors to be treated are as described above, and methods of administration are as described above.

[Examples]

Hereafter, the present invention is described in greater detail with reference to the examples, although the present invention is not limited thereto.

[Example 1: Preparation of pH-dependent anti-sGAG monoclonal antibody (mAB) by recombinant DNA method]

cDNA constructs were prepared from the genes of the heavy chains and the light chains encoding Antibody 1 to Antibody 4 shown in Table 2. cDNA constructs encoding the heavy chains and the light chains were integrated into the pcDNA3 plasmid (Life Technologies) and introduced into HEK293 cells in combination with clones to forcibly express the heavy chains and the light chains. After the culture supernatant was filtered through a 0.22-μm filter, Protein A purification and gel filtration purification were performed to obtain the pH-dependent anti-sGAG antibody.

[Table 2]

| | | CDR1 | CDR2 | CDR3 | Variable region | Full-length |
|---|---|---|---|---|---|---|
| Antibody 1 | IgH | GGFITSHH (SEQ ID NO: 1) | LHHTGTT (SEQ ID NO: 2) | VREDSSSWHPGRYIQL (SEQ ID NO: 3) | Nucleotide sequence: SEQ ID NO: 19; amino acid sequence: SEQ ID NO: 20 | Nucleotide sequence: SEQ ID NO: 35; amino acid sequence: SEQ ID NO: 36 |
| | IgK | QSLLQNNRYNR (SEQ ID NO: 4) | LGS | MHALEPPYT (SEQ ID NO: 6) | Nucleotide sequence: SEQ ID NO: 21; amino acid sequence: SEQ ID NO: 22 | Nucleotide sequence: SEQ ID NO: 37; amino acid sequence: SEQ ID NO: 38 |

(continued)

| | | CDR1 | CDR2 | CDR3 | Variable region | Full-length |
|---|---|---|---|---|---|---|
| Antibody 2 | IgH | GFTFNTYT (SEQ ID NO: 7) | ISLSSNTI (SEQ ID NO: 8) | ARAESYYDSHYYY NGLDV (SEQ ID NO: 9) | Nucleotide sequence: SEQ ID NO: 23; amino acid sequence: SEQ ID NO: 24 | Nucleotide sequence: SEQ ID NO: 39; amino acid sequence: SEQ ID NO: 40 |
| | IgK | QSVGGRY (SEQ ID NO: 10) | AAS | QQYGSSPRT (SEQ ID NO: 12) | Nucleotide sequence: SEQ ID NO: 25; amino acid sequence: SEQ ID NO: 26 | Nucleotide sequence: SEQ ID NO: 41; amino acid sequence: SEQ ID NO: 42 |
| Antibody 3 | IgH | GFTFSDYY (SEQ ID NO: 13) | ISISGTTR (SEQ ID NO: 14) | ARGPELLSQNYYYY YGMDV (SEQ ID NO: 15) | Nucleotide sequence: SEQ ID NO: 27; amino acid sequence: SEQ ID NO: 28 | Nucleotide sequence: SEQ ID NO: 43; amino acid sequence: SEQ ID NO: 44 |
| | IgK | ETLLYRSNNKN Y (SEQ ID NO: 16) | WAS | QQYYTTPRT (SEQ ID NO: 18) | Nucleotide sequence: SEQ ID NO: 29; amino acid sequence: SEQ ID NO: 30 | Nucleotide sequence: SEQ ID NO: 45; amino acid sequence: SEQ ID NO: 46 |
| Antibody 4 | IgH | GGFITSHH (SEQ ID NO: 1) | LHHTGTT (SEQ ID NO: 2) | VREDSSSWHPGRYI QL (SEQ ID NO: 3) | Nucleotide sequence: SEQ ID NO: 31; amino acid sequence: SEQ ID NO: 32 | Nucleotide sequence: SEQ ID NO: 47; amino acid sequence: SEQ ID NO: 48 |
| | IgK | QSLLQNNRYNR (SEQ ID NO: 4) | LGS | MHALEPPYT (SEQ ID NO: 6) | Nucleotide sequence: SEQ ID NO: 33; amino acid sequence: SEQ ID NO: 34 | Nucleotide sequence: SEQ ID NO: 49; amino acid sequence: SEQ ID NO: 50 |

[Example 2: pH-depending binding of anti-sGAG antibody to cancer cell]

The cells of the gastric cancer cell line NUGC-3 (HSRRB) were washed with PBS and then collected using a scraper. The number of the cells was counted and collected at $1 \times 10^6$ cells/well on a 96-well plate. The anti-sGAG antibody obtained in Example 1 and the human IgG isotype control (Invitrogen) were diluted to the final concentration of 15 μg/ml using 1% BSA/PBS adjusted to a relevant pH level (pH 8.0 to 5.0), added to the NUGC-3 cells, and then allowed to stand at room temperature for 30 minutes. After the reaction with the sGAG antibody, the cells were washed with PBS (pH 8.0 to 5.0), and anti-human IgG-Alexa Fluor 488 (Invitrogen) dissolved in 1% BSA/PBS (pH 8.0 to 5.0) was then added

thereto. After the reaction with the secondary antibody, the cells were washed with PBS (pH 8.0 to 5.0), and the Alexa Fluor 488 signal (the mean fluorescence intensity (MFI)) was detected using a flow cytometer (CytoFLEX S, Beckman Coulter). The results demonstrate that all the evaluated sGAG antibodies exhibit an increase in the pH-dependent binding capability at low pH levels from 6.8 to 5.5 (Figure 1).

PNP-xyl has been found to decrease the amount of GAG on the cell surface by competitive inhibition (Biochemical Pharmacology, 1991, 42, (10), pp. 1987-1995; Oncotarget, 2017, 8, (40), pp. 66960-66974). The pH-dependent binding of the sGAG antibody is inhibited to a significant extent by subjecting the cultured cells to treatment with PNP-Xyl (4 mM, 48 hours). This indicates that pH-dependent binding of the sGAG antibody to cells does not occur due to nonspecific activity (Figure 2).

[Example 3: Evaluation of binding capability of sGAG antibody to a plurality of cancer cell lines]

The binding capability of Antibody 1 to a plurality of cancer cell lines was evaluated in the same manner as in Example 2. The binding capability of the isotype control antibody and that of Antibody 1 to the gastric cancer cell lines (NCI-N87 (ATCC) and NUGC-3 (HSRRB)), the pancreatic cancer cell lines (PANC-1 (Dainippon Pharma Co., Ltd.), AsPC-1 (JCRB), KP-4 (HSRRB), and SUIT2 (JCRB)), the breast cancer cell line (MDA-MB-231 (ATCC)), the large bowel cancer cell lines (SK-CO-1 (ATCC) and HCT116 (ATCC)), and the lung cancer cell lines (NCI-H460 (ATCC), DMS273 (ECACC), and A549 (Dainippon Pharma Co., Ltd.)) at pH 6.0 were evaluated. As a result, expression of the target antigen of Antibody 1 was observed in all the evaluated cancer cell lines, and Antibody 1 was found to be useful for treatment of a plurality of types of cancers (Figure 3).

[Example 4: Preparation of antibody-drug conjugate with anti-sGAG antibody]

The ADC of the present invention can be prepared by allowing a drug linker to be added to ADC to react with an antibody having an amino group and a sulfhydryl group in the manner described below. Concerning the ADCs obtained with the use of the anti-sGAG antibody, Figure 4 shows combinations of antibodies and drug linkers bound thereto. The prepared ADCs are subjected to concentration, buffer exchange, purification, and measurement of the antibody concentration and the average number of drugs bound per one molecule of antibody in accordance with the common operations described below to identify the ADCs.

Common operation A: Concentration of antibody or ADC solution

The antibody or ADC solution was introduced into the Amicon Ultra container (100,000 MWCO, Millipore Corporation) and concentrated by centrifugation (at 2000 G to 15000 G for 5 to 20 minutes) using a centrifuge (himac CF5RE, HITACHI).

Common operation B: Measurement of antibody concentration

Antibody concentration was measured with the use of the UV photometer (Nanodrop 8000, Thermo Fisher Scientific Inc.) in accordance with the manufacturer's instructions.

Common operation C: Antibody/ADC buffer exchange and purification

The Zeba(TM) Spin Desalting Column (7 K MWCO to 40 K MWCO, Cat. 89890, Thermo Scientific) was equilibrated with D-PBS (Nacalai Tesque Inc.) in accordance with the manufacturer's instructions. After the antibody/ADC solution was placed, the antibody/ADC was eluted with D-PBS in the amount prescribed by the manufacturer, and the concentration was measured in accordance with the common operation B.

Common operation D-1: Measurement of antibody concentration and average number of drugs bound per one molecule of antibody in ADC

The drug-antibody ratio of DM1 bound to the antibody can be determined by assaying the UV absorbance of the ADC solution at 280 nm and at 252 nm and implementing calculation in accordance with the following equation.

$$\mathrm{DAR} = \frac{\varepsilon^{252}_{\mathrm{mAb}} - R\varepsilon^{280}_{\mathrm{mAb}}}{R\varepsilon^{280}_{\mathrm{Drug}} - \varepsilon^{252}_{\mathrm{Drug}}}$$

In the equation, R represents the absorbance ratio of ADC at A252/A280.

$\varepsilon_{mAb}^{252}$ and $\varepsilon_{mAb}^{280}$ each indicate the molar absorption coefficient of the antibody used at 252 nm or 280 nm.

$\varepsilon_{DM1}^{252}$ and $\varepsilon_{DM1}^{280}$ each indicate the molar absorption coefficient of DM1 at 252 nm or 280 nm.

$\varepsilon_{DM1}^{252}$ and $\varepsilon_{DM1}^{280}$ were determined in accordance with the following equations.

$$\varepsilon_{DM1}^{252} = 2.64 \times 10^5\,M^{-1}\,cm^{-1}; \text{ and } \varepsilon_{DM1}^{280} = 5.23 \times 10^3\,M^{-1}\,cm^{-1}$$

The value $\varepsilon_{mAb}^{280}$ was deduced based on the amino acid sequence of the antibody in accordance with the known calculation formula (Protein Science, 1995, 4, pp. 2411-2423), the value of Antibody 1 was determined to be 214,460 $M^{-1}$ $cm^{-1}$, and the value of Antibody 2 and that of Antibody 3 were each determined to be 207360 $M^{-1}$ $cm^{-1}$.

The value $\varepsilon_{mAb}^{252}$ was determined by calculating the average of the actually measured values of the antibodies.

Common operation D-2: Measurement of average number of drugs bound per one molecule of antibody in ADC

The drug-antibody ratio was analyzed by native size-exclusion chromatography mass spectrometry (SEC-MS). The SEC-MS analysis enables measurement of the drug-antibody ratio based on a mass change resulting from binding of a drug linker to an antibody. ADC was injected at 2 mg/ml into Acquity LC Xevo G2-S Q Tof MS (Waters Corporation), and ADC was eluted from the column by isocratic elution (100 mM aqueous ammonium acetate solution, 20 min). The obtained data were analyzed using MassLynx software (Waters Corporation). Measurement was performed using the ACQUITY UPLC Protein BEH SEC Column, 200 Å, 1.7 μm, 4.6 mm × 150 mm (Waters) at a flow rate of 0.1 to 0.3 ml/min and column temperature of 25°C.

[Production Example 1: Antibody-drug conjugate 1 (ADC1)]

[Chemical formula 28]

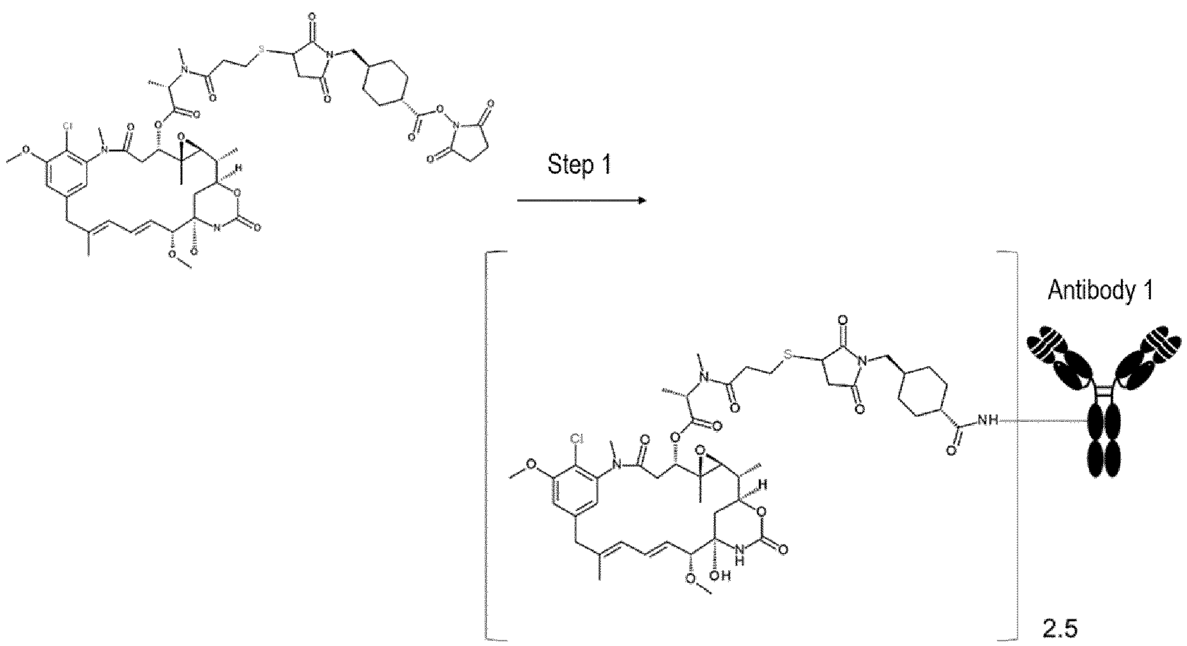


Step 1: Conjugation of antibody to drug linker

To a solution containing 0.15 mg of Antibody 1 prepared in Example 1, 10 molar equivalents of a DMSO solution containing 10 mM SMCC-DM1 (MedChemExpress) was added per one molecule of the antibody at room temperature, and N,N-dimethylacetamide (DMA) was added thereto to a final concentration of 10%. The resultant was agitated using a

rotator at room temperature for 24 hours, and the drug linker was allowed to bind to the antibody. Thus, an antibody-drug conjugate 1 (ADC1) was obtained.

Step 2: Purification and physical property evaluation

A solution containing the antibody-drug conjugate 1 (ADC1) obtained in Step 1 was purified and the concentration thereof was measured in accordance with the common operation C, and the property values indicated below were obtained in accordance with the common operation D-1. The product was verified to be free from aggregates by dynamic light scattering. Antibody concentration: 0.255 mg/ml; average number of drugs bound per one molecule of antibody (n): 2.5

[Production Example 2: Antibody-drug conjugate 2 (ADC2)]

[Chemical formula 29]

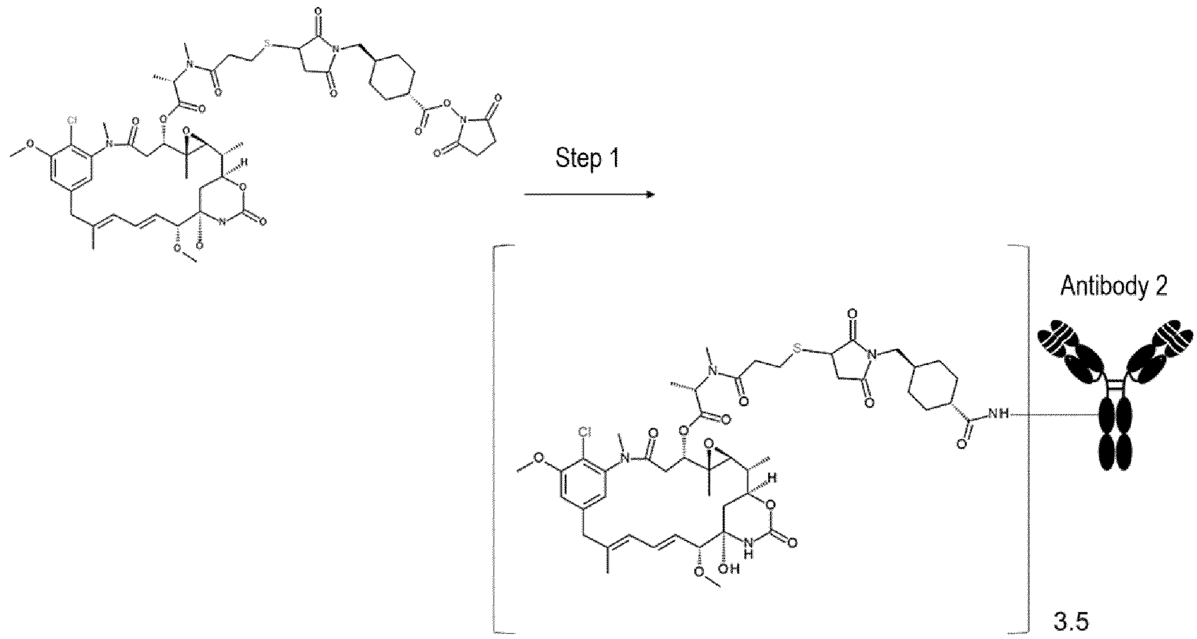


Step 1: Conjugation of antibody to drug linker

To a solution containing 0.29 mg of Antibody 2 prepared in Example 1, 10 molar equivalents of a DMSO solution containing 10 mM SMCC-DM1 (MedChemExpress) was added per one molecule of the antibody at room temperature, and DMA was added thereto to a final concentration of 10%. The resultant was agitated using a rotator at room temperature for 24 hours, and the drug linker was allowed to bind to the antibody. Thus, an antibody-drug conjugate 2 (ADC2) was obtained.

Step 2: Purification and physical property evaluation

A solution containing the antibody-drug conjugate 2 (ADC2) obtained in Step 1 was purified and the concentration thereof was measured in accordance with the common operation C, and the property values indicated below were obtained in accordance with the common operation D-1. The product was verified to be free from aggregates by dynamic light scattering. Antibody concentration: 2.24 mg/ml; average number of drugs bound per one molecule of antibody (n): 3.5

[Production Example 3: Antibody-drug conjugate 3 (ADC3)]

[Chemical formula 30]

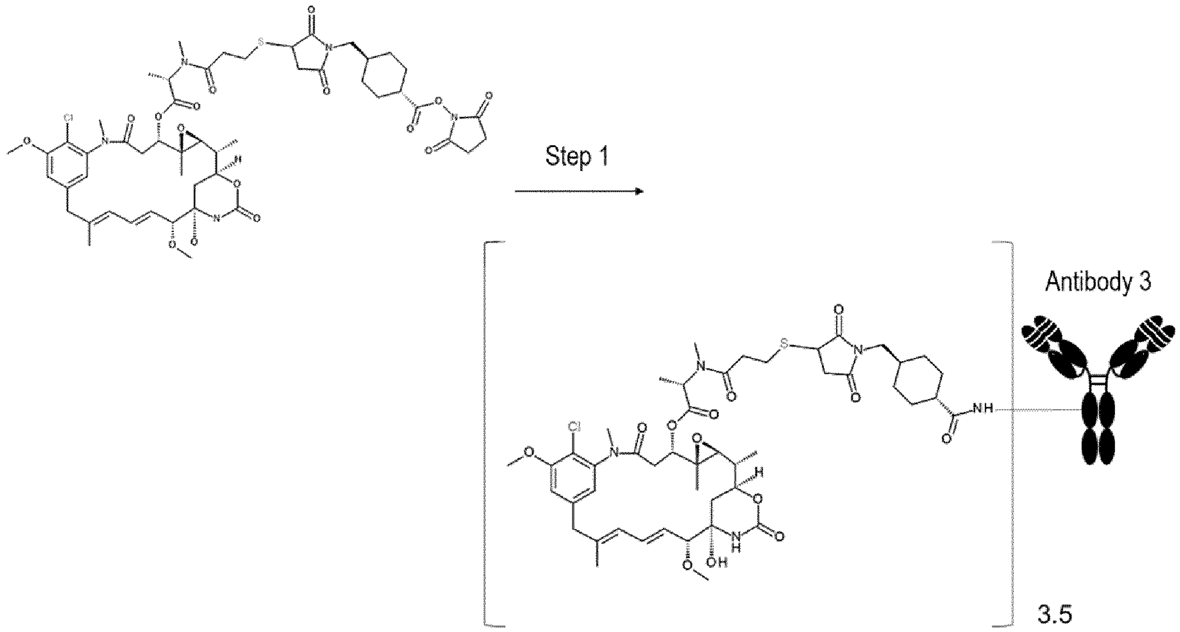


Step 1: Conjugation of antibody to drug linker

To a solution containing 0.18 mg of Antibody 3 prepared in Example 1, 10 molar equivalents of a DMSO solution containing 10 mM SMCC-DM1 (MedChemExpress) was added per one molecule of the antibody at room temperature, and DMA was added thereto to a final concentration of 10%. The resultant was agitated using a rotator at room temperature for 24 hours, and the drug linker was allowed to bind to the antibody. Thus, an antibody-drug conjugate 3 (ADC3) was obtained.

Step 2: Purification and physical property evaluation

A solution containing the antibody-drug conjugate 3 (ADC3) obtained in Step 1 was purified and the concentration thereof was measured in accordance with the common operation C, and the property values indicated below were obtained in accordance with the common operation D-1. The product was verified to be free from aggregates by dynamic light scattering. Antibody concentration: 0.182 mg/ml; average number of drugs bound per one molecule of antibody (n): 3.5

[Production Example 4: Antibody-drug conjugate 4 (ADC4)]

[Chemical formula 31]

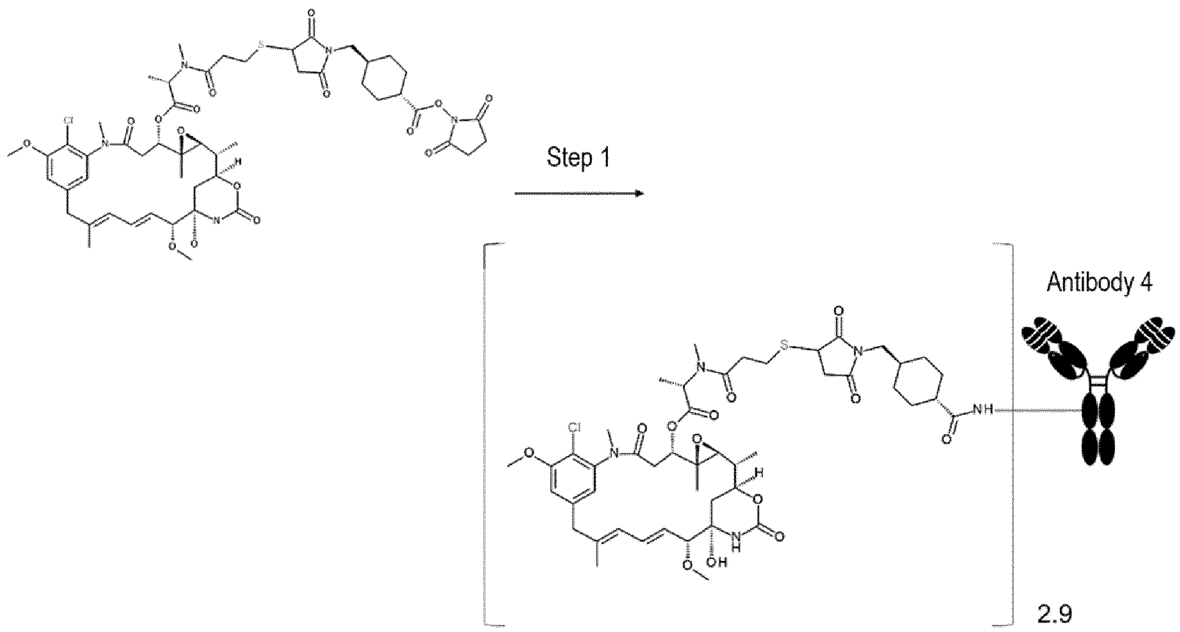


Step 1: Conjugation of antibody to drug linker

To a solution containing 1 mg of Antibody 4 prepared in Example 1, 9.6 molar equivalents of a DMSO solution containing 10 mM SMCC-DM1 (MedChemExpress) was added per one molecule of the antibody at room temperature, and DMA was added thereto to a final concentration of 10%. The resultant was agitated using a rotator at room temperature for 24 hours, and the drug linker was allowed to bind to the antibody. Thus, an antibody-drug conjugate 4 (ADC4) was obtained.

Step 2: Purification and physical property evaluation

A solution containing the antibody-drug conjugate 4 (ADC4) obtained in Step 1 was purified and the concentration thereof was measured in accordance with the common operation C, and the property values indicated below were obtained in accordance with the common operation D-2. The product was verified to be free from aggregates by dynamic light scattering. Antibody concentration: 0.503 mg/ml; average number of drugs bound per one molecule of antibody (n): 2.9

[Production Example 5: Antibody-drug conjugate 5 (ADC5)]

[Chemical formula 32]

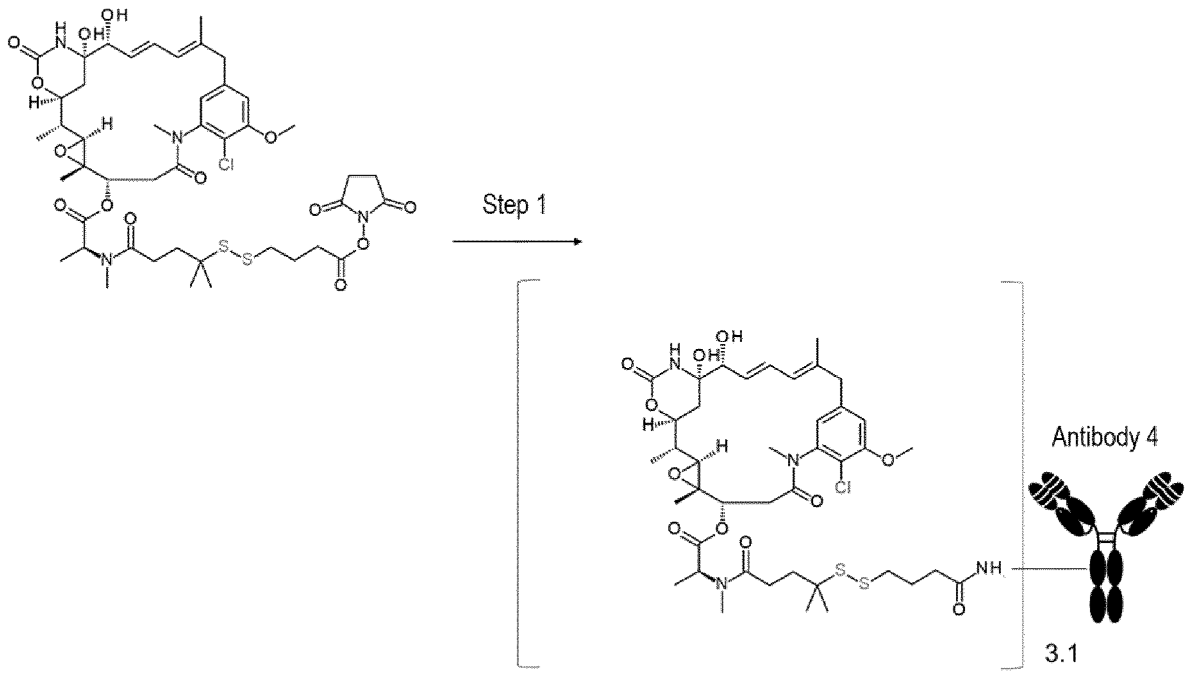


Step 1: Conjugation of antibody to drug linker

To a solution containing 1 mg of Antibody 4 prepared in Example 1, 16 molar equivalents of a DMSO solution containing 10 mM SPDB-DM4 (MedChemExpress) was added per one molecule of the antibody at room temperature, and DMA was added thereto to a final concentration of 20%. The resultant was agitated using a rotator at room temperature for 24 hours, and the drug linker was allowed to bind to the antibody. Thus, an antibody-drug conjugate 5 (ADC5) was obtained.

Step 2: Purification and physical property evaluation

A solution containing the antibody-drug conjugate 5 (ADC5) obtained in Step 1 was purified and the concentration thereof was measured in accordance with the common operation C, and the property values indicated below were obtained in accordance with the common operation D-2. The product was verified to be free from aggregates by dynamic light scattering. Antibody concentration: 0.500 mg/ml; average number of drugs bound per one molecule of antibody (n): 3.1

[Production Example 6: Antibody-drug conjugate 6 (ADC6)]

[Chemical formula 33]

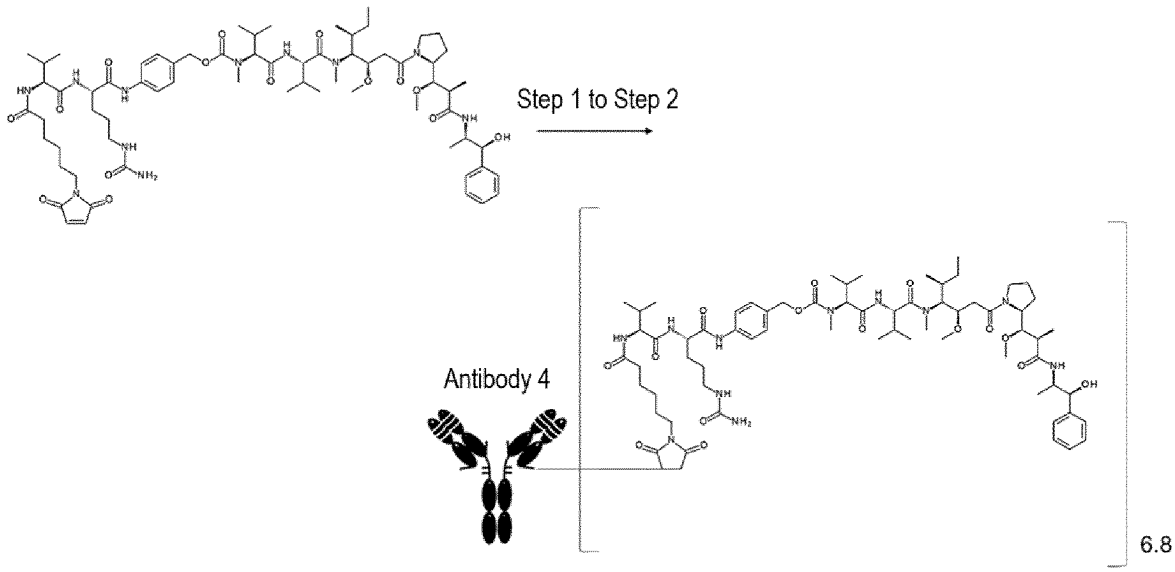


Step 1: Antibody reduction

To a solution containing 64.8 mg of Antibody 4 prepared in Example 1, an aqueous solution of 5 mM TCEP (20 molar equivalents per one molecule of the antibody) was added, and the resultant was incubated at 37°C for 2 hours to reduce a disulfide bond in the hinge region of the antibody.

Step 2: Conjugation of antibody to drug linker

To the solution obtained in Step 1, a DMSO solution containing 10 mM MC-VC-PAB-MMAE (MedChemExpress) (10 molar equivalents per one molecule of the antibody) was added at room temperature, the resultant was agitated using a rotator at 4°C for 24 hours, and the drug linker was allowed to bind to the antibody. Thus, an antibody-drug conjugate 6 (ADC6) was obtained.

Step 3: Purification and physical property evaluation

A solution containing the antibody-drug conjugate 6 (ADC6) obtained in Step 2 was purified and concentrated, and the concentration thereof was measured in accordance with the common operation A and the common operation C. The property values indicated below were obtained in accordance with the common operation D-2. The product was verified to be free from aggregates by dynamic light scattering.
Antibody concentration: 4.38 mg/ml; average number of drugs bound per one molecule of antibody (n): 6.8

[Production Example 7: Antibody-drug conjugate 7 (ADC7)]

[Chemical formula 34]

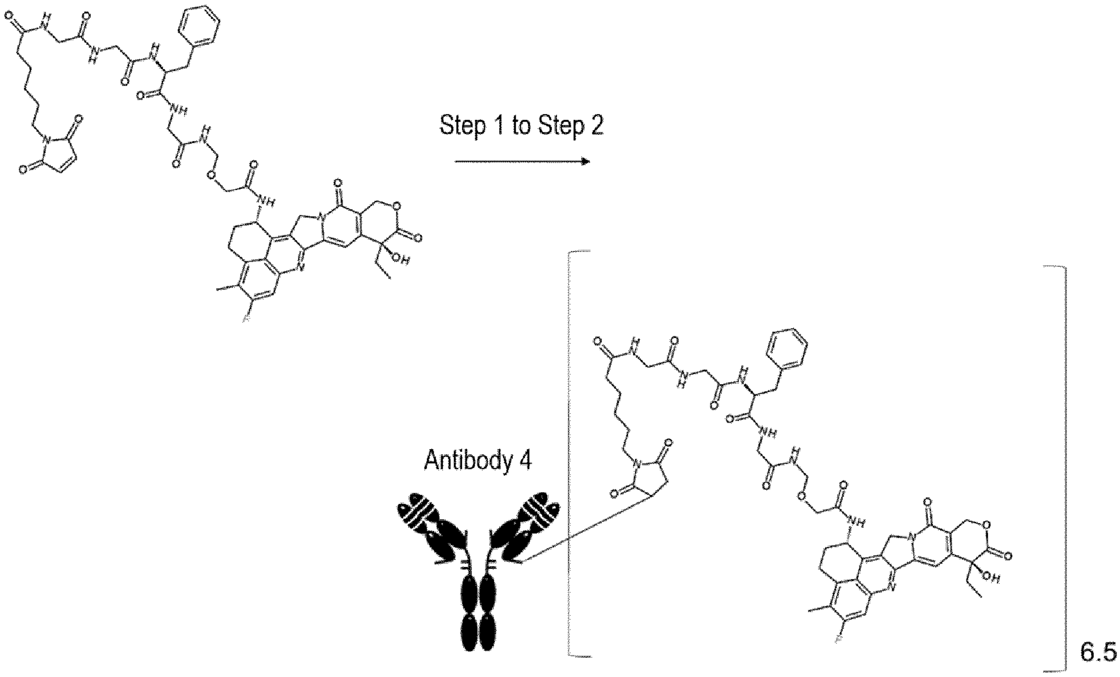


Step 1: Antibody reduction

To a solution containing 6.5 mg of Antibody 4 prepared in Example 1, an aqueous solution of 5 mM TCEP (30 molar equivalents per one molecule of the antibody) was added, and the resultant was incubated at 37°C for 2 hours to reduce a disulfide bond in the hinge region of the antibody.

Step 2: Conjugation of antibody to drug linker

To a 1-mg antibody fraction of the solution, a DMSO solution containing 10 mM MC-GGFG-Dxd (MedChemExpress) (9.6 molar equivalents per one molecule of the antibody) was added at room temperature, the resultant was agitated using a rotator at 4°C for 24 hours, and the drug linker was allowed to bind to the antibody. Thus, an antibody-drug conjugate 7 (ADC7) was obtained.

Step 3: Purification and physical property evaluation

A solution containing the antibody-drug conjugate 7 (ADC7) obtained in Step 2 was purified and the concentration thereof was measured in accordance with the common operation C, and the property values indicated below were obtained in accordance with the common operation D-2. The product was verified to be free from aggregates by dynamic light scattering. Antibody concentration: 1.17 mg/ml; average number of drugs bound per one molecule of antibody (n): 6.5

[Production Example 8: Antibody-drug conjugate 8 (ADC8)]

[Chemical formula 35]

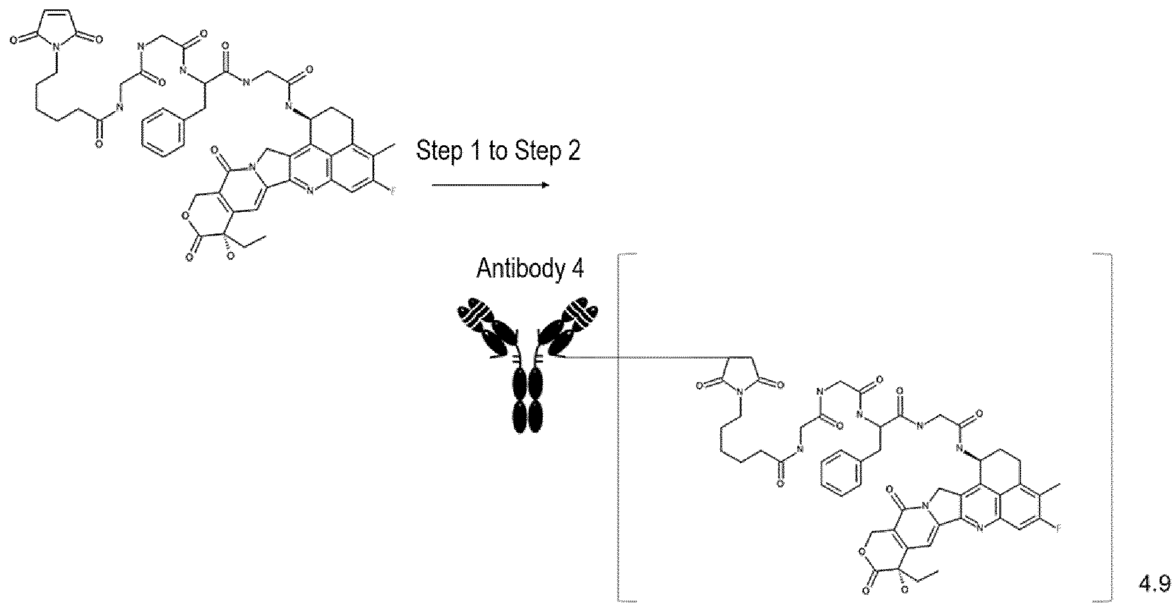


Step 1: Antibody reduction

To a solution containing 6.5 mg of Antibody 4 prepared in Example 1, an aqueous solution of 5 mM TCEP (30 molar equivalents per one molecule of the antibody) was added, and the resultant was incubated at 37°C for 2 hours to reduce a disulfide bond in the hinge region of the antibody.

Step 2: Conjugation of antibody to drug linker

To a 1-mg antibody fraction of the solution obtained in Step 1, a DMSO solution containing 10 mM MC-GGFG-Dx8951 (MedChemExpress) (9.6 molar equivalents per one molecule of the antibody) was added at room temperature, the resultant was agitated using a rotator at 4°C for 24 hours, and the drug linker was allowed to bind to the antibody. Thus, an antibody-drug conjugate 8 (ADC8) was obtained.

Step 3: Purification and physical property evaluation

A solution containing the antibody-drug conjugate 8 (ADC8) obtained in Step 2 was purified and the concentration thereof was measured in accordance with the common operation C, and the property values indicated below were obtained in accordance with the common operation D-2. The product was verified to be free from aggregates by dynamic light scattering. Antibody concentration: 1.07 mg/ml; average number of drugs bound per one molecule of antibody (n): 4.9

[Production Example 9: Antibody-drug conjugate 9 (ADC9)]

[Chemical formula 36]

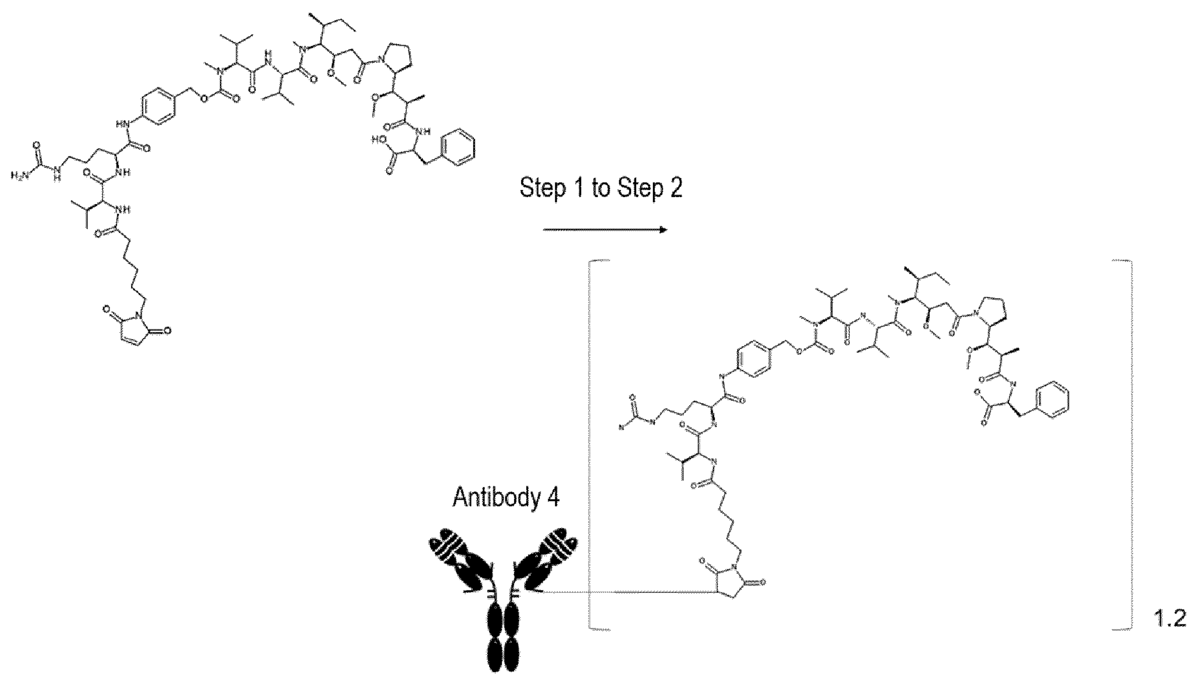


Step 1: Antibody reduction

To a solution containing 6.5 mg of Antibody 4 prepared in Example 1, an aqueous solution of 5 mM TCEP (30 molar equivalents per one molecule of the antibody) was added, and the resultant was incubated at 37°C for 2 hours to reduce a disulfide bond in the hinge region of the antibody.

Step 2: Conjugation of antibody to drug linker

To a 1-mg antibody fraction of the solution obtained in Step 1, a DMSO solution containing 10 mM MC-VC-PAB-MMAF (MedChemExpress) (9.6 molar equivalents per one molecule of the antibody) was added at room temperature, the resultant was agitated using a rotator at 4°C for 24 hours, and the drug linker was allowed to bind to the antibody. Thus, an antibody-drug conjugate 9 (ADC9) was obtained.

Step 3: Purification and physical property evaluation

A solution containing the antibody-drug conjugate 9 (ADC9) obtained in Step 2 was purified and the concentration thereof was measured in accordance with the common operation C, and the property values indicated below were obtained in accordance with the common operation D-2. The product was verified to be free from aggregates by dynamic light scattering. Antibody concentration: 1.64 mg/ml; average number of drugs bound per one molecule of antibody (n): 1.2

[Production Example 10: Antibody-drug conjugate 10 (ADC10)]

[Chemical formula 37]

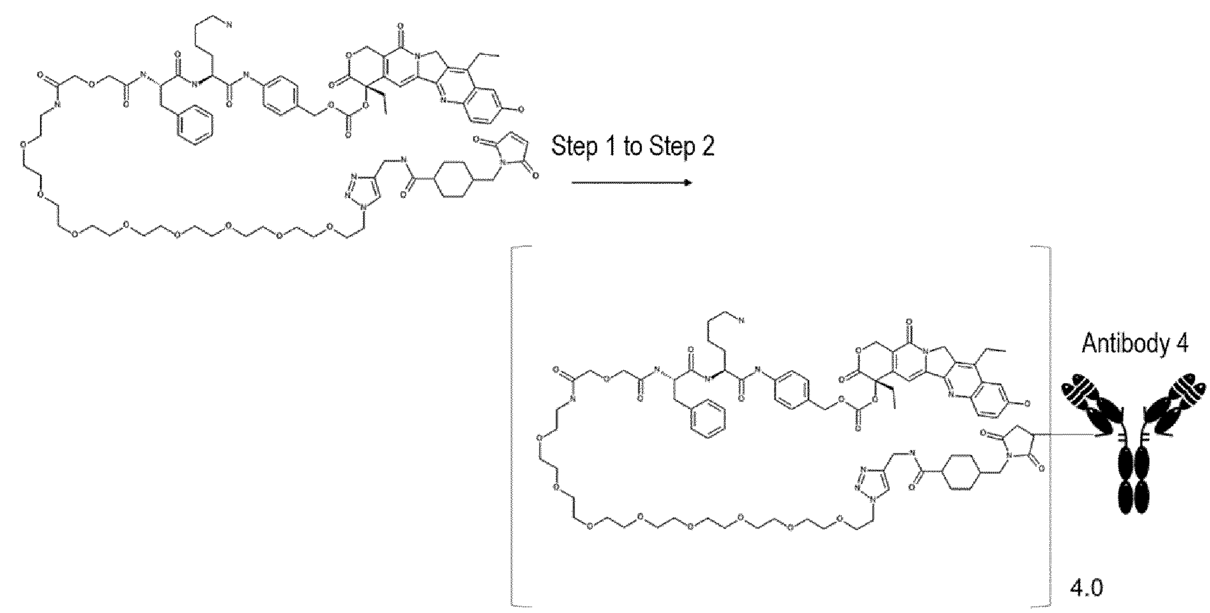


Step 1: Antibody reduction

To a solution containing 6.5 mg of Antibody 4 prepared in Example 1, an aqueous solution of 5 mM TCEP (30 molar equivalents per one molecule of the antibody) was added, and the resultant was incubated at 37°C for 2 hours to reduce a disulfide bond in the hinge region of the antibody.

Step 2: Conjugation of antibody to drug linker

To a 1-mg antibody fraction of the solution obtained in Step 1, a DMSO solution containing 10 mM MC-CL2-SN38 (MedChemExpress) (9.6 molar equivalents per one molecule of the antibody) was added at room temperature, the resultant was agitated using a rotator at 4°C for 24 hours, and the drug linker was allowed to bind to the antibody. Thus, an antibody-drug conjugate 10 (ADC10) was obtained.

Step 3: Purification and physical property evaluation

A solution containing the antibody-drug conjugate 10 (ADC10) obtained in Step 2 was purified and the concentration thereof was measured in accordance with the common operation C, and the property values indicated below were obtained in accordance with the common operation D-2. The product was verified to be free from aggregates by dynamic light scattering. Antibody concentration: 1.65 mg/ml; average number of drugs bound per one molecule of antibody (n): 4.0

[Example 5: Evaluation of binding capability of ADC to a plurality of cancer cell lines]

The binding capability of ADC1 to ADC10 prepared in Example 4 and Antibody 1 to Antibody 4 prepared in Example 1 to the gastric cancer cell line NUGC-4 and the pH dependence thereof were evaluated by flow cytometry in the same manner as in Example 2. The antibodies and the ADCs were serially diluted from 300 μg/ml at a common ratio of 3, and the median fluorescence intensity (MFI) was evaluated at pH 7.4 and pH 6.0. The data were analyzed using the XLfit software (IDBS) to determine and $EC_{50}$ for NUGC-4. The results demonstrate that none of Antibody 1 to Antibody 4 showed lowered activity because of preparation of ADCs (Figure 5).

[Example 6: Evaluation of *ex vivo* stability of ADC in plasma]

Stability of ADC6 to ADC10 prepared in Test Example 4 in mouse and human plasma samples was evaluated. ADCs adjusted to 1 mg/ml were added to the final concentration of 20 μg/ml to the human and BALB/c mouse plasma samples (anticoagulant: heparin sodium) and the resultant was incubated for 48 hours. The incubation sample was deproteinated with ethanol or acetonitrile/methanol (9/1, v/v), and the drug released from the ADC extract was assayed by LC/MS/MS. The free payload release (%) was determined in accordance with the equation indicated below.

$$\text{Free payload release (\%)} = \frac{\text{Free payload concentration (nmol/L)}}{\text{ADC concentration in incubation solution (20 }\mu\text{g/mL) x 1000 x 1000 / ADC MW x DAR}} \text{ x 100}$$

The results demonstrate that ADC6 to ADC9 are stable in mouse and human plasma samples (Figure 6).

[Example 7: Cytotoxicity mediated by ADC]

ADC1 to ADC3 were prepared in the manner described in Example 4, the cytotoxicity thereof on the gastric cancer cell line NUGC-3 was evaluated by the CellTiter-Glo 2.0 luminescent cell viability assay (Promega). The cells were seeded at 1000 cells/well on a 96-well plate, ADCs were added, and culture was performed at 37°C for 5 days. Thereafter, the CellTiter-Glo 2.0 luminescent cell viability assay was performed in accordance with the manufacturer's instructions. The relative luminescence was measured using the EnSpire Multimode Plate Reader (PerkinElmer), the data were analyzed using the XLfit software (IDBS), and $IC_{50}$ for each cancer cell line was determined (Figure 7). The results demonstrate that Antibody 1 to Antibody 3 did not exert the cytotoxicity on NUGC-3 by themselves but ADCs comprising Antibody 1 to Antibody 3 exerted the cytotoxicity on NUGC-3. That is, the ADCs of the present invention are useful as anti-tumor agents.

Separately, ADC4 to ADC10 comprising different drug linkers were prepared in the manner described in Example 4, the cytotoxicity thereof on the pancreatic cancer cell line (Capan-1) and the gastric cancer cell line (NUGC-4) was evaluated in the manner described above (Figure 8). The results demonstrate that all ADCs except for ADC7 exert growth inhibitory effects on NUGC-4 and Capan-1. MC-GGFG-Dxd, which is a drug linker used for ADC7, is the same as, for example, the drug linker used for the commercialized antitumor agent, trastuzumab deruxtecan (ADC of the anti-HER2 antibody). That is, the results do not indicate that the drug linker indicated above is inactive. Specifically, a drug linker having activity would not always exert its activity when it is formulated into ADC because of the correlation thereof with the antibody. In addition, ADC8 comprising MC-GGFG-Dx8951 as a drug linker that is structurally similar to the drug linker of ADC7 exerts growth inhibitory effects. Accordingly, it is difficult to accurately predict as to whether or not a drug linker would exert its activity when formulated into ADC.

[Example 8: Anti-tumor effects of ADC (*in vivo*)]

Tumor-bearing animal models were prepared using the cells of the gastric cancer cell line (NUGC-4) and the cells of the pancreatic cancer cell line (BxPC-3). At the outset, the cancer cells were prepared and inoculated subcutaneously into the right chest of nude mice (BxPC-3: $5 \times 10^6$ cells/100 ul; NUGC-4: $2 \times 10^6$ cells/100 ul). When the volume of the inoculated tumor was increased to approximately 100 to 300 $mm^3$, the nude mice were randomly divided into a control group (PBS administration) and test groups, and ADC6 was injected intravenously thereinto at 5 mg/kg, 10 mg/kg, or 20 mg/kg. Administration was performed every week, every two weeks, or a single time, as shown in Figure 9. The tumor volume was measured two times a week, and the tumor volume was calculated in accordance with the equation indicated below:

$$\text{Tumor volume (mm}^3\text{)} = (\text{long axis} \times \text{short axis}^2)/2$$

As shown in Figure 9, it was confirmed 7 days after the initiation of the test that the tumor growth was more inhibited in the ADC6 administration groups, compared with the control group. The results demonstrate that the ADC of the present invention exerts significant growth inhibitory effects on pancreatic cancer and gastric cancer. While target expression was observed in healthy cell lines (MSC and HUVEC) at pH 6 with the use of Antibody 4, body weight loss or the like was not observed in the group to which the ADC of the present invention was administered at a high dose. The results indicate that the ADC of the present invention binds in a tumor-tissue-selective manner because of its pH-dependence, it has high safety, and it is useful for therapeutic applications.

Sequence Listing Free Text

SEQ ID NO: 1: the amino acid sequence of the heavy chain CDR1 of Antibody 1 and Antibody 4;
SEQ ID NO: 2: the amino acid sequence of the heavy chain CDR2 of Antibody 1 and Antibody 4;
SEQ ID NO: 3: the amino acid sequence of the heavy chain CDR3 of Antibody 1 and Antibody 4;
SEQ ID NO: 4: the amino acid sequence of the light chain CDR1 of Antibody 1 and Antibody 4;
LGS: the amino acid sequence of the light chain CDR2 of Antibody 1 and Antibody 4;
SEQ ID NO: 6: the amino acid sequence of the light chain CDR3 of Antibody 1 and Antibody 4;
SEQ ID NO: 7: the amino acid sequence of the heavy chain CDR1 of Antibody 2;
SEQ ID NO: 8: the amino acid sequence of the heavy chain CDR2 of Antibody 2;
SEQ ID NO: 9: the amino acid sequence of the heavy chain CDR3 of Antibody 2;
SEQ ID NO: 10: the amino acid sequence of the light chain CDR1 of Antibody 2;
AAS: the amino acid sequence of the light chain CDR2 of Antibody 2;
SEQ ID NO: 12: the amino acid sequence of the light chain CDR3 of Antibody 2;
SEQ ID NO: 13: the amino acid sequence of the heavy chain CDR1 of Antibody 3;
SEQ ID NO: 14: the amino acid sequence of the heavy chain CDR2 of Antibody 3;
SEQ ID NO: 15: the amino acid sequence of the heavy chain CDR3 of Antibody 3;
SEQ ID NO: 16: the amino acid sequence of the light chain CDR1 of Antibody 3;
WAS: the amino acid sequence of the light chain CDR2 of Antibody 3;
SEQ ID NO: 18: the amino acid sequence of the light chain CDR3 of Antibody 3;
SEQ ID NO: 19: the nucleotide sequence of cDNA encoding a heavy chain variable region of Antibody 1;
SEQ ID NO: 20: the amino acid sequence of a heavy chain variable region of Antibody 1;
SEQ ID NO: 21: the nucleotide sequence of cDNA encoding a light chain variable region of Antibody 1;
SEQ ID NO: 22: the amino acid sequence of a light chain variable region of Antibody 1;
SEQ ID NO: 23: the nucleotide sequence of cDNA encoding a heavy chain variable region of Antibody 2;
SEQ ID NO: 24: the amino acid sequence of a heavy chain variable region of Antibody 2;
SEQ ID NO: 25: the nucleotide sequence of cDNA encoding a light chain variable region of Antibody 2;
SEQ ID NO: 26: the amino acid sequence of a light chain variable region of Antibody 2;
SEQ ID NO: 27: the nucleotide sequence of cDNA encoding a heavy chain variable region of Antibody 3;
SEQ ID NO: 28: the amino acid sequence of a heavy chain variable region of Antibody 3;
SEQ ID NO: 29: the nucleotide sequence of cDNA encoding a light chain variable region of Antibody 3;
SEQ ID NO: 30: the amino acid sequence of a light chain variable region of Antibody 3;
SEQ ID NO: 31: the nucleotide sequence of cDNA encoding a heavy chain variable region of Antibody 4;
SEQ ID NO: 32: the amino acid sequence of a heavy chain variable region of Antibody 4;
SEQ ID NO: 33: the nucleotide sequence of cDNA encoding a light chain variable region of Antibody 4;
SEQ ID NO: 34: the amino acid sequence of a light chain variable region of Antibody 4;
SEQ ID NO: 35: the nucleotide sequence of the heavy chain of Antibody 1;
SEQ ID NO: 36: the amino acid sequence of the heavy chain of Antibody 1;
SEQ ID NO: 37: the nucleotide sequence of the light chain of Antibody 1;
SEQ ID NO: 38: the amino acid sequence of the light chain of Antibody 1;
SEQ ID NO: 39: the nucleotide sequence of the heavy chain of Antibody 2;
SEQ ID NO: 40: the amino acid sequence of the heavy chain of Antibody 2;
SEQ ID NO: 41: the nucleotide sequence of the light chain of Antibody 2;
SEQ ID NO: 42: the amino acid sequence of the light chain of Antibody 2;
SEQ ID NO: 43: the nucleotide sequence of the heavy chain of Antibody 3;
SEQ ID NO: 44: the amino acid sequence of the heavy chain of Antibody 3;
SEQ ID NO: 45: the nucleotide sequence of the light chain of Antibody 3;
SEQ ID NO: 46: the amino acid sequence of the light chain of Antibody 3;

SEQ ID NO: 47: the nucleotide sequence of the heavy chain of Antibody 4;
SEQ ID NO: 48: the amino acid sequence of the heavy chain of Antibody 4;
SEQ ID NO: 49: the nucleotide sequence of the light chain of Antibody 4; and
SEQ ID NO: 50: the amino acid sequence of the light chain of Antibody 4.

All publications, patents, and patent applications cited herein are incorporated herein by reference in their entirety.

## Claims

1. An antibody binding specifically to sulfated glycosaminoglycan, which is at least one antibody selected from the group consisting of (1) to (3) below:

   (1) an antibody comprising

   a heavy chain that comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 and
   a light chain that comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6;

   (2) an antibody comprising

   a heavy chain that comprises CDR1 consisting of the amino acid sequence represented by SEQ ID NO: 7, CDR2 consisting of the amino acid sequence represented by SEQ ID NO: 8, and CDR3 consisting of the amino acid sequence represented by SEQ ID NO: 9 and
   a light chain that comprises CDR1 consisting of the amino acid sequence represented by SEQ ID NO: 10, CDR2 consisting of the amino acid sequence represented by AAS, and CDR3 consisting of the amino acid sequence represented by SEQ ID NO: 12; and

   (3) an antibody comprising

   a heavy chain that comprises CDR1 consisting of the amino acid sequence represented by SEQ ID NO: 13, CDR2 consisting of the amino acid sequence represented by SEQ ID NO: 14, and CDR3 consisting of the amino acid sequence represented by SEQ ID NO: 15 and
   a light chain that comprises CDR1 consisting of the amino acid sequence represented by SEQ ID NO: 16, CDR2 consisting of the amino acid sequence represented by WAS, and CDR3 consisting of the amino acid sequence represented by SEQ ID NO: 18.

2. The antibody according to claim 1, which is at least one antibody selected from the group consisting of (1) to (4) below:

   (1) an antibody comprising

   a heavy chain comprising a variable region that consists of the amino acid sequence represented by SEQ ID NO: 20 or a heavy chain comprising a variable region that has at least 90% sequence identity to the amino acid sequence represented by SEQ ID NO: 20 and having activity of binding to sulfated glycosaminoglycan and
   a light chain comprising a variable region that consists of the amino acid sequence represented by SEQ ID NO: 22 or a light chain comprising a variable region that has at least 90% sequence identity to the amino acid sequence represented by SEQ ID NO: 22 and has activity of binding to sulfated glycosaminoglycan;

   (2) an antibody comprising

   a heavy chain comprising a variable region that consists of the amino acid sequence represented by SEQ ID NO: 24 or a heavy chain comprising a variable region that has at least 90% sequence identity to the amino acid sequence represented by SEQ ID NO: 24 and having activity of binding to sulfated glycosaminoglycan

and
a light chain comprising a variable region that consists of the amino acid sequence represented by SEQ ID NO: 26 or a light chain comprising a variable region that has at least 90% sequence identity to the amino acid sequence represented by SEQ ID NO: 26 and has activity of binding to sulfated glycosaminoglycan;

(3) an antibody comprising

a heavy chain comprising a variable region that consists of the amino acid sequence represented by SEQ ID NO: 28 or a heavy chain comprising a variable region that has at least 90% sequence identity to the amino acid sequence represented by SEQ ID NO: 28 and has activity of binding to sulfated glycosaminoglycan and
a light chain comprising a variable region that consists of the amino acid sequence represented by SEQ ID NO: 30 or a light chain comprising a variable region that has at least 90% sequence identity to the amino acid sequence represented by SEQ ID NO: 30 and has activity of binding to sulfated glycosaminoglycan; and

(4) an antibody comprising

a heavy chain comprising a variable region that consists of the amino acid sequence represented by SEQ ID NO: 32 or a heavy chain comprising a variable region that has at least 90% sequence identity to the amino acid sequence represented by SEQ ID NO: 32 and has activity of binding to sulfated glycosaminoglycan and
a light chain comprising a variable region that consists of the amino acid sequence represented by SEQ ID NO: 34 or a light chain comprising a variable region that has at least 90% sequence identity to the amino acid sequence represented by SEQ ID NO: 34 and has activity of binding to sulfated glycosaminoglycan.

**3.** The antibody according to claim 1 or 2, which is at least one antibody selected from the group consisting of (1) to (4) below:

(1) an antibody comprising

a heavy chain consisting of the amino acid sequence represented by SEQ ID NO: 36 or a heavy chain having at least 90% sequence identity to the amino acid sequence represented by SEQ ID NO: 36 and having activity of binding to sulfated glycosaminoglycan and
a light chain consisting of the amino acid sequence represented by SEQ ID NO: 38 or a light chain having at least 90% sequence identity to the amino acid sequence represented by SEQ ID NO: 38 and having activity of binding to sulfated glycosaminoglycan;

(2) an antibody comprising

a heavy chain consisting of the amino acid sequence represented by SEQ ID NO: 40 or a heavy chain having at least 90% sequence identity to the amino acid sequence represented by SEQ ID NO: 40 and having activity of binding to sulfated glycosaminoglycan and
a light chain consisting of the amino acid sequence represented by SEQ ID NO: 42 or a light chain having at least 90% sequence identity to the amino acid sequence represented by SEQ ID NO: 42 and has activity of binding to sulfated glycosaminoglycan;

(3) an antibody comprising

a heavy chain consisting of the amino acid sequence represented by SEQ ID NO: 44 or a heavy chain having at least 90% sequence identity to the amino acid sequence represented by SEQ ID NO: 44 and having activity of binding to sulfated glycosaminoglycan and
a light chain consisting of the amino acid sequence represented by SEQ ID NO: 46 or a light chain having at least 90% sequence identity to the amino acid sequence represented by SEQ ID NO: 46 and having activity of binding to sulfated glycosaminoglycan; and

(4) an antibody comprising

a heavy chain consisting of the amino acid sequence represented by SEQ ID NO: 48 or a heavy chain having at least 90% sequence identity to the amino acid sequence represented by SEQ ID NO: 48 and having activity of binding to sulfated glycosaminoglycan and

a light chain consisting of the amino acid sequence represented by SEQ ID NO: 50 or a light chain having at least 90% sequence identity to the amino acid sequence represented by SEQ ID NO: 50 and having activity of binding to sulfated glycosaminoglycan.

**4.** The antibody according to any one of claims 1 to 3, which is a human antibody.

**5.** The antibody according to any one of claims 1 to 4, which is an antibody of the IgG1 subclass.

**6.** The antibody according to any one of claims 1 to 5, which binds with high affinity in an acidic pH range.

**7.** An antibody-drug conjugate (ADC) represented by Formula 1:

Ab-(L-D)p (1)

wherein
Ab represents an antibody binding specifically to sulfated glycosaminoglycan, which is at least one antibody selected from the group consisting of (1) to (3) below:

(1) an antibody comprising

a heavy chain that comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 and
a light chain that comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6;

(2) an antibody comprising

a heavy chain that comprises CDR1 consisting of the amino acid sequence represented by SEQ ID NO: 7, CDR2 consisting of the amino acid sequence represented by SEQ ID NO: 8, and CDR3 consisting of the amino acid sequence represented by SEQ ID NO: 9 and
a light chain that comprises CDR1 consisting of the amino acid sequence represented by SEQ ID NO: 10, CDR2 consisting of the amino acid sequence represented by AAS, and CDR3 consisting of the amino acid sequence represented by SEQ ID NO: 12; and

(3) an antibody comprising

a heavy chain that comprises CDR1 consisting of the amino acid sequence represented by SEQ ID NO: 13, CDR2 consisting of the amino acid sequence represented by SEQ ID NO: 14, and CDR3 consisting of the amino acid sequence represented by SEQ ID NO: 15 and
a light chain that comprises CDR1 consisting of the amino acid sequence represented by SEQ ID NO: 16, CDR2 consisting of the amino acid sequence represented by WAS, and CDR3 consisting of the amino acid sequence represented by SEQ ID NO: 18,

wherein

L represents 6-maleimidocaproyl-valine-citrulline-p-aminobenzyloxycarbonyl (MC-Val-Cit-PAB), 6-maleimidocaproyl-glycine-glycine-phenylalanine-glycine (MC-GGFG), N-succinimidyl 4-(N-maleimidomethyl) cyclohexane-1-carboxylate (SMCC), 4-(2-pyridyldithio)butyric acid N-hydroxysuccinimide ester (SPDB), or MC-CL2;
D represents monomethyl auristatin, maytansinoid, or a camptothecin derivative; and
p is an integer selected from among 1 to 12.

**8.** The antibody-drug conjugate (ADC) according to claim 7, wherein Ab represents at least one antibody selected from the group consisting of (1) to (4) below:

(1) an antibody comprising

a heavy chain comprising a variable region that consists of the amino acid sequence represented by SEQ ID NO: 20 or a heavy chain comprising a variable region that has at least 90% sequence identity to the amino acid sequence represented by SEQ ID NO: 20 and having activity of binding to sulfated glycosaminoglycan and
a light chain comprising a variable region that consists of the amino acid sequence represented by SEQ ID NO: 22 or a light chain comprising a variable region that has at least 90% sequence identity to the amino acid sequence represented by SEQ ID NO: 22 and has activity of binding to sulfated glycosaminoglycan;

(2) an antibody comprising

a heavy chain comprising a variable region that consists of the amino acid sequence represented by SEQ ID NO: 24 or a heavy chain comprising a variable region that has at least 90% sequence identity to the amino acid sequence represented by SEQ ID NO: 24 and having activity of binding to sulfated glycosaminoglycan and
a light chain comprising a variable region that consists of the amino acid sequence represented by SEQ ID NO: 26 or a light chain comprising a variable region that has at least 90% sequence identity to the amino acid sequence represented by SEQ ID NO: 26 and has activity of binding to sulfated glycosaminoglycan;

(3) an antibody comprising

a heavy chain comprising a variable region that consists of the amino acid sequence represented by SEQ ID NO: 28 or a heavy chain comprising a variable region that has at least 90% sequence identity to the amino acid sequence represented by SEQ ID NO: 28 and has activity of binding to sulfated glycosaminoglycan and
a light chain comprising a variable region that consists of the amino acid sequence represented by SEQ ID NO: 30 or a light chain comprising a variable region that has at least 90% sequence identity to the amino acid sequence represented by SEQ ID NO: 30 and has activity of binding to sulfated glycosaminoglycan; and

(4) an antibody comprising

a heavy chain comprising a variable region that consists of the amino acid sequence represented by SEQ ID NO: 32 or a heavy chain comprising a variable region that has at least 90% sequence identity to the amino acid sequence represented by SEQ ID NO: 32 and has activity of binding to sulfated glycosaminoglycan and
a light chain comprising a variable region that consists of the amino acid sequence represented by SEQ ID NO: 34 or a light chain comprising a variable region that has at least 90% sequence identity to the amino acid sequence represented by SEQ ID NO: 34 and has activity of binding to sulfated glycosaminoglycan.

**9.** The antibody-drug conjugate (ADC) according to claim 7 or 8, wherein Ab represents at least one antibody selected from the group consisting of (1) to (4) below:

(1) an antibody comprising

a heavy chain consisting of the amino acid sequence represented by SEQ ID NO: 36 or a heavy chain having at least 95% sequence identity to the amino acid sequence represented by SEQ ID NO: 36 and having activity of binding to sulfated glycosaminoglycan and
a light chain consisting of the amino acid sequence represented by SEQ ID NO: 38 or a light chain having at least 95% sequence identity to the amino acid sequence represented by SEQ ID NO: 38 and having activity of binding to sulfated glycosaminoglycan;

(2) an antibody comprising

a heavy chain consisting of the amino acid sequence represented by SEQ ID NO: 40 or a heavy chain having at least 95% sequence identity to the amino acid sequence represented by SEQ ID NO: 40 and having activity of binding to sulfated glycosaminoglycan and
a light chain consisting of the amino acid sequence represented by SEQ ID NO: 42 or a light chain having at least 95% sequence identity to the amino acid sequence represented by SEQ ID NO: 42 and has activity of binding to sulfated glycosaminoglycan;

(3) an antibody comprising

a heavy chain consisting of the amino acid sequence represented by SEQ ID NO: 44 or a heavy chain having at least 95% sequence identity to the amino acid sequence represented by SEQ ID NO: 44 and having activity of binding to sulfated glycosaminoglycan and
a light chain consisting of the amino acid sequence represented by SEQ ID NO: 46 or a light chain having at least 95% sequence identity to the amino acid sequence represented by SEQ ID NO: 46 and having activity of binding to sulfated glycosaminoglycan; and

(4) an antibody comprising

a heavy chain consisting of the amino acid sequence represented by SEQ ID NO: 48 or a heavy chain having at least 95% sequence identity to the amino acid sequence represented by SEQ ID NO: 48 and having activity of binding to sulfated glycosaminoglycan and
a light chain consisting of the amino acid sequence represented by SEQ ID NO: 50 or a light chain having at least 95% sequence identity to the amino acid sequence represented by SEQ ID NO: 50 and having activity of binding to sulfated glycosaminoglycan.

**10.** The antibody-drug conjugate (ADC) according to any one of claims 7 to 9, wherein Ab represents an antibody comprising a heavy chain that comprises CDR1 consisting of the amino acid sequence represented by SEQ ID NO: 1, CDR2 consisting of the amino acid sequence represented by SEQ ID NO: 2, and CDR3 consisting of the amino acid sequence represented by SEQ ID NO: 3 and a light chain that comprises CDR1 consisting of the amino acid sequence represented by SEQ ID NO: 4, CDR2 consisting of the amino acid sequence represented by LGS, and CDR3 consisting of the amino acid sequence represented by SEQ ID NO: 6.

**11.** The antibody-drug conjugate (ADC) according to any one of claims 7 to 10, wherein Ab represents an antibody comprising a heavy chain comprising a variable region that consists of the amino acid sequence represented by SEQ ID NO: 20 and a light chain comprising a variable region that consists of the amino acid sequence represented by SEQ ID NO: 22.

**12.** The antibody-drug conjugate (ADC) according to any one of claims 7 to 11, wherein Ab represents an antibody comprising a heavy chain consisting of the amino acid sequence represented by SEQ ID NO: 36 and a light chain consisting of the amino acid sequence represented by SEQ ID NO: 38.

**13.** The antibody-drug conjugate (ADC) according to any one of claims 7 to 10, wherein Ab represents an antibody comprising a heavy chain comprising a variable region that consists of the amino acid sequence represented by SEQ ID NO: 32 and a light chain comprising a variable region that consists of the amino acid sequence represented by SEQ ID NO: 34.

**14.** The antibody-drug conjugate (ADC) according to any one of claims 7 to 10, wherein Ab represents an antibody comprising a heavy chain consisting of the amino acid sequence represented by SEQ ID NO: 48 and a light chain consisting of the amino acid sequence represented by SEQ ID NO: 50.

**15.** The antibody-drug conjugate (ADC) according to any one of claims 7 to 14, wherein Ab represents the IgG1 antibody.

**16.** The antibody-drug conjugate (ADC) according to any one of claims 7 to 15, wherein Ab represents a human antibody.

**17.** The antibody-drug conjugate (ADC) according to any one of claims 7 to 16, wherein Ab represents an antibody that binds with higher affinity in an acidic pH range than in a neutral pH range.

**18.** The antibody-drug conjugate (ADC) according to any one of claims 7 to 17, wherein L represents 6-maleimidocaproyl-valine-citrulline-p-aminobenzyloxycarbonyl (MC-Val-Cit-PAB), 6-maleimidocaproyl-glycine-glycine-phenylalanine-glycine (MC-GGFG), N-succinimidyl 4-(N-maleimidomethyl)cyclohexane-1-carboxylate (SMCC), 4-(2-pyridyldithio)butyric acid N-hydroxysuccinimide ester (SPDB), or MC-CL2.

**19.** The antibody-drug conjugate (ADC) according to any one of claims 7 to 17, wherein D represents MMAE, MMAF, DM1, DM4, Dx8951, Dxd, or SN-38.

**20.** The antibody-drug conjugate (ADC) according to any one of claims 7 to 19, wherein L-D represents SMCC-DM1, SPDB-DM4, MC-Val-Cit-PAB-MMAE, MC-Val-Cit-PAB-MMAF, MC-GGFG-Dx8591, or MC-CL2-SN-38.

**21.** The antibody-drug conjugate (ADC) according to any one of claims 7 to 10 or claims 15 to 20, wherein

Ab represents an antibody comprising a heavy chain comprising a variable region that consists of the amino acid sequence represented by SEQ ID NO: 20 and a light chain comprising a variable region that consists of the amino acid sequence represented by SEQ ID NO: 22; and
L-D represents MC-Val-Cit-PAB-MMAE.

**22.** The antibody-drug conjugate (ADC) according to any one of claims 7 to 10 or claims 15 to 20, wherein

Ab represents an antibody comprising a heavy chain consisting of the amino acid sequence represented by SEQ ID NO: 36 and a light chain consisting of the amino acid sequence represented by SEQ ID NO: 38; and
L-D represents MC-Val-Cit-PAB-MMAE.

**23.** The antibody-drug conjugate (ADC) according to any one of claims 7 to 10 or claims 15 to 20, wherein

Ab represents an antibody comprising a heavy chain comprising a variable region that consists of the amino acid sequence represented by SEQ ID NO: 32 and a light chain comprising a variable region that consists of the amino acid sequence represented by SEQ ID NO: 34; and
L-D represents MC-Val-Cit-PAB-MMAE.

**24.** The antibody-drug conjugate (ADC) according to any one of claims 7 to 10 or claims 15 to 20, wherein

Ab represents an antibody comprising a heavy chain consisting of the amino acid sequence represented by SEQ ID NO: 48 and a light chain consisting of the amino acid sequence represented by SEQ ID NO: 50; and
L-D represents MC-Val-Cit-PAB-MMAE.

**25.** The antibody-drug conjugate (ADC) according to any one of claims 7 to 24, wherein p is 3 to 8.

**26.** A pharmaceutical composition comprising the antibody-drug conjugate (ADC) according to any one of claims 7 to 25 and a carrier.

**27.** An antitumor agent comprising, as an active ingredient, the antibody-drug conjugate (ADC) according to any one of claims 7 to 25.

**28.** The antitumor agent according to claim 27, which is used for treating cancer that expresses sulfated glycosaminoglycan.

**29.** The antitumor agent according to claim 27, which is used for treating gastric cancer, pancreatic cancer, breast cancer, large bowel cancer, or lung cancer.

Fig. 1

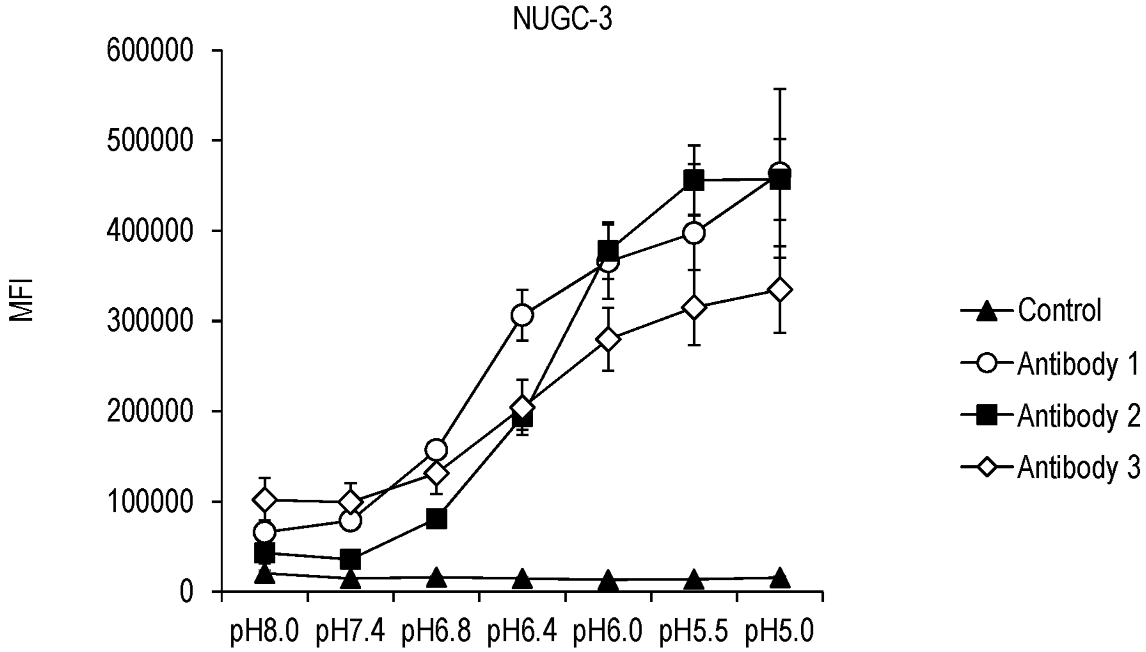
NUGC-3
MFI
600000
500000
400000
300000
200000
100000
0
pH8.0
pH7.4
pH6.8
pH6.4
pH6.0
pH5.5
pH5.0
Control
Antibody 1
Antibody 2
Antibody 3

Fig. 2

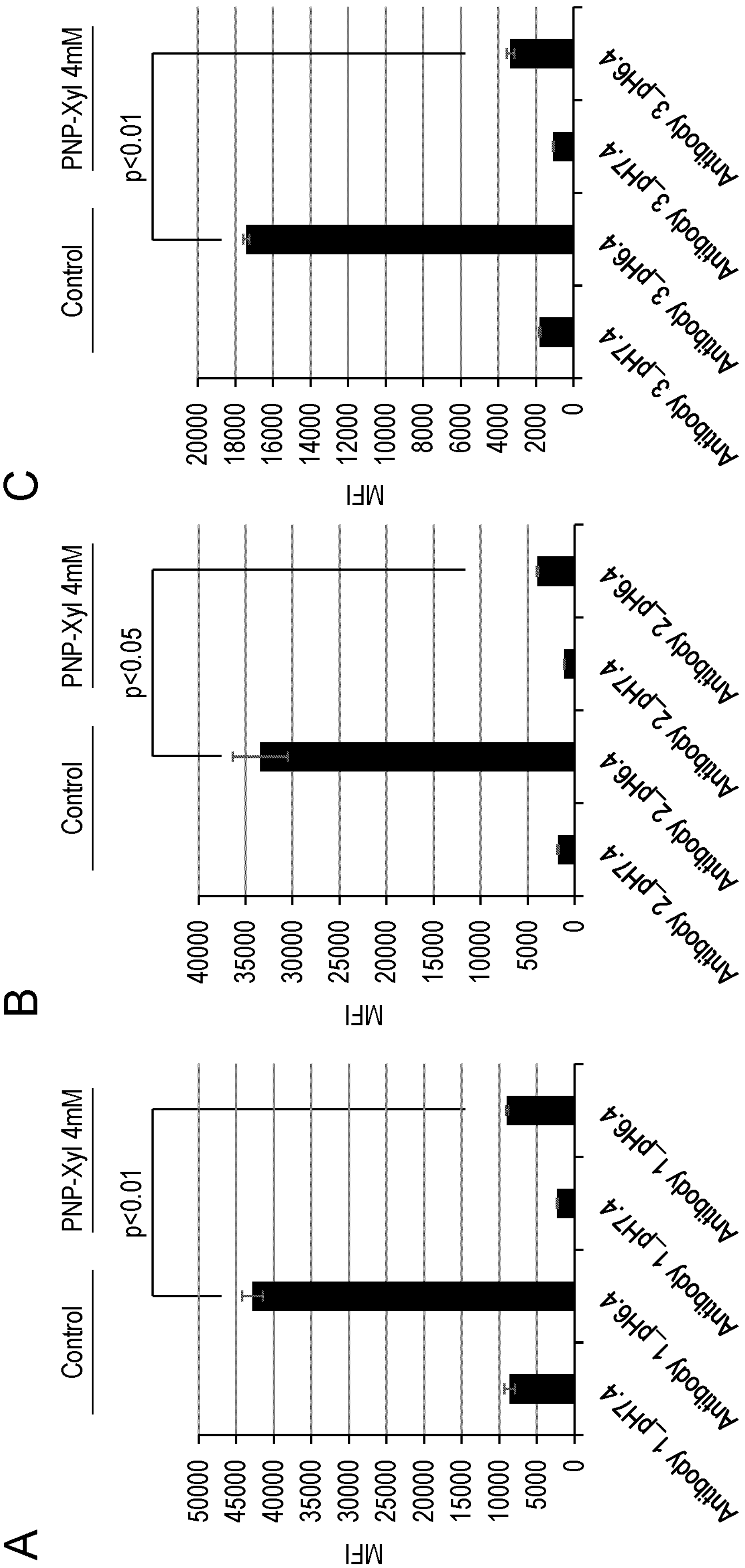
A
B
C
MFI
Control
PNP-Xyl 4mM
p<0.01
p<0.05
Antibody 1_pH7.4
Antibody 1_pH6.4
Antibody 2_pH7.4
Antibody 2_pH6.4
Antibody 3_pH7.4
Antibody 3_pH6.4
50000
45000
40000
35000
30000
25000
20000
15000
10000
5000
0
20000
18000
16000
14000
12000
10000
8000
6000
4000
2000

Fig. 3

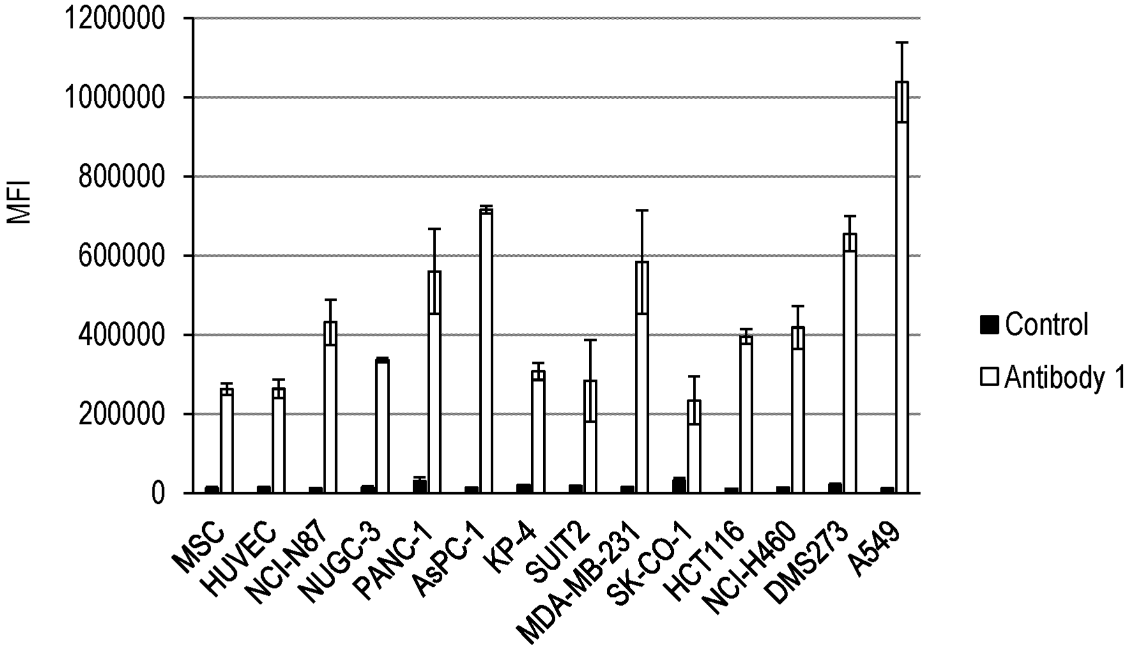
1200000
1000000
800000
600000
400000
200000
0
MFI
Control
Antibody 1
MSC
HUVEC
NCI-N87
NUGC-3
PANC-1
AsPC-1
KP-4
SUIT2
MDA-MB-231
SK-CO-1
HCT116
NCI-H460
DMS273
A549

# Fig. 4-1

| | Antibody | Name and structure of linker-payload | |
|---|---|---|---|
| ADC1 | Antibody 1 | SMCC-DM1 | 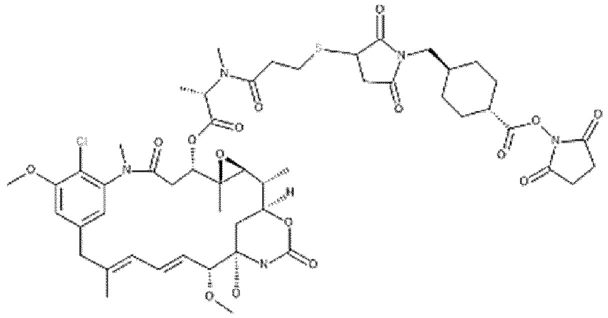 |
| ADC2 | Antibody 2 | SMCC-DM1 | 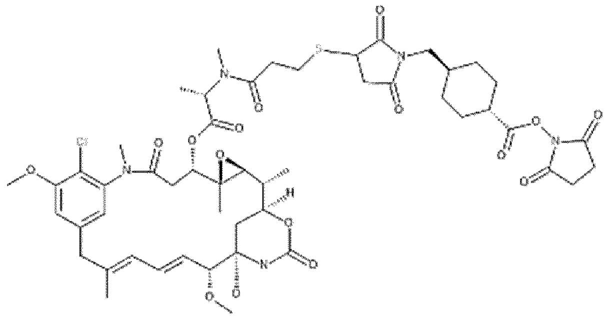 |
| ADC3 | Antibody 3 | SMCC-DM1 | 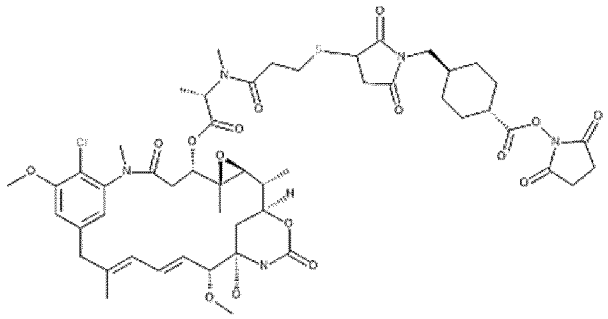 |
| ADC4 | Antibody 4 | SMCC-DM1 | 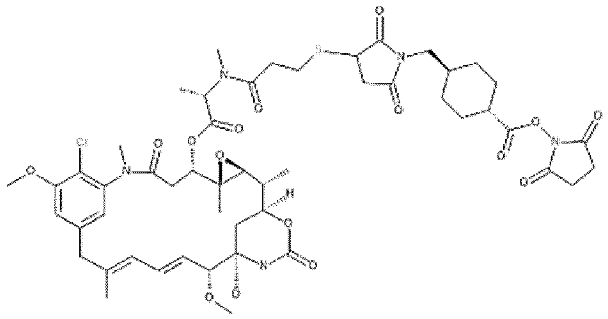 |
| ADC5 | Antibody 4 | SPDB-DM4 | 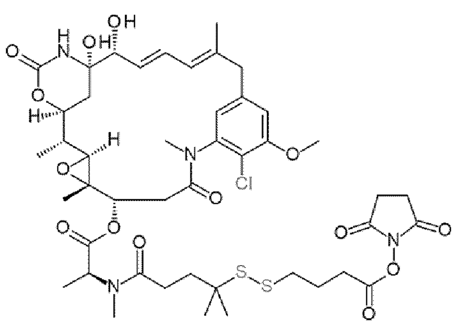 |

# Fig. 4-2

| | Antibody | Name and structure of linker-payload | |
|---|---|---|---|
| ADC6 | Antibody 4 | MC-VC-PAB-MMAE | 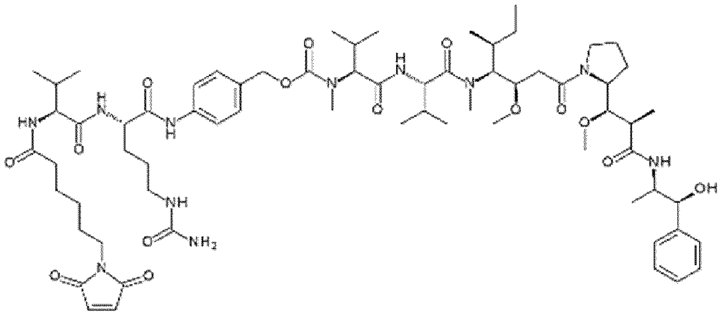 |
| ADC7 | Antibody 4 | MC-GGFG-Dxd | 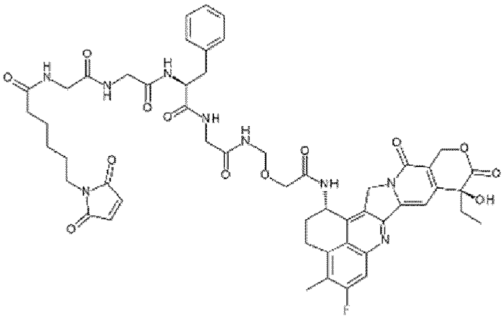 |
| ADC8 | Antibody 4 | MC-GGFG-Dx8951 | 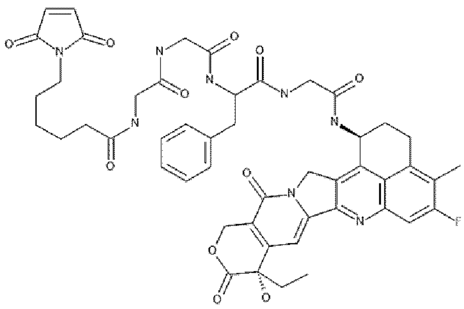 |
| ADC9 | Antibody 4 | MC-Val-Cit-PAB-MMAF | 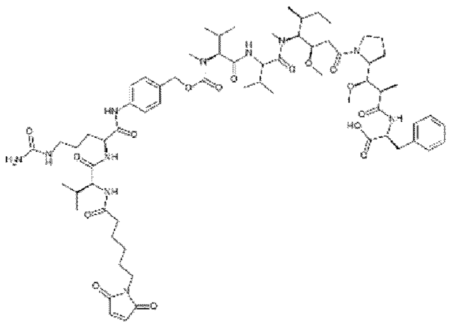 |
| ADC10 | Antibody 4 | MC-CL2-SN-38 | 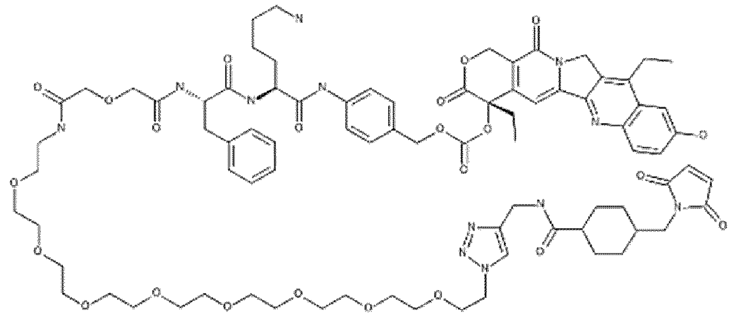 |

Fig. 5

| | $EC_{50}$ (μg/mL) _pH7.4 | $EC_{50}$ (μg/mL) _pH6.0 |
|---|---|---|
| Antibody 1 | >300 | 1.5 |
| ADC1 | >300 | 1.3 |
| Antibody 2 | >300 | 1.6 |
| ADC2 | >300 | 1.6 |
| Antibody 3 | >300 | 11.6 |
| ADC3 | >300 | 12.7 |
| Antibody 4 | >300 | 3.2 |
| ADC4 | >300 | 2.4 |
| ADC5 | >300 | 2.2 |
| ADC6 | >300 | 2.0 |
| ADC7 | >300 | 2.1 |
| ADC8 | >300 | 1.9 |
| ADC9 | >300 | 1.9 |
| ADC10 | >300 | 1.7 |

Fig. 6

| | DAR | Free Payload Release (%) _37°C, 48h | |
|---|---|---|---|
| | | Human | Mouse |
| ADC6 | 6.8 | < 0.03 | 5.05 |
| ADC7 | 6.5 | 0.35 | 0.43 |
| ADC8 | 4.9 | < 0.08 | < 0.08 |
| ADC9 | 1.2 | < 0.09 | < 0.09 |
| ADC10 | 4.0 | > 93.98 | > 93.98 |

Fig. 7

| Name | DAR | $IC_{50}$ (ng/mL) |
|---|---|---|
| | | NUGC-3 |
| Antibody 1 | - | >10000 |
| ADC1 | 2.5 | 240 |
| Antibody 2 | - | >10000 |
| ADC2 | 3.5 | 30 |
| Antibody 3 | - | >10000 |
| ADC3 | 3.5 | 311 |

Fig. 8

| Name | DAR | $IC_{50}$ (ng/mL) | |
|---|---|---|---|
| | | NUGC-4 (Scirrhous, DGC) | Capan-1 (PDAC) |
| ADC4 | 2.9 | 4928 | 2378 |
| ADC5 | 3.1 | 927 | 2118 |
| ADC6 | 6.8 | 306 | 285 |
| ADC7 | 6.5 | >10000 | >10000 |
| ADC8 | 4.9 | 3986 | 1784 |
| ADC9 | 1.2 | 7021 | 3083 |
| ADC10 | 4.0 | 244 | 233 |

Fig. 9-1

BxPC-3

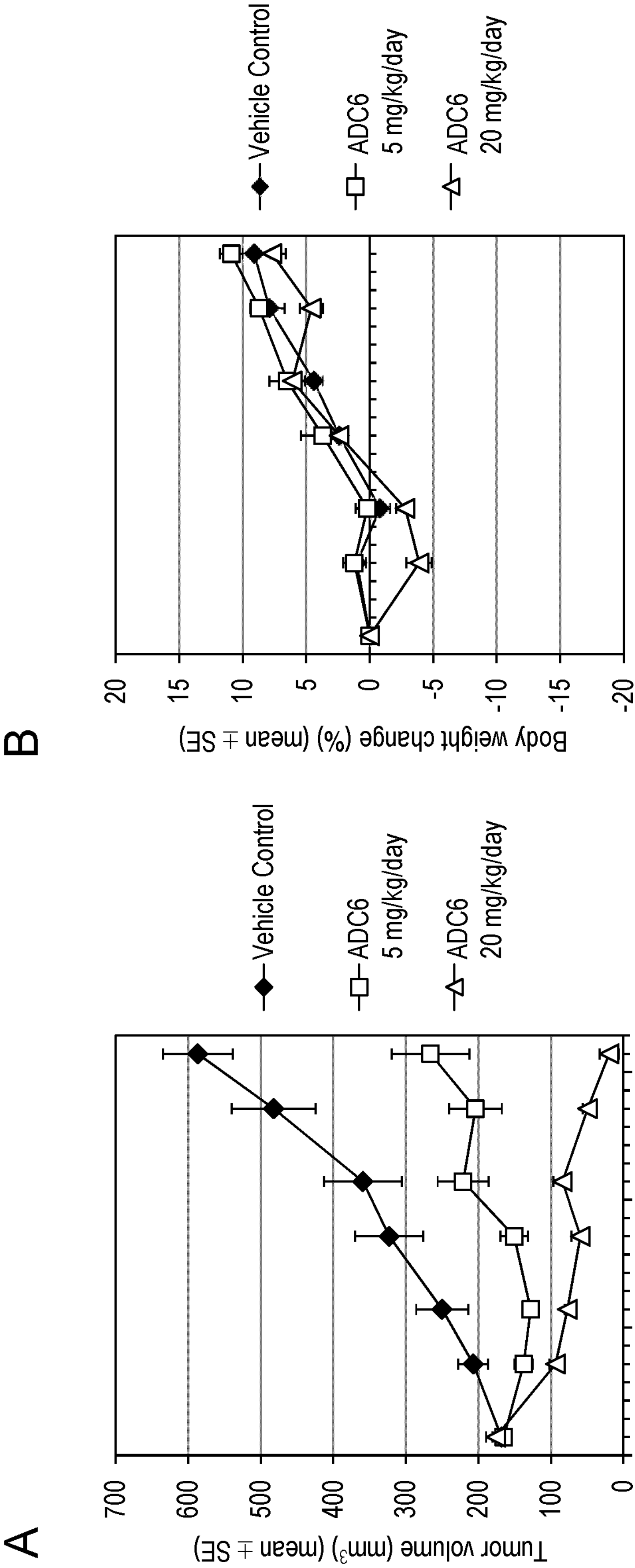
A
Tumor volume (mm3) (mean ± SE)
700
600
500
400
300
200
100
0
0
7
14
21
days
Vehicle Control
ADC6
5 mg/kg/day
ADC6
20 mg/kg/day
B
Body weight change (%) (mean ± SE)
20
15
10
5
0
-5
-10
-15
-20
0
7
14
21
days
Vehicle Control
ADC6
5 mg/kg/day
ADC6
20 mg/kg/day

Fig. 9-2

NUGC-4

A

B

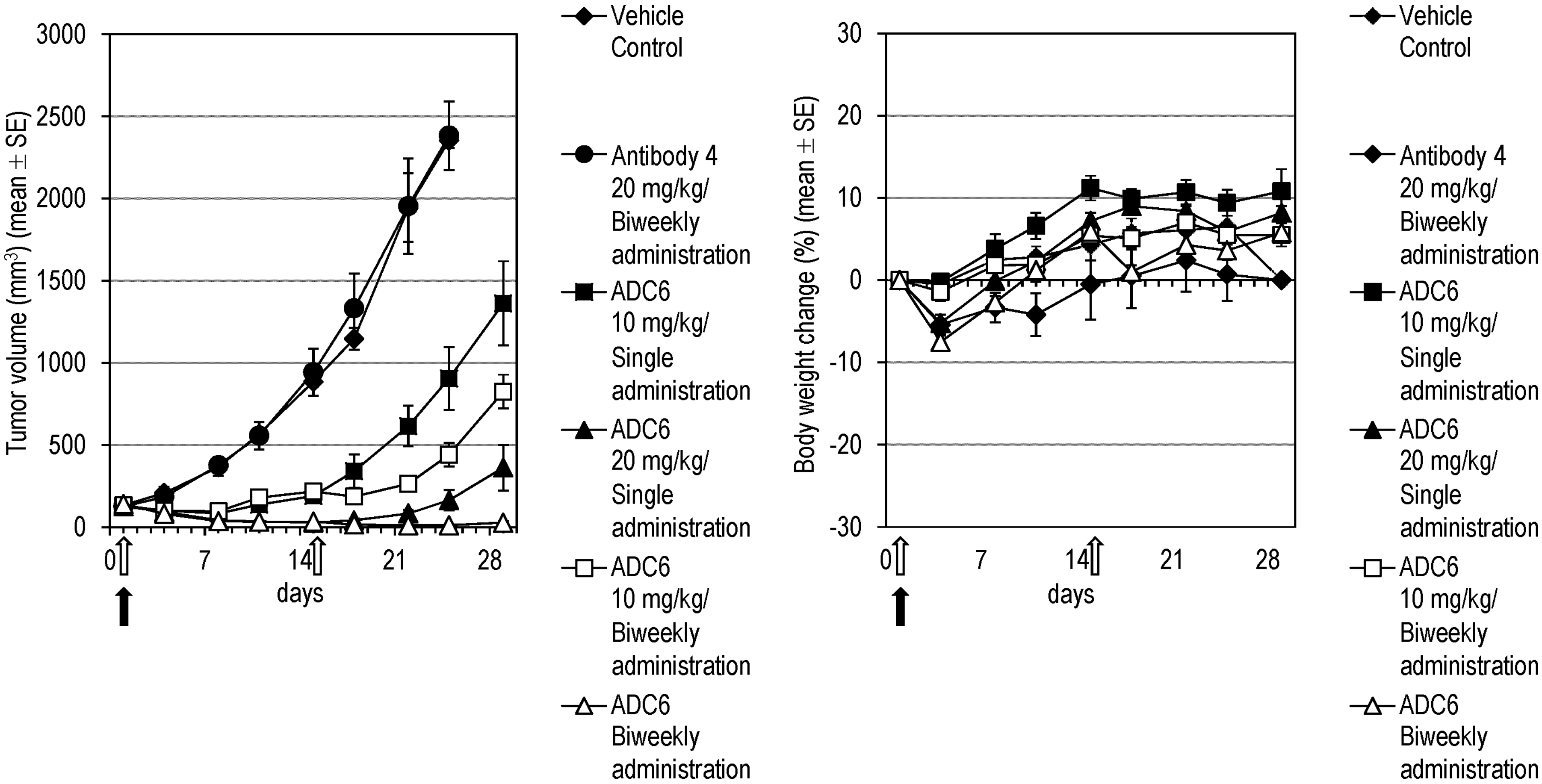

# Fig. 10

SEQ ID NO: 1
<223> Amino acid sequence of CDR1 of heavy chain of Antibody 1 and Antibody 4
GGFITSHH

SEQ ID NO: 2
<223> Amino acid sequence of CDR2 of heavy chain of Antibody 1 and Antibody 4
LHHTGTT

SEQ ID NO: 3
<223> Amino acid sequence of CDR3 of heavy chain of Antibody 1 and Antibody 4
VREDSSSWHPGRYIQL

SEQ ID NO: 4
<223> Amino acid sequence of CDR1 of light chain of Antibody 1 and Antibody 4
QSLLQNNRYNR

LGS
<223> Amino acid sequence of CDR2 of light chain of Antibody 1 and Antibody 4

SEQ ID NO: 6
<223> Amino acid sequence of CDR3 of light chain of Antibody 1 and Antibody 4
MHALEPPYT

SEQ ID NO: 7
<223> Amino acid sequence of CDR1 of heavy chain of Antibody 2
GFTFNTYT

SEQ ID NO: 8
<223> Amino acid sequence of CDR2 of heavy chain of Antibody 2
ISLSSNTI

SEQ ID NO: 9
<223> Amino acid sequence of CDR3 of heavy chain of Antibody 2
ARAESYYDSHYYYNGLDV

SEQ ID NO: 10
<223> Amino acid sequence of CDR1 of light chain of Antibody 2
QSVGGRY

AAS
<223> Amino acid sequence of CDR2 of light chain of Antibody 2

# Fig. 11

SEQ ID NO: 12
<223>  Amino acid sequence of CDR3 of light chain of Antibody 2
QQYGSSPRT

SEQ ID NO: 13
<223>  Amino acid sequence of CDR1 of heavy chain of Antibody 3
GFTFSDYY

SEQ ID NO: 14
<223>  Amino acid sequence of CDR2 of heavy chain of Antibody 3
ISISGTTR

SEQ ID NO: 15
<223>  Amino acid sequence of CDR3 of heavy chain of Antibody 3
ARGPELLSQNYYYYYGMDV

SEQ ID NO: 16
<223>  Amino acid sequence of CDR1 of light chain of Antibody 3
ETLLYRSNNKNY

WAS
<223>  Amino acid sequence of CDR2 of light chain of Antibody 3

SEQ ID NO: 18
<223>  Amino acid sequence of CDR3 of light chain of Antibody 3
QQYYTTPRT

SEQ ID NO: 19
<223>  Nucleotide sequence of cDNA encoding the variable region of the heavy chain of Antibody 1
CAGGTGCAGTTACAAGAGAGCGGCCCCGGCCTGGTGAAGCCCAGCGAGACCCTGTCCCTGACCTG
CACCGTGTCCGGCGGCTTCATCACCTCCCACCACTGGAGCTGGATCAGACAGCCCCCCGGCCAGG
GCCTGGAGTGGATCGGCTTCCTGCACCACACCGGCACCACCCACTACAACCCCTCCCTGAAGTCC
AGAGTGACCACCTCCGTGGACACCGCCAAGAACCAGTTCAGCCTGACCCTGAAGAGCCTGACCGC
CGCCGACACCGCCGTGTACTACTGCGTGAGGGAGGACTCCAGCAGCTGGCACCCCGGCAGATACA
TCCAGCTGTGGGGCCAGGGCACCCTGGTGACCGTGAGCGCC

SEQ ID NO: 20
<223>  Amino acid sequence of the variable region of the heavy chain of Antibody 1
QVQLQESGPGLVKPSETLSLTCTVSGGFITSHHWSWIRQPPGQGLEWIGFLHHTGTTHYNPSLKS
RVTTSVDTAKNQFSLTLKSLTAADTAVYYCVREDSSSWHPGRYIQLWGQGTLVTVSA

# Fig. 12

SEQ ID NO: 21
<223> Nucleotide sequence of cDNA encoding the variable region of the light chain of Antibody 1
GACGTAGTGATGACCCAGAGCCCCCTGAGCCTGCCCGTGACCCCCGGCGAGCCCGCCAGCATCAG
TTGCAGAAGCAGCCAGAGCCTGCTTCAAAACAACAGATACAACAGATTCGACTGGTATCTCCAAA
AGCCCGGCCAGAGCCCCAGAATCCTGATCTACCTGGGCAGCAACAGAGCCAGCGGCGTGCCCGAC
AGATTCAGCGGCACCACCAGCGGCACCGACTACACCCTGAGAATCAGCAGAGTGGAGGCCGAGGA
CGCCGGCGTGTACTACTGTATGCACGCCCTGGAGCCCCCCTACACCTTCGGCCAGGGCACCAAGC
TGGAGATCAAG

SEQ ID NO: 22
<223> Amino acid sequence of the variable region of the light chain of Antibody 1
DVVMTQSPLSLPVTPGEPASISCRSSQSLLQNNRYNRFDWYLQKPGQSPRILIYLGSNRASGVPD
RFSGTTSGTDYTLRISRVEAEDAGVYYCMHALEPPYTFGQGTKLEIK

SEQ ID NO: 23
<223> Nucleotide sequence of cDNA encoding the variable region of the heavy chain of Antibody 2
GAGGTGCAGCTGGTGGAGAGCGGCGGCGGCCTGGTGCAGCCCGGCGGCAGCCTGAGACTGAGCTG
CGCCGCCAGCGGCTTCACATTCAACACCTACACCATGAACTGGGTGAGACAGGCCCCCGGCAAGG
GCCTGGAGTGGGTGGCCTACATCAGCCTGAGCAGCAACACCATCTTCTACGCCCCTTCCGTGAAG
GGCCGGTTTACAGTGTCCCGGGATAATGCCAAGAATTCCCTGTACCTGCAGATGAATACACTGTC
CGATGATGACACAGCCGTGTACTACTGTGCCCGCGCCGAGAGCTACTACGACTCCCACTACTACT
ACAACGGCCTGGACGTGTGGGGCCAGGGCACCACCGTGTCCGTGACATCC

SEQ ID NO: 24
<223> Amino acid sequence of the variable region of the heavy chain of Antibody 2
EVQLVESGGGLVQPGGSLRLSCAASGFTFNTYTMNWVRQAPGKGLEWVAYISLSSNTIFYAPSVK
GRFTVSRDNAKNSLYLQMNTLSDDDTAVYYCARAESYYDSHYYYNGLDVWGQGTTVSVTS

SEQ ID NO: 25
<223> Nucleotide sequence of cDNA encoding the variable region of the light chain of Antibody 2
GAAATCGTGCTGACACAGTCCCCAGGCACACTGTCCCTGTCCCCAGGCGAGCGCGCCACCCTGAG
CTGTAGAGCCAGCCAGTCCGTGGGCGGCAGGTACCTGGCCTGGTACCAGAAGAAGGCCGGCCAGG
CCCCTAGGCTGCTGATCTCCGCCGCCAGCAGCAGAGCCACAGGCATCCCTGATCGCTTTTCCGGC
TCCGGCTCCGGCACAGATTTTACACTGACCATCAGCAGGGTGGAGCCCGAGGACTTCGCCGTGTA
CTACTGCCAGCAGTACGGCAGCAGCCCCAGAACCTTCGGCCAGGGCACCAAGGTGGAGATCAAG

SEQ ID NO: 26
<223> Amino acid sequence of the variable region of the light chain of Antibody 2
EIVLTQSPGTLSLSPGERATLSCRASQSVGGRYLAWYQKKAGQAPRLLISAASSRATGIPDRFSG
SGSGTDFTLTISRVEPEDFAVYYCQQYGSSPRTFGQGTKVEIK

# Fig. 13

SEQ ID NO: 27
<223> Nucleotide sequence of cDNA encoding the variable region of the heavy chain of Antibody 3
CAGGTGCAGCTGGTGGAGTCCGGCGGCGGCCTGGTGAAGCCCGGCGGCAGCCTGAGACTGAGCTG
TGCCGCCAGCGGCTTTACATTTTCCGATTACTACATGTCCTGGATCCGCCAGGCCCCAGGCAAGG
GCCTGGAGTGGGTGAGCTACATCTCCATCTCCGGCACAACACGCTACTACGCCGATTCCGTGAAG
GGCCGGTTCACAATCTCCCGGGATAATGCCAAGAATTCCCTGAGCCTGCAGATGAACAGCCTGAG
GGCCGAGGACACCGCCGTGTACTACTGCGCCAGGGGCCCCGAGCTGCTGAGCCAGAACTACTACT
ACTACTACGGCATGGACGTGTGGGGCCAGGGCACCACCGTGACCGTGAGCCTG

SEQ ID NO: 28
<223> Amino acid sequence of the variable region of the heavy chain of Antibody 3
QVQLVESGGGLVKPGGSLRLSCAASGFTFSDYYMSWIRQAPGKGLEWVSYISISGTTRYYADSVK
GRFTISRDNAKNSLSLQMNSLRAEDTAVYYCARGPELLSQNYYYYYGMDVWGQGTTVTVSL

SEQ ID NO: 29
<223> Nucleotide sequence of cDNA encoding the variable region of the light chain of Antibody 3
GACATCGAGATGACCCAGAGCCCCGACAGCCTGGCCGTGAGCCTGGGCGAGAGAGCCACCATCAA
CTGCAAGAGCAGCGAGACCCTGCTGTACAGAAGCAACAACAAGAATTACCTGGCCTGGTACCAGC
AGAAGCCCGGCCAGCCCCCTAGGCTGCTGCTGTACTGGGCCAGCACACGGGAGTCCGGCGTGCCT
GACAGGATCTCCGGCTCCGGCTCCGGCACCGATTTCACCCTGACCATCTCCTCCCTGCAGGCCGA
GGATGTGGCCGTGTACTACTGCCAGCAGTACTACACCACACCACGGACATTCGGCCAGGGCACAA
AGGTGGAGATCAAG

SEQ ID NO: 30
<223> Amino acid sequence of the variable region of the light chain of Antibody 3
DIEMTQSPDSLAVSLGERATINCKSSETLLYRSNNKNYLAWYQQKPGQPPRLLLYWASTRESGVP
DRISGSGSGTDFTLTISSLQAEDVAVYYCQQYYTTPRTFGQGTKVEIK

SEQ ID NO: 31
<223> Nucleotide sequence of cDNA encoding the variable region of the heavy chain of Antibody 4
GAGGTGCAGCTGGTTGAATCTGGCGGCGGATTGGTTCAGCCTGGCGGATCTCTGAGACTGTCTTG
TGCTGCCTCTGGCGGCTTCATCACCTCTCACCATTGGGTCCGACAGGCCCCTGGCAAAGGACTGG
AATGGGTTGCCAGACTGCACCACACCGGCACCACACACTACGCCGATTCTGTGAAGGGCAGATTC
ACCATCTCCGCCGACACCTCCAAGAACACCGCCTACCTGCAGATGAACTCCCTGAGAGCCGAGGA
CACCGCCGTGTACTACTGTGTGCGCGAGGACTCCTCTTCTTGGCACCCTGGCAGATATATCCAGC
TGTGGGGCCAGGGCACACTGGTCACAGTTTCTTCC

SEQ ID NO: 32
<223> Amino acid sequence of the variable region of the heavy chain of Antibody 4
EVQLVESGGGLVQPGGSLRLSCAASGGFITSHHWVRQAPGKGLEWVARLHHTGTTHYADSVKGRF
TISADTSKNTAYLQMNSLRAEDTAVYYCVREDSSSWHPGRYIQLWGQGTLVTVSS

# Fig. 14

SEQ ID NO: 33
<223> Nucleotide sequence of cDNA encoding the variable region of the light chain of Antibody 4
GACATCCAGATGACCCAGTCTCCATCCTCTCTGTCCGCCTCTGTGGGCGACAGAGTGACCATCTC
CTGCCGGTCATCTCAGTCCCTGCTGCAGAACAACCGGTACAACAGATTCGACTGGTATCAGCAGA
AGCCCGGCAAGGCCCCTAAGCTGCTGATCTACCTGGGCTCCAATAGAGCCTCTGGCGTGCCCTCT
AGATTCTCCGGCTCTAGATCTGGCACCGACTTTACCCTGACAATCTCCAGCCTGCAGCCTGAGGA
CTTCGCCACCTACTACTGCATGCACGCCCTGGAACCTCCATACACCTTTGGCCAGGGCACCAAGG
TGGAAATCAAG

SEQ ID NO: 34
<223> Amino acid sequence of the variable region of the light chain of Antibody 4
DIQMTQSPSSLSASVGDRVTISCRSSQSLLQNNRYNRFDWYQQKPGKAPKLLIYLGSNRASGVPS
RFSGSRSGTDFTLTISSLQPEDFATYYCMHALEPPYTFGQGTKVEIK

SEQ ID NO: 35
<223> Nucleotide sequence of the heavy chain of Antibody 1
CAGGTGCAGTTACAAGAGAGCGGCCCCGGCCTGGTGAAGCCCAGCGAGACCCTGTCCCTGACCTG
CACCGTGTCCGGCGGCTTCATCACCTCCCACCACTGGAGCTGGATCAGACAGCCCCCCGGCCAGG
GCCTGGAGTGGATCGGCTTCCTGCACCACACCGGCACCACCCACTACAACCCCTCCCTGAAGTCC
AGAGTGACCACCTCCGTGGACACCGCCAAGAACCAGTTCAGCCTGACCCTGAAGAGCCTGACCGC
CGCCGACACCGCCGTGTACTACTGCGTGAGGGAGGACTCCAGCAGCTGGCACCCCGGCAGATACA
TCCAGCTGTGGGGCCAGGGCACCCTGGTGACCGTGAGCGCCGCTAGCACCAAGGGCCCATCGGTC
TTCCCCCTGGCACCCTCCTCCAAGAGCACCTCTGGGGGCACAGCGGCCCTGGGCTGCCTGGTCAA
GGACTACTTCCCCGAACCGGTGACGGTGTCGTGGAACTCAGGCGCCCTGACCAGCGGCGTGCACA
CCTTCCCGGCTGTCCTACAGTCCTCAGGACTCTACTCCCTCAGCAGCGTGGTGACCGTGCCCTCC
AGCAGCTTGGGCACCCAGACCTACATCTGCAACGTGAATCACAAGCCCAGCAACACCAAGGTGGA
CAAGAAAGTTGAGCCCAAATCTTGTGACAAAACTCACACATGCCCACCGTGCCCAGCACCTGAAC
TCCTGGGGGGACCGTCAGTCTTCCTCTTCCCCCCAAAACCCAAGGACACCCTCATGATCTCCCGG
ACCCCTGAGGTCACGTGCGTGGTGGTGGACGTGAGCCACGAAGACCCCGAGGTCAAGTTCAACTG
GTACGTGGACGGCGTGGAGGTGCATAATGCCAAGACAAAGCCGCGGGAGGAGCAGTACAACAGCA
CGTACCGTGTGGTCAGCGTCCTCACCGTCCTGCACCAGGACTGGCTGAATGGCAAGGAGTACAAG
TGCAAGGTCTCCAACAAAGCCCTCCCAGCCCCCATCGAGAAAACCATCTCCAAAGCCAAAGGGCA
GCCCCGAGAACCACAGGTGTACACCCTGCCCCCATCCCGGGATGAGCTGACCAAGAACCAGGTCA
GCCTGACCTGCCTGGTCAAAGGCTTCTATCCCAGCGACATCGCCGTGGAGTGGGAGAGCAATGGG
CAGCCGGAGAACAACTACAAGACCACGCCTCCCGTGCTGGACTCCGACGGCTCCTTCTTCCTCTA
CAGCAAGCTCACCGTGGACAAGAGCAGGTGGCAGCAGGGGAACGTCTTCTCATGCTCCGTGATGC
ATGAGGCTCTGCACAACCACTACACGCAGAAGAGCCTCTCCCTGTCTCCGGGTAAA

SEQ ID NO: 36
<223> Amino acid sequence of heavy chain of Antibody 1
QVQLQESGPGLVKPSETLSLTCTVSGGFITSHHWSWIRQPPGQGLEWIGFLHHTGTTHYNPSLKS
RVTTSVDTAKNQFSLTLKSLTAADTAVYYCVREDSSSWHPGRYIQLWGQGTLVTVSAASTKGPSV
FPLAPSSKSTSGGTAALGCLVKDYFPEPVTVSWNSGALTSGVHTFPAVLQSSGLYSLSSVVTVPS
SSLGTQTYICNVNHKPSNTKVDKKVEPKSCDKTHTCPPCPAPELLGGPSVFLFPPKPKDTLMISR
TPEVTCVVVDVSHEDPEVKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYPSDIAVEWESNG
QPENNYKTTPPVLDSDGSFFLYSKLTVDKSRWQQGNVFSCSVMHEALHNHYTQKSLSLSPGK

# Fig. 15

SEQ ID NO: 37
<223> Nucleotide sequence of the light chain of Antibody 1
GACGTAGTGATGACCCAGAGCCCCCTGAGCCTGCCCGTGACCCCCGGCGAGCCCGCCAGCATCAG
TTGCAGAAGCAGCCAGAGCCTGCTTCAAAACAACAGATACAACAGATTCGACTGGTATCTCCAAA
AGCCCGGCCAGAGCCCCAGAATCCTGATCTACCTGGGCAGCAACAGAGCCAGCGGCGTGCCCGAC
AGATTCAGCGGCACCACCAGCGGCACCGACTACACCCTGAGAATCAGCAGAGTGGAGGCCGAGGA
CGCCGGCGTGTACTACTGTATGCACGCCCTGGAGCCCCCCTACACCTTCGGCCAGGGCACCAAGC
TGGAGATCAAGCGTACGGTGGCTGCACCATCTGTCTTCATCTTCCCGCCATCTGATGAGCAGTTG
AAATCTGGAACTGCCTCTGTTGTGTGCCTGCTGAATAACTTCTATCCCAGAGAGGCCAAAGTACA
GTGGAAGGTGGATAACGCCCTCCAATCGGGTAACTCCCAGGAGAGTGTCACAGAGCAGGACAGCA
AGGACAGCACCTACAGCCTCAGCAGCACCCTGACGCTGAGCAAAGCAGACTACGAGAAACACAAA
GTCTACGCCTGCGAAGTCACCCATCAGGGCCTGAGCTCGCCCGTCACAAAGAGCTTCAACAGGGG
AGAGTGT

SEQ ID NO: 38
<223> Amino acid sequence of the light chain of Antibody 1
DVVMTQSPLSLPVTPGEPASISCRSSQSLLQNNRYNRFDWYLQKPGQSPRILIYLGSNRASGVPD
RFSGTTSGTDYTLRISRVEAEDAGVYYCMHALEPPYTFGQGTKLEIKRTVAAPSVFIFPPSDEQL
KSGTASVVCLLNNFYPREAKVQWKVDNALQSGNSQESVTEQDSKDSTYSLSSTLTLSKADYEKHK
VYACEVTHQGLSSPVTKSFNRGEC

SEQ ID NO: 39
<223> Nucleotide sequence of the heavy chain of Antibody 2
GAGGTGCAGCTGGTGGAGAGCGGCGGCGGCCTGGTGCAGCCCGGCGGCAGCCTGAGACTGAGCTG
CGCCGCCAGCGGCTTCACATTCAACACCTACACCATGAACTGGGTGAGACAGGCCCCCGGCAAGG
GCCTGGAGTGGGTGGCCTACATCAGCCTGAGCAGCAACACCATCTTCTACGCCCCTTCCGTGAAG
GGCCGGTTTACAGTGTCCCGGGATAATGCCAAGAATTCCCTGTACCTGCAGATGAATACACTGTC
CGATGATGACACAGCCGTGTACTACTGTGCCCGCGCCGAGAGCTACTACGACTCCCACTACTACT
ACAACGGCCTGGACGTGTGGGGCCAGGGCACCACCGTGTCCGTGACATCCGCTAGCACCAAGGGC
CCATCGGTCTTCCCCCTGGCACCCTCCTCCAAGAGCACCTCTGGGGGCACAGCGGCCCTGGGCTG
CCTGGTCAAGGACTACTTCCCCGAACCGGTGACGGTGTCGTGGAACTCAGGCGCCCTGACCAGCG
GCGTGCACACCTTCCCGGCTGTCCTACAGTCCTCAGGACTCTACTCCCTCAGCAGCGTGGTGACC
GTGCCCTCCAGCAGCTTGGGCACCCAGACCTACATCTGCAACGTGAATCACAAGCCCAGCAACAC
CAAGGTGGACAAGAAAGTTGAGCCCAAATCTTGTGACAAAACTCACACATGCCCACCGTGCCCAG
CACCTGAACTCCTGGGGGGACCGTCAGTCTTCCTCTTCCCCCCAAAACCCAAGGACACCCTCATG
ATCTCCCGGACCCCTGAGGTCACGTGCGTGGTGGTGGACGTGAGCCACGAAGACCCCGAGGTCAA
GTTCAACTGGTACGTGGACGGCGTGGAGGTGCATAATGCCAAGACAAAGCCGCGGGAGGAGCAGT
ACAACAGCACGTACCGTGTGGTCAGCGTCCTCACCGTCCTGCACCAGGACTGGCTGAATGGCAAG
GAGTACAAGTGCAAGGTCTCCAACAAAGCCCTCCCAGCCCCCATCGAGAAAACCATCTCCAAAGC
CAAAGGGCAGCCCCGAGAACCACAGGTGTACACCCTGCCCCCATCCCGGGATGAGCTGACCAAGA
ACCAGGTCAGCCTGACCTGCCTGGTCAAAGGCTTCTATCCCAGCGACATCGCCGTGGAGTGGGAG
AGCAATGGGCAGCCGGAGAACAACTACAAGACCACGCCTCCCGTGCTGGACTCCGACGGCTCCTT
CTTCCTCTACAGCAAGCTCACCGTGGACAAGAGCAGGTGGCAGCAGGGGAACGTCTTCTCATGCT
CCGTGATGCATGAGGCTCTGCACAACCACTACACGCAGAAGAGCCTCTCCCTGTCTCCGGGTAAA

# Fig. 16

SEQ ID NO: 40
<223> Amino acid sequence of heavy chain of Antibody 2
EVQLVESGGGLVQPGGSLRLSCAASGFTFNTYTMNWVRQAPGKGLEWVAYISLSSNTIFYAPSVK
GRFTVSRDNAKNSLYLQMNTLSDDDTAVYYCARAESYYDSHYYYNGLDVWGQGTTVSVTSASTKG
PSVFPLAPSSKSTSGGTAALGCLVKDYFPEPVTVSWNSGALTSGVHTFPAVLQSSGLYSLSSVVT
VPSSSLGTQTYICNVNHKPSNTKVDKKVEPKSCDKTHTCPPCPAPELLGGPSVFLFPPKPKDTLM
ISRTPEVTCVVVDVSHEDPEVKFNWYVDGVEVHNAKTKPREEQYNSTYRVVSVLTVLHQDWLNGK
EYKCKVSNKALPAPIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNHYTQKSLSLSPGK

SEQ ID NO: 41
<223> Nucleotide sequence of the light chain of Antibody 2
GAAATCGTGCTGACACAGTCCCCAGGCACACTGTCCCTGTCCCCAGGCGAGCGCGCCACCCTGAG
CTGTAGAGCCAGCCAGTCCGTGGGCGGCAGGTACCTGGCCTGGTACCAGAAGAAGGCCGGCCAGG
CCCCTAGGCTGCTGATCTCCGCCGCCAGCAGCAGAGCCACAGGCATCCCTGATCGCTTTTCCGGC
TCCGGCTCCGGCACAGATTTTACACTGACCATCAGCAGGGTGGAGCCCGAGGACTTCGCCGTGTA
CTACTGCCAGCAGTACGGCAGCAGCCCCAGAACCTTCGGCCAGGGCACCAAGGTGGAGATCAAGC
GTACGGTGGCTGCACCATCTGTCTTCATCTTCCCGCCATCTGATGAGCAGTTGAAATCTGGAACT
GCCTCTGTTGTGTGCCTGCTGAATAACTTCTATCCCAGAGAGGCCAAAGTACAGTGGAAGGTGGA
TAACGCCCTCCAATCGGGTAACTCCCAGGAGAGTGTCACAGAGCAGGACAGCAAGGACAGCACCT
ACAGCCTCAGCAGCACCCTGACGCTGAGCAAAGCAGACTACGAGAAACACAAAGTCTACGCCTGC
GAAGTCACCCATCAGGGCCTGAGCTCGCCCGTCACAAAGAGCTTCAACAGGGGAGAGTGT

SEQ ID NO: 42
<223> Amino acid sequence of the light chain of Antibody 2
EIVLTQSPGTLSLSPGERATLSCRASQSVGGRYLAWYQKKAGQAPRLLISAASSRATGIPDRFSG
SGSGTDFTLTISRVEPEDFAVYYCQQYGSSPRTFGQGTKVEIKRTVAAPSVFIFPPSDEQLKSGT
ASVVCLLNNFYPREAKVQWKVDNALQSGNSQESVTEQDSKDSTYSLSSTLTLSKADYEKHKVYAC
EVTHQGLSSPVTKSFNRGEC

# Fig. 17

SEQ ID NO: 43
<223> Nucleotide sequence of the heavy chain of Antibody 3
CAGGTGCAGCTGGTGGAGTCCGGCGGCGGCCTGGTGAAGCCCGGCGGCAGCCTGAGACTGAGCTG
TGCCGCCAGCGGCTTTACATTTTCCGATTACTACATGTCCTGGATCCGCCAGGCCCCAGGCAAGG
GCCTGGAGTGGGTGAGCTACATCTCCATCTCCGGCACAACACGCTACTACGCCGATTCCGTGAAG
GGCCGGTTCACAATCTCCCGGGATAATGCCAAGAATTCCCTGAGCCTGCAGATGAACAGCCTGAG
GGCCGAGGACACCGCCGTGTACTACTGCGCCAGGGGCCCCGAGCTGCTGAGCCAGAACTACTACT
ACTACTACGGCATGGACGTGTGGGGCCAGGGCACCACCGTGACCGTGAGCCTGGCTAGCACCAAG
GGCCCATCGGTCTTCCCCCTGGCACCCTCCTCCAAGAGCACCTCTGGGGGCACAGCGGCCCTGGG
CTGCCTGGTCAAGGACTACTTCCCCGAACCGGTGACGGTGTCGTGGAACTCAGGCGCCCTGACCA
GCGGCGTGCACACCTTCCCGGCTGTCCTACAGTCCTCAGGACTCTACTCCCTCAGCAGCGTGGTG
ACCGTGCCCTCCAGCAGCTTGGGCACCCAGACCTACATCTGCAACGTGAATCACAAGCCCAGCAA
CACCAAGGTGGACAAGAAAGTTGAGCCCAAATCTTGTGACAAAACTCACACATGCCCACCGTGCC
CAGCACCTGAACTCCTGGGGGGACCGTCAGTCTTCCTCTTCCCCCCAAAACCCAAGGACACCCTC
ATGATCTCCCGGACCCCTGAGGTCACGTGCGTGGTGGTGGACGTGAGCCACGAAGACCCCGAGGT
CAAGTTCAACTGGTACGTGGACGGCGTGGAGGTGCATAATGCCAAGACAAAGCCGCGGGAGGAGC
AGTACAACAGCACGTACCGTGTGGTCAGCGTCCTCACCGTCCTGCACCAGGACTGGCTGAATGGC
AAGGAGTACAAGTGCAAGGTCTCCAACAAAGCCCTCCCAGCCCCCATCGAGAAAACCATCTCCAA
AGCCAAAGGGCAGCCCCGAGAACCACAGGTGTACACCCTGCCCCCATCCCGGGATGAGCTGACCA
AGAACCAGGTCAGCCTGACCTGCCTGGTCAAAGGCTTCTATCCCAGCGACATCGCCGTGGAGTGG
GAGAGCAATGGGCAGCCGGAGAACAACTACAAGACCACGCCTCCCGTGCTGGACTCCGACGGCTC
CTTCTTCCTCTACAGCAAGCTCACCGTGGACAAGAGCAGGTGGCAGCAGGGGAACGTCTTCTCAT
GCTCCGTGATGCATGAGGCTCTGCACAACCACTACACGCAGAAGAGCCTCTCCCTGTCTCCGGGT
AAA

SEQ ID NO: 44
<223> Amino acid sequence of heavy chain of Antibody 3
QVQLVESGGGLVKPGGSLRLSCAASGFTFSDYYMSWIRQAPGKGLEWVSYISISGTTRYYADSVK
GRFTISRDNAKNSLSLQMNSLRAEDTAVYYCARGPELLSQNYYYYYGMDVWGQGTTVTVSLASTK
GPSVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKDTL
MISRTPEVTCVVVDVSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLTCLVKGFYPSDIAVEW
ESNGQPENNYKTTPPVLDSDGSFFLYSKLTVDKSRWQQGNVFSCSVMHEALHNHYTQKSLSLSPG
K

SEQ ID NO: 45
<223> Nucleotide sequence of the light chain of Antibody 3
GACATCGAGATGACCCAGAGCCCCGACAGCCTGGCCGTGAGCCTGGGCGAGAGAGCCACCATCAA
CTGCAAGAGCAGCGAGACCCTGCTGTACAGAAGCAACAACAAGAATTACCTGGCCTGGTACCAGC
AGAAGCCCGGCCAGCCCCCTAGGCTGCTGCTGTACTGGGCCAGCACACGGGAGTCCGGCGTGCCT
GACAGGATCTCCGGCTCCGGCTCCGGCACCGATTTCACCCTGACCATCTCCTCCCTGCAGGCCGA
GGATGTGGCCGTGTACTACTGCCAGCAGTACTACACCACACCACGGACATTCGGCCAGGGCACAA
AGGTGGAGATCAAGCGTACGGTGGCTGCACCATCTGTCTTCATCTTCCCGCCATCTGATGAGCAG
TTGAAATCTGGAACTGCCTCTGTTGTGTGCCTGCTGAATAACTTCTATCCCAGAGAGGCCAAAGT
ACAGTGGAAGGTGGATAACGCCCTCCAATCGGGTAACTCCCAGGAGAGTGTCACAGAGCAGGACA
GCAAGGACAGCACCTACAGCCTCAGCAGCACCCTGACGCTGAGCAAAGCAGACTACGAGAAACAC
AAAGTCTACGCCTGCGAAGTCACCCATCAGGGCCTGAGCTCGCCCGTCACAAAGAGCTTCAACAG
GGGAGAGTGT

# Fig. 18

SEQ ID NO: 46
<223> Amino acid sequence of the light chain of Antibody 3
DIEMTQSPDSLAVSLGERATINCKSSETLLYRSNNKNYLAWYQQKPGQPPRLLLYWASTRESGVP
DRISGSGSGTDFTLTISSLQAEDVAVYYCQQYYTTPRTFGQGTKVEIKRTVAAPSVFIFPPSDEQ
LKSGTASVVCLLNNFYPREAKVQWKVDNALQSGNSQESVTEQDSKDSTYSLSSTLTLSKADYEKH
KVYACEVTHQGLSSPVTKSFNRGEC

SEQ ID NO: 47
<223> Nucleotide sequence of the heavy chain of Antibody 4
GAGGTGCAGCTGGTTGAATCTGGCGGCGGATTGGTTCAGCCTGGCGGATCTCTGAGACTGTCTTG
TGCTGCCTCTGGCGGCTTCATCACCTCTCACCATTGGGTCCGACAGGCCCCTGGCAAAGGACTGG
AATGGGTTGCCAGACTGCACCACACCGGCACCACACACTACGCCGATTCTGTGAAGGGCAGATTC
ACCATCTCCGCCGACACCTCCAAGAACACCGCCTACCTGCAGATGAACTCCCTGAGAGCCGAGGA
CACCGCCGTGTACTACTGTGTGCGCGAGGACTCCTCTTCTTGGCACCCTGGCAGATATATCCAGC
TGTGGGGCCAGGGCACACTGGTCACAGTTTCTTCCGCCTCCACCAAGGGACCCAGCGTTTTCCCT
CTGGCTCCATCCTCCAAGTCTACCTCCGGTGGAACAGCTGCTCTGGGCTGCCTGGTCAAGGACTA
CTTTCCTGAGCCTGTGACCGTGTCCTGGAACTCTGGCGCTCTGACATCTGGCGTGCACACCTTTC
CAGCAGTGCTGCAGTCCTCCGGCCTGTACTCTCTGTCCTCTGTCGTGACCGTGCCTTCCAGCTCT
CTGGGAACCCAGACCTACATCTGCAATGTGAACCACAAGCCTTCCAACACCAAGGTGGACAAGAA
GGTGGAACCCAAGTCCTGCGACAAGACCCACACCTGTCCTCCATGTCCTGCTCCAGAACTGCTCG
GCGGACCTTCCGTGTTTCTGTTCCCTCCAAAGCCTAAGGACACCCTGATGATCTCTCGGACCCCT
GAAGTGACCTGCGTGGTGGTGGATGTGTCTCACGAGGATCCCGAAGTGAAGTTCAATTGGTACGT
GGACGGCGTGGAAGTGCACAACGCCAAGACCAAGCCTAGAGAGGAACAGTACAACTCCACCTACA
GAGTGGTGTCCGTGCTGACCGTGCTGCACCAGGATTGGCTGAACGGCAAAGAGTACAAGTGCAAG
GTGTCCAACAAGGCCCTGCCTGCTCCTATCGAAAAGACCATCTCCAAGGCCAAGGGCCAGCCTAG
GGAACCCCAGGTTTACACCCTGCCTCCAAGCCGGGAAGAGATGACCAAGAACCAGGTGTCCCTGA
CCTGCCTCGTGAAGGGCTTCTACCCTTCCGATATCGCCGTGGAATGGGAGAGCAATGGCCAGCCT
GAGAACAACTACAAGACAACCCCTCCTGTGCTGGACTCCGACGGCTCATTCTTCCTGTACTCCAA
GCTGACAGTGGACAAGTCCAGATGGCAGCAGGGCAACGTGTTCTCCTGCTCCGTGATGCACGAGG
CCCTGCACAATCACTACACCCAGAAGTCCCTGTCTCTGTCCCCTGGCTAA

SEQ ID NO: 48
<223> Amino acid sequence of the heavy chain of Antibody 4
EVQLVESGGGLVQPGGSLRLSCAASGGFITSHHWVRQAPGKGLEWVARLHHTGTTHYADSVKGRF
TISADTSKNTAYLQMNSLRAEDTAVYYCVREDSSSWHPGRYIQLWGQGTLVTVSSASTKGPSVFP
LAPSSKSTSGGTAALGCLVKDYFPEPVTVSWNSGALTSGVHTFPAVLQSSGLYSLSSVVTVPSSS
LGTQTYICNVNHKPSNTKVDKKVEPKSCDKTHTCPPCPAPELLGGPSVFLFPPKPKDTLMISRTP
EVTCVVVDVSHEDPEVKFNWYVDGVEVHNAKTKPREEQYNSTYRVVSVLTVLHQDWLNGKEYKCK
VSNKALPAPIEKTISKAKGQPREPQVYTLPPSREEMTKNQVSLTCLVKGFYPSDIAVEWESNGQP
ENNYKTTPPVLDSDGSFFLYSKLTVDKSRWQQGNVFSCSVMHEALHNHYTQKSLSLSPG

# Fig. 19

SEQ ID NO: 49
<223> Nucleotide sequence of the light chain of Antibody 4
GACATCCAGATGACCCAGTCTCCATCCTCTCTGTCCGCCTCTGTGGGCGACAGAGTGACCATCTC
CTGCCGGTCATCTCAGTCCCTGCTGCAGAACAACCGGTACAACAGATTCGACTGGTATCAGCAGA
AGCCCGGCAAGGCCCCTAAGCTGCTGATCTACCTGGGCTCCAATAGAGCCTCTGGCGTGCCCTCT
AGATTCTCCGGCTCTAGATCTGGCACCGACTTTACCCTGACAATCTCCAGCCTGCAGCCTGAGGA
CTTCGCCACCTACTACTGCATGCACGCCCTGGAACCTCCATACACCTTTGGCCAGGGCACCAAGG
TGGAAATCAAGCGGACAGTGGCCGCTCCTTCCGTGTTCATCTTCCCACCTTCCGACGAGCAGCTG
AAGTCCGGCACAGCTTCTGTCGTGTGCCTGCTGAACAACTTCTACCCTCGGGAAGCCAAGGTGCA
GTGGAAGGTGGACAATGCCCTGCAGTCCGGCAACTCCCAAGAGTCTGTGACCGAGCAGGACTCCA
AGGACAGCACCTACAGCCTGTCCTCCACACTGACCCTGTCCAAGGCCGACTACGAGAAGCACAAG
GTGTACGCCTGCGAAGTGACCCATCAGGGCCTGTCTAGCCCTGTGACCAAGTCTTTCAACCGGGG
CGAGTGCTGA

SEQ ID NO: 50
<223> Amino acid sequence of the light chain of Antibody 4
DIQMTQSPSSLSASVGDRVTISCRSSQSLLQNNRYNRFDWYQQKPGKAPKLLIYLGSNRASGVPS
RFSGSRSGTDFTLTISSLQPEDFATYYCMHALEPPYTFGQGTKVEIKRTVAAPSVFIFPPSDEQL
KSGTASVVCLLNNFYPREAKVQWKVDNALQSGNSQESVTEQDSKDSTYSLSSTLTLSKADYEKHK
VYACEVTHQGLSSPVTKSFNRGEC

**INTERNATIONAL SEARCH REPORT**

International application No.
**PCT/JP2023/013095**

**A. CLASSIFICATION OF SUBJECT MATTER**

***C07K 16/30***(2006.01)i; ***A61K 39/395***(2006.01)i; ***A61K 47/68***(2017.01)i; ***A61P 35/00***(2006.01)i; ***C12N 15/13***(2006.01)i
FI: C07K16/30 ZNA; A61K47/68; A61K39/395 Y; A61P35/00; A61K39/395 T; C12N15/13

According to International Patent Classification (IPC) or to both national classification and IPC

**B. FIELDS SEARCHED**

Minimum documentation searched (classification system followed by classification symbols)

C07K16/30; A61K39/395; A61K47/68; A61P35/00; C12N15/13

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2018/235855 A1 (NATIONAL UNIVERSITY CORPORATION TOKYO MEDICAL AND DENTAL UNIVERSITY) 27 December 2018 (2018-12-27) claims, paragraphs [0051], [0052] | 1-29 |
| A | FUJII, Masahiro et al. Cytoplasmic expression of the JM403 antigen GlcA-GlcNH3+ on heparan sulfate glycosaminoglycan in mammary carcinomas - a novel proliferative biomarker for breast cancers with high malignancy. Glycoconj. J. 2010, vol. 27, pp. 661-672, doi:10.1007/s10719-010-9311-4 abstract, results, discussion | 1-29 |
| A | NAGASE, Haruna et al. Reliable and sensitive detection of glycosaminoglycan chains with immunoblots. Glycobiology. 2021, vol. 31, no. 2, pp. 116-125, DOI:10.1093/glycob/cwaa060 abstract, Results and discussion | 1-29 |
| A | JP 2021-518133 A (FIVE PRIME THERAPEUTICS, INC.) 02 August 2021 (2021-08-02) claims, paragraph [0004] | 1-29 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 June 2023** | **20 June 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/013095**

**C. DOCUMENTS CONSIDERED TO BE RELEVANT**

| Category* | Citation of document, with indication, where appropriate, of the relevant passages | Relevant to claim No. |
|---|---|---|
| P, X | FURUYA, Genta et al. Nucleic acid-triggered tumoral immunity propagates pH-selective therapeutic antibodies through tumor-driven epitope spreading. Cancer Science. 2023, vol. 114, pp. 321-338, DOI:10.1111/cas.15596<br>whole document, in particular, #008T-2, #021T-1, #022T-2 | 1-29 |

Form PCT/ISA/210 (second sheet) (January 2015)

**INTERNATIONAL SEARCH REPORT**

International application No.
**PCT/JP2023/013095**

**Box No. I** **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

The “form of an Annex C/ST.25 text file” is replaced with the “form of ST.26.”

**INTERNATIONAL SEARCH REPORT**
**Information on patent family members**

International application No.
**PCT/JP2023/013095**

| Patent document cited in search report | Publication date (day/month/year) | Patent family member(s) | Publication date (day/month/year) |
|---|---|---|---|
| WO 2018/235855 A1 | 27 December 2018 | US 2020/0283510 A1<br>claims, paragraphs [0069], [0070]<br>EP 3643725 A1<br>CN 111051342 A | |
| JP 2021-518133 A | 02 August 2021 | US 2021/0017283 A1<br>claims, paragraph [0004]<br>WO 2019/183040 A1<br>EP 3768716 A1<br>TW 201945393 A<br>CN 111971304 A<br>KR 10-2020-0135421 A<br>CA 3092589 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018235855 A
- JP 2022057542 A


### Non-patent literature cited in the description


- *Int. J. Mol. Sci.*, 2020, vol. 21 (17), 5983
- *Biomolecules*, 2021, vol. 11 (3), 395
- *Cancer Cell*, 2015, vol. 28 (4), 500-514
- *Bioconjugate Chem.*, 2010, vol. 21, 5-13
- *Current Opinion in Chemical Biology*, 2010, vol. 14, 529-537
- *Cell Rep*, 2017, vol. 20 (5), 1073-1087
- *J. Med. Chem*, 13 November 2008, vol. 51 (21), 6916-6926
- *Int. J. Mol. Sci*, 2020, vol. 21 (17), 5983
- *Biochemical Pharmacology*, 1991, vol. 42 (10), 1987-1995
- *Oncotarget*, 2017, vol. 8 (40), 66960-66974
- *Protein Science*, 1995, vol. 4, 2411-2423